(19) Europäisches Patentamt
European Patent Office
Office européen des brevets

(11) EP 4 155 320 A1

(12) **EUROPEAN PATENT APPLICATION**
published in accordance with Art. 153(4) EPC

(43) Date of publication:
**29.03.2023 Bulletin 2023/13**

(21) Application number: **21834047.9**

(22) Date of filing: **29.06.2021**

(51) International Patent Classification (IPC):
***C07K 16/28*** *(2006.01)* ***A61K 39/395*** *(2006.01)*
***A61P 35/00*** *(2006.01)*

(52) Cooperative Patent Classification (CPC):
**A61K 39/395; A61P 35/00; C07K 16/28**

(86) International application number:
**PCT/CN2021/102952**

(87) International publication number:
**WO 2022/002012 (06.01.2022 Gazette 2022/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.06.2020 CN 202010618159**

(71) Applicant: **Nona Biosciences (Suzhou) Co., Ltd.**
**Suzhou, Jiangsu 215000 (CN)**

(72) Inventors:
- **WU, Xiaodong**
  **Shanghai 201203 (CN)**
- **WANG, Yongqiang**
  **Shanghai 201203 (CN)**
- **CHEN, Fei**
  **Shanghai 201203 (CN)**
- **HE, Jinqiu**
  **Shanghai 201203 (CN)**
- **JIA, Gezi**
  **Shanghai 201203 (CN)**
- **ZHAO, Chuchu**
  **Shanghai 201203 (CN)**
- **CHEN, Qingfang**
  **Shanghai 201203 (CN)**
- **RONG, Yiping**
  **Shanghai 201203 (CN)**

(74) Representative: **Maiwald GmbH**
**Elisenhof**
**Elisenstraße 3**
**80335 München (DE)**

Remarks:
The complete document including Reference Table(s) and the Sequence Listing(s) can be downloaded from the EPO website

# (54) ANTI-B7H4 ANTIBODY, AND BISPECIFIC ANTIBODY AND USE THEREOF

(57) Provided are a B7H4-targeting antibody or a variant thereof, and a bispecific antibody containing the B7H4-targeting antibody, wherein the B7H4-targeting antibody comprises a light chain variable region and a heavy chain variable region. The variant has an amino acid mutation in the light chain variable region or heavy chain variable region of the antibody and can maintain the function of the antibody. The B7H4-targeting antibody has the activity of binding to human B7H4 and cynomolgus macaque B7H4, and does not cross-react with other B7 family members. The B7H4-targeting antibody exhibits its good anti-tumor activity, T-cell activation activity and internalization activity in in-vivo and in-vitro experiments, and is more suitable for use as an ADC drug. Further provided is a bispecific antibody targeting B7H4 and CD3, which has a longer half-life, retains good stability and hydrophilicity, and reduces toxicity while ensuring efficacy.



Processed by Luminess, 75001 PARIS (FR)

## Description

The present application claims priority to Chinese Patent Application No. 202010618159.5 filed on Jun. 30, 2020, the content of which is incorporated herein by reference in its entirety.

## TECHNICAL FIELD

The present invention relates to the field of biomedicine, and particularly to an anti-B7H4 antibody, a bispecific antibody and use thereof.

## BACKGROUND

Breast cancer, ovarian cancer and endometrioma are common malignant tumors in women, wherein the breast cancer ranks first among cancers in women in incidence, and the data of the International Agency for Research on Cancer (IARC) in 2018 show that the incidence rate of the breast cancer in the cancers in women all over the world is 24.2%. For the treatment of these cancers, immunotherapy and targeted therapy are the current hot spots. For example, Her2-targeting Herceptin has a good therapeutic effect on Her2 positive breast cancer. For triple negative breast cancer (TNBC), few therapeutic approaches exist at present due to the lack of corresponding targets, mainly including chemotherapy. FDA approved the PD-L1 inhibitor Atezolizumab in combination with albumin-bound paclitaxel for the treatment of metastatic triple negative breast cancer (TNBC) in March 2019, but PD-L1 is not highly expressed on triple negative breast cancer.

Immune checkpoint inhibitors are the most studied form of immunotherapy for breast cancer. Immune checkpoint molecules are often highly expressed in the tumor microenvironment, and tumors can evade the attack by the immune system by inhibiting the activation of T cells and inducing the depletion of T cells. The B7 family and the TNF family are two main co-stimulatory molecule families, wherein the B7 family comprises 10 molecules, namely CD80 (B7.1), CD86 (B7.2), B7H1 (PD-L1/CD274), B7-DC (PD-L2/CD273), B7H2 (ICOSL), B7H3 (CD276), B7H4 (B7S1/B7x/Vtcn1), B7H5 (VISTA), B7H6 and B7H7 (HHLA2). Several members of the B7 family and their receptors have been shown to be immune checkpoints, such as PD-L1/PD1, CTLA4 and VISTA.

B7H4 is a relatively new B7 family member, although widely expressed in body cells at mRNA level, its protein level expression is very limited, and only in part of ductal epithelial cells of the body, such as mammary duct and lobule, oviduct epithelium, endometrium and other tissues, has low-level expression. In contrast, B7H4 is abundantly expressed in a variety of tumor tissues, such as on tumor cells of breast cancer, particularly triple negative breast cancer, ovarian cancer, and endometrioma. In terms of expression profile, B7H4 can be considered as a tumor-associated antigen with high specificity. On the other hand, B7H4 is a new immune checkpoint molecule, and *in vitro* experiments prove that B7H4 inhibits the proliferation and activation of T cells and the production of cytokines by interacting with its unknown T cell surface receptors. The tumor cells inhibit the activation of T cells through high expression of B7H4 molecules and inhibitory macrophages with high expression of B7H4 molecules in a tumor microenvironment, thereby realizing immune evasion. The expression profile of B7H4 on the tumor does not overlap with PD-L1. The method for reactivating the immune system through the treatment with a B7H4-targeting antibody and by blocking the negative regulation effect of B7H4 is a promising means for treating B7H4 positive tumors.

Monoclonal antibodies against B7-H4, or antibody-drug conjugates, or bispecific antibodies are currently being developed by several pharmaceutical companies. Genentech, BMS, Jounce, Jiangsu Hanson, etc. are in preclinical development stage, wherein the most rapidly advancing company is FivePrime with its anti-B7H4 monoclonal antibody, which is currently in clinically phase I, and its mechanism is primarily through ADCC and blocking of immune checkpoints to activate T cells. However, a human anti-B7H4 antibody with high affinity, high selectivity and high bioactivity has not yet been found clinically.

## SUMMARY

To solve the technical problem that a human anti-B7H4 antibody with high affinity, high selectivity and high bioactivity is unavailable in the art, the present invention provides a fully human anti-B7H4 antibody with high affinity, high selectivity and high bioactivity by utilizing a specific humanized mouse platform from Harbour, and a bispecific antibody of anti-B7H4×CD3 constructed on the basis of the antibody, so as to be applied to the treatment of B7H4 positive tumors.

The first technical solution of the present invention is as follows: provided is a B7H4-targeting antibody or a variant thereof, wherein the antibody comprises a light chain variable region and a heavy chain variable region, wherein:

the light chain variable region comprises a LCDR1, a LCDR2 and a LCDR3 with amino acid sequences as set forth in SEQ ID NOs: 47, 54 and 64, respectively; the heavy chain variable region comprises a HCDR1, a HCDR2 and a HCDR3 with amino acid sequences as set forth in SEQ ID NOs: 9, 21 and 35, respectively; or

the light chain variable region comprises a LCDR1, a LCDR2 and a LCDR3 with amino acid sequences as set forth in SEQ ID NOs: 48, 54 and 65, respectively; the heavy chain variable region comprises a HCDR1, a HCDR2 and a HCDR3 with amino acid sequences as set forth in SEQ ID NOs: 11, 22 and 36, respectively;

the variant has an amino acid mutation in the light chain variable region and/or the heavy chain variable region of the antibody and can maintain functions of the antibody. In the present invention, the amino acid mutation may be one or more amino acid residue deletions, substitutions or additions in the original amino acid sequence, for example, CDR. Preferably, the amino acid mutation is an amino acid substitution, and the number of the amino acid substitutions is 1 to 3. Moreover, the amino acid sequence of the mutation has at least 85% sequence identity to the original amino acid sequence and maintains or improves the binding of the antibody to the target antigen; the at least 85% sequence identity is preferably at least 90% sequence identity, more preferably at least 95%, 96%, 97% or 98% sequence identity, and most preferably at least 99% sequence identity. The B7H4-targeting antibody is also referred to as an anti-B7H4 antibody in the present invention.

In some specific embodiments, for the antibody or the variant thereof as described above, the variant has an amino acid substitution at position 3 or 4 of the HCDR2 and position 3 of the HCDR3 in the heavy chain variable region of the antibody, and/or an amino acid substitution at position 4 of the LCDR3 in the light chain variable region of the antibody.

Preferably, D at position 3 of the HCDR2 is substituted with G or E, and/or G at position 4 is substituted with A, and/or G at position 3 of the HCDR3 is substituted with A; N at position 4 of the LCDR3 is substituted with S, R or Q. That is, the light chain variable region comprises a LCDR1, a LCDR2, and a LCDR3 with amino acid sequences as set forth in SEQ ID NOs: 48, 55 and 66, respectively; the heavy chain variable region comprises a HCDR1, a HCDR2 and a HCDR3 with amino acid sequences as set forth in SEQ ID NOs: 11, 22 and 36, respectively; or the light chain variable region comprises a LCDR1, a LCDR2 and a LCDR3 with amino acid sequences as set forth in SEQ ID NOs: 47, 54 and 67, respectively; the heavy chain variable region comprises a HCDR1, a HCDR2 and a HCDR3 with amino acid sequences as set forth in SEQ ID NOs: 9, 23 and 35, respectively; or the light chain variable region comprises a LCDR1, a LCDR2 and a LCDR3 with amino acid sequences as set forth in SEQ ID NOs: 47, 54 and 67, respectively; the heavy chain variable region comprises a HCDR1, a HCDR2 and a HCDR3 with amino acid sequences as set forth in SEQ ID NOs: 9, 24 and 35, respectively; or the light chain variable region comprises a LCDR1, a LCDR2, and a LCDR3 with amino acid sequences as set forth in SEQ ID NOs: 47, 54, and 69, respectively; the heavy chain variable region comprises a HCDR1, a HCDR2 and a HCDR3 with amino acid sequences as set forth in SEQ ID NOs: 9, 24 and 38, respectively.

In some specific embodiments, for the antibody or the variant thereof as described above, the heavy chain variable region further comprises a heavy chain variable region framework region HFWR, and/or the light chain variable region further comprises a light chain variable region framework region LFWR, wherein the HFWR is a heavy chain variable region framework region of a human antibody and the LFWR is a light chain variable region framework region of a human antibody.

Preferably,

the light chain variable region comprises an amino acid sequence as set forth in SEQ ID NO: 87; the heavy chain variable region comprises an amino acid sequence as set forth in SEQ ID NO: 77; or,

the light chain variable region comprises an amino acid sequence as set forth in SEQ ID NO: 88; the heavy chain variable region comprises an amino acid sequence as set forth in SEQ ID NO: 79; or,

the light chain variable region comprises an amino acid sequence as set forth in SEQ ID NO: 89; the heavy chain variable region comprises an amino acid sequence as set forth in SEQ ID NO: 79; or,

the light chain variable region comprises an amino acid sequence as set forth in SEQ ID NO: 90; the heavy chain variable region comprises an amino acid sequence as set forth in SEQ ID NO: 80; or,

the light chain variable region comprises an amino acid sequence as set forth in SEQ ID NO: 90; the heavy chain variable region comprises an amino acid sequence as set forth in SEQ ID NO: 81; or,

the light chain variable region comprises an amino acid sequence as set forth in SEQ ID NO: 93; the heavy chain variable region comprises an amino acid sequence as set forth in SEQ ID NO: 83; or,

the light chain variable region comprises an amino acid sequence as set forth in SEQ ID NO: 91; the heavy chain variable region comprises an amino acid sequence as set forth in SEQ ID NO: 79.

In the present invention, the amino acid mutation may also be one or more amino acid residue deletions, substitutions or additions in the original amino acid sequence, for example, FWR. Preferably, the amino acid mutation is an amino acid substitution, and the number of the amino acid substitutions is 1 to 3. Moreover, the amino acid sequence of the mutation has at least 85% sequence identity to the original amino acid sequence and maintains or improves the binding of the antibody to the target antigen; the at least 85% sequence identity is preferably at least 90% sequence identity, more preferably at least 95%, 96%, 97% or 98% sequence identity, and most preferably at least 99% sequence identity.

In some specific embodiments, for the antibody or the variant thereof as described above, the antibody further comprises a heavy chain constant region and/or a light chain constant region. Preferably, the heavy chain constant region of the antibody is selected from that of hIgG1, hIgG2, hIgG3 and hIgG4, and the light chain constant region is selected from that of a κ chain and a λ chain; more preferably, the variant has an amino acid substitution at position 239 and/or 332 of an Fc of the antibody, and preferably, the amino acid substitution is S239D and/or I332E.

In some specific embodiments, for the antibody or the variant thereof as described above, the antibody is a full-length antibody, an Fab, an Fab', an $F(ab')_2$, an Fv, an scFv, or a monoclonal or polyclonal antibody prepared from an antibody as above.

In some specific embodiments, for the antibody as described above, the antibody comprises (1) a heavy chain and a light chain, wherein the heavy chain comprises an amino acid sequence as set forth in SEQ ID NO: 95; the light chain comprises an amino acid sequence as set forth in SEQ ID NO: 109; or the heavy chain comprises an amino acid sequence as set forth in SEQ ID NO: 98; the light chain comprises an amino acid sequence as set forth in SEQ ID NO: 112; or,

the heavy chain comprises an amino acid sequence as set forth in SEQ ID NO: 98; the light chain comprises an amino acid sequence as set forth in SEQ ID NO: 113; or,
the heavy chain comprises an amino acid sequence as set forth in SEQ ID NO: 99; the light chain comprises an amino acid sequence as set forth in SEQ ID NO: 114; or,
the heavy chain comprises an amino acid sequence as set forth in SEQ ID NO: 100; the light chain comprises an amino acid sequence as set forth in SEQ ID NO: 114; or,
the heavy chain comprises an amino acid sequence as set forth in SEQ ID NO: 101; the light chain comprises an amino acid sequence as set forth in SEQ ID NO: 109; or,
the heavy chain comprises an amino acid sequence as set forth in SEQ ID NO: 102; the light chain comprises an amino acid sequence as set forth in SEQ ID NO: 114; or,
the heavy chain comprises an amino acid sequence as set forth in SEQ ID NO: 106; the light chain comprises an amino acid sequence as set forth in SEQ ID NO: 117; or,
the heavy chain comprises an amino acid sequence as set forth in SEQ ID NO: 98; the light chain comprises an amino acid sequence as set forth in SEQ ID NO: 115; or,
the heavy chain comprises an amino acid sequence as set forth in SEQ ID NO: 103; the light chain comprises an amino acid sequence as set forth in SEQ ID NO: 112; or,
the heavy chain comprises an amino acid sequence as set forth in SEQ ID NO: 103; the light chain comprises an amino acid sequence as set forth in SEQ ID NO: 115; or,
(2) a heavy chain, wherein the heavy chain comprises an amino acid sequence as set forth in SEQ ID NO: 132.

The term "antibody" may include an immunoglobulin, which is of a tetrapeptide chain structure formed by connection between two identical heavy chains and two identical light chains by interchain disulfide bonds. Immunoglobulins differ in amino acid composition and arrangement of their heavy chain constant regions and therefore in their antigenicity. Accordingly, immunoglobulins can be classified into five classes, or isotypes of immunoglobulins, namely IgM, IgD, IgG, IgA and IgE, with their corresponding heavy chains being the μ, δ, γ, α and ε chains, respectively. The Ig of the same class can be divided into different subclasses according to the differences in amino acid composition of the hinge regions and the number and location of disulfide bonds in the heavy chains; for example, IgG can be divided into IgG1, IgG2, IgG3, and IgG4. Light chains are classified into κ or λ chains by the difference in the constant regions. Each of the five classes of Ig can have a κ chain or a λ chain.

In the present application, the light chain variable region of the antibody of the present application may further comprise a light chain constant region comprising a human κ or λ chain or a variant thereof. In the present application, the heavy chain variable region of the antibody of the present application may further comprise a heavy chain constant region comprising human IgG1, IgG2, IgG3, IgG4 or a variant thereof.

In light chains and heavy chains, the variable region and constant region are linked by a "J" region of about 12 or more amino acids, and the heavy chain further comprises a "D" region of about 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain CL. The constant region of the antibody can mediate the binding of immunoglobulins to host tissues or factors, including the binding of various cells of the immune system (e.g., effector cells) to the first component (C1q) of classical complement system. The sequences of about 110 amino acids of the heavy and light chains of the antibody near the N-terminus vary considerably and thus are referred to as variable regions (V regions); the remaining amino acid sequences near the C-terminus are relatively stable and thus are referred to as constant regions (C regions). The variable regions comprise 3 hypervariable regions (HVRs) and 4 framework regions (FWRs) with relatively conservative sequences. The 3 hypervariable regions determine the specificity of the antibody and thus are also known as complementarity determining regions (CDRs). Each light chain variable region (VL) or heavy chain variable region (VH) consists of 3 CDR regions and 4 FWR regions arranged from the amino-terminus to the carboxyl-terminus in the following order: FWR1, CDR1, FWR2, CDR2, FWR3, CDR3, and FWR4.

To solve the above technical problem, the second technical solution of the present invention is as follows: provided is a B7H4-targeting bispecific antibody comprising a protein functional region A and a protein functional region B, wherein the protein functional region A is the B7H4-targeting antibody as described above; the protein functional region B is an antibody not targeting B7H4; preferably, the antibody not targeting B7H4 is a CD3-targeting antibody; more preferably, the CD3-targeting antibody comprises a light chain variable region and a heavy chain variable region, wherein the light chain variable region comprises a LCDR1, a LCDR2 and a LCDR3 with amino acid sequences as set forth in SEQ ID NOs: 46, 53 and 63, respectively; the heavy chain variable region comprises a HCDR1, a HCDR2 and a HCDR3 with amino acid sequences as set forth in SEQ ID NOs: 8, 20 and 34, respectively; or the light chain variable region comprises a LCDR1, a LCDR2 and a LCDR3 with amino acid sequences as set forth in SEQ ID NOs: 46, 53 and 63, respectively; the heavy chain variable region comprises a HCDR1, a HCDR2 and a HCDR3 with amino acid sequences as set forth in SEQ ID NOs: 10, 20 and 34, respectively; and even more preferably, in the CD3-targeting antibody, the light chain variable region comprises an amino acid sequence as set forth in SEQ ID NO: 86; the heavy chain variable region comprises an amino acid sequence as set forth in SEQ ID NO: 76; or the light chain variable region VL comprises an amino acid sequence as set forth in SEQ ID NO: 86; the heavy chain variable region VH comprises an amino acid sequence as set forth in SEQ ID NO: 78; or the light chain variable region VL comprises an amino acid sequence as set forth in SEQ ID NO: 86; the heavy chain variable region VH comprises an amino acid sequence as set forth in SEQ ID NO: 84.

In some specific embodiments, for the bispecific antibody as described above, the protein functional region B comprises a light chain variable region and a heavy chain variable region, and the protein functional region A comprises a light chain variable region and a heavy chain variable region; wherein,

in the protein functional region B, the light chain variable region comprises a LCDR1, a LCDR2 and a LCDR3 with amino acid sequences as set forth in SEQ ID NOs: 46, 53 and 63, respectively, and the heavy chain variable region comprises a HCDR1, a HCDR2 and a HCDR3 with amino acid sequences as set forth in SEQ ID NOs: 8, 20 and 34, respectively; in the protein functional region A, the light chain variable region comprises a LCDR1, a LCDR2 and a LCDR3 with amino acid sequences as set forth in SEQ ID NOs: 47, 54 and 64, respectively, and the heavy chain variable region comprises a HCDR1, a HCDR2 and a HCDR3 with amino acid sequences as set forth in SEQ ID NOs: 9, 21 and 35, respectively; or,

in the protein functional region B, the light chain variable region comprises a LCDR1, a LCDR2 and a LCDR3 with amino acid sequences as set forth in SEQ ID NOs: 46, 53 and 63, respectively, and the heavy chain variable region comprises a HCDR1, a HCDR2 and a HCDR3 with amino acid sequences as set forth in SEQ ID NOs: 8, 20 and 34, respectively; in the protein functional region A, the light chain variable region comprises a LCDR1, a LCDR2 and a LCDR3 with amino acid sequences as set forth in SEQ ID NOs: 47, 54 and 67, respectively, and the heavy chain variable region comprises a HCDR1, a HCDR2 and a HCDR3 with amino acid sequences as set forth in SEQ ID NOs: 9, 23 and 35, respectively; or,

in the protein functional region B, the light chain variable region comprises a LCDR1, a LCDR2 and a LCDR3 with amino acid sequences as set forth in SEQ ID NOs: 46, 53 and 63, respectively, and the heavy chain variable region comprises a HCDR1, a HCDR2 and a HCDR3 with amino acid sequences as set forth in SEQ ID NOs: 8, 20 and 34, respectively; in the protein functional region A, the light chain variable region comprises a LCDR1, a LCDR2 and a LCDR3 with amino acid sequences as set forth in SEQ ID NOs: 47, 54 and 67, respectively, and the heavy chain variable region comprises a HCDR1, a HCDR2 and a HCDR3 with amino acid sequences as set forth in SEQ ID NOs: 9, 24 and 35, respectively; or,

in the protein functional region B, the light chain variable region comprises a LCDR1, a LCDR2 and a LCDR3 with amino acid sequences as set forth in SEQ ID NOs: 46, 53 and 63, respectively, and the heavy chain variable region comprises a HCDR1, a HCDR2 and a HCDR3 with amino acid sequences as set forth in SEQ ID NOs: 8, 20 and 34, respectively; in the protein functional region A, the light chain variable region comprises a LCDR1, a LCDR2 and a LCDR3 with amino acid sequences as set forth in SEQ ID NOs: 48, 54 and 65, respectively, and the heavy chain variable region comprises a HCDR1, a HCDR2 and a HCDR3 with amino acid sequences as set forth in SEQ ID NOs: 11, 22 and 36, respectively; or,

in the protein functional region B, the light chain variable region comprises a LCDR1, a LCDR2 and a LCDR3 with amino acid sequences as set forth in SEQ ID NOs: 46, 53 and 63, respectively, and the heavy chain variable region comprises a HCDR1, a HCDR2 and a HCDR3 with amino acid sequences as set forth in SEQ ID NOs: 10, 20 and 34, respectively; in the protein functional region A, the light chain variable region comprises a LCDR1, a LCDR2 and a LCDR3 with amino acid sequences as set forth in SEQ ID NOs: 47, 54 and 64, respectively, and the heavy chain variable region comprises a HCDR1, a HCDR2 and a HCDR3 with amino acid sequences as set forth in SEQ ID NOs: 9, 21 and 35, respectively; or,

in the protein functional region B, the light chain variable region comprises a LCDR1, a LCDR2 and a LCDR3 with amino acid sequences as set forth in SEQ ID NOs: 46, 53 and 63, respectively, and the heavy chain variable region comprises a HCDR1, a HCDR2 and a HCDR3 with amino acid sequences as set forth in SEQ ID NOs: 10, 20 and 34, respectively; in the protein functional region A, the light chain variable region comprises a LCDR1, a LCDR2 and a LCDR3 with amino acid sequences as set forth in SEQ ID NOs: 47, 54 and 67, respectively, and the heavy chain variable region comprises a HCDR1, a HCDR2 and a HCDR3 with amino acid sequences as set forth in SEQ ID NOs: 9, 23 and 35, respectively; or

in the protein functional region B, the light chain variable region comprises a LCDR1, a LCDR2 and a LCDR3 with amino acid sequences as set forth in SEQ ID NOs: 46, 53 and 63, respectively, and the heavy chain variable region comprises a HCDR1, a HCDR2 and a HCDR3 with amino acid sequences as set forth in SEQ ID NOs: 10, 20 and 34, respectively; in the protein functional region A, the light chain variable region comprises a LCDR1, a LCDR2 and a LCDR3 with amino acid sequences as set forth in SEQ ID NOs: 47, 54 and 67, respectively, and the heavy chain variable region comprises a HCDR1, a HCDR2 and a HCDR3 with amino acid sequences as set forth in SEQ ID NOs: 9, 24 and 35, respectively; or,

in the protein functional region B, the light chain variable region comprises a LCDR1, a LCDR2 and a LCDR3 with amino acid sequences as set forth in SEQ ID NOs: 46, 53 and 63, respectively, and the heavy chain variable region comprises a HCDR1, a HCDR2 and a HCDR3 with amino acid sequences as set forth in SEQ ID NOs: 10, 20 and 34, respectively; in the protein functional region A, the light chain variable region comprises a LCDR1, a LCDR2 and a LCDR3 with amino acid sequences as set forth in SEQ ID NOs: 48, 54 and 65, respectively, and the heavy chain variable region comprises a HCDR1, a HCDR2 and a HCDR3 with amino acid sequences as set forth in SEQ ID NOs: 11, 22 and 36, respectively.

In the bispecific antibody of the present invention, the protein functional region B comprises a light chain variable region and a heavy chain variable region, and the protein functional region A comprises a light chain variable region and a heavy chain variable region; wherein,

in the protein functional region B, the light chain variable region comprises an amino acid sequence as set forth in SEQ ID NO: 86, and the heavy chain variable region comprises an amino acid sequence as set forth in SEQ ID NO: 76; in the protein functional region A, the light chain variable region comprises an amino acid sequence as set forth in SEQ ID NO: 87, and the heavy chain variable region comprises an amino acid sequence as set forth in SEQ ID NO: 77; or,

in the protein functional region B, the light chain variable region comprises an amino acid sequence as set forth in SEQ ID NO: 86, and the heavy chain variable region comprises an amino acid sequence as set forth in SEQ ID NO: 76; in the protein functional region A, the light chain variable region comprises an amino acid sequence as set forth in SEQ ID NO: 90, and the heavy chain variable region comprises an amino acid sequence as set forth in SEQ ID NO: 80; or,

in the protein functional region B, the light chain variable region comprises an amino acid sequence as set forth in SEQ ID NO: 86, and the heavy chain variable region comprises an amino acid sequence as set forth in SEQ ID NO: 76; in the protein functional region A, the light chain variable region comprises an amino acid sequence as set forth in SEQ ID NO: 90, and the heavy chain variable region comprises an amino acid sequence as set forth in SEQ ID NO: 81; or,

in the protein functional region B, the light chain variable region comprises an amino acid sequence as set forth in SEQ ID NO: 86, and the heavy chain variable region comprises an amino acid sequence as set forth in SEQ ID NO: 76; in the protein functional region A, the light chain variable region comprises an amino acid sequence as set forth in SEQ ID NO: 91, and the heavy chain variable region comprises an amino acid sequence as set forth in SEQ ID NO: 79; or,

in the protein functional region B, the light chain variable region comprises an amino acid sequence as set forth in SEQ ID NO: 86, and the heavy chain variable region comprises an amino acid sequence as set forth in SEQ ID NO: 78; in the protein functional region A, the light chain variable region comprises an amino acid sequence as set forth in SEQ ID NO: 87, and the heavy chain variable region comprises an amino acid sequence as set forth in SEQ ID NO: 77; or,

in the protein functional region B, the light chain variable region comprises an amino acid sequence as set forth in SEQ ID NO: 86, and the heavy chain variable region comprises an amino acid sequence as set forth in SEQ ID NO: 78; in the protein functional region A, the light chain variable region comprises an amino acid sequence as set forth in SEQ ID NO: 90, and the heavy chain variable region comprises an amino acid sequence as set forth in SEQ ID NO: 80; or,

in the protein functional region B, the light chain variable region comprises an amino acid sequence as set forth in SEQ ID NO: 86, and the heavy chain variable region comprises an amino acid sequence as set forth in SEQ ID NO: 78; in the protein functional region A, the light chain variable region comprises an amino acid sequence as set forth in SEQ ID NO: 90, and the heavy chain variable region comprises an amino acid sequence as set forth in SEQ ID NO: 81; or,

in the protein functional region B, the light chain variable region comprises an amino acid sequence as set forth in SEQ ID NO: 86, and the heavy chain variable region comprises an amino acid sequence as set forth in SEQ ID NO: 78; in the protein functional region A, the light chain variable region comprises an amino acid sequence as set forth in SEQ ID NO: 91, and the heavy chain variable region comprises an amino acid sequence as set forth in SEQ ID NO: 79; or,

in the protein functional region B, the light chain variable region comprises an amino acid sequence as set forth in SEQ ID NO: 86, and the heavy chain variable region comprises an amino acid sequence as set forth in SEQ ID NO: 84; in the protein functional region A, the light chain variable region comprises an amino acid sequence as set forth in SEQ ID NO: 91, and the heavy chain variable region comprises an amino acid sequence as set forth in SEQ ID NO: 79.

In some specific embodiments, for the bispecific antibody as described above, the bispecific antibody is selected from the group consisting of:

(1) a polypeptide chain-1 of structure n'-[VL]-CL-c', a polypeptide chain-2 of structure n'-[VH]-CH1-h-CH2-CH3-c', and a polypeptide chain-3 of structure n'-{VH-L-VL}-h-CH2-CH3-c' or n'-{VL-L-VH}-h-CH2-CH3-c', wherein the polypeptide chain-1 and the polypeptide chain-2 are protein functional regions A and the polypeptide chain-3 is a protein functional region B, or the polypeptide chain-1 and the polypeptide chain-2 are protein functional regions B and the polypeptide chain-3 is a protein functional region A;

(2) a polypeptide chain-1 of structure n'-[VL]-CL-c', a polypeptide chain-2 of structure n'-[VH]-CH1-h-CH2-CH3-c', a polypeptide chain-3 of structure n'-{VH}-CH1-h-CH2-CH3-c' and a polypeptide chain-4 of structure n'-{VL}-CL-c', or a polypeptide chain-1 of structure n'-[VH]-CH-c', a polypeptide chain-2 of structure n'-[VL]-CL-h-CH2-CH3-c', a polypeptide chain-3 of structure n'-{VH}-CH1-h-CH2-CH3-c' and a polypeptide chain-4 of structure n'-{VL}-CL-c', wherein the polypeptide chain-1 and the polypeptide chain-2 are protein functional regions A, and the polypeptide chain-3 and the polypeptide chain-4 are protein functional regions B, or the polypeptide chain-1 and the polypeptide chain-2 are protein functional regions B, and the polypeptide chain-3 and the polypeptide chain-4 are protein functional regions A;

(3) a polypeptide chain-1 of structure n'-[VH]-CH1-c', a polypeptide chain-2 of structure n'- {VH}-CH1-L-[VL]-CL-h-CH2-CH3-c' or n'- {VH}-CH1-h-CH2-CH3-L-[VL]-CL-c', a polypeptide chain-3 of structure n'-{VH}-CH1-h-CH2-CH3-c' and a polypeptide chain-4 of structure n'-{VL}-CL-c', wherein the polypeptide chain-1 is a protein functional region A, the polypeptide chain-2 sequentially comprises a protein functional region A and a protein functional region B from n' to c' or sequentially comprises a protein functional region B and a protein functional region A from n' to c', and the polypeptide chain-3 and the polypeptide chain-4 are protein functional regions B; or the polypeptide chain-1 is a protein functional region B, the polypeptide chain-2 sequentially comprises a protein functional region B and a protein functional region A from n' to c' or sequentially comprises a protein functional region A and a protein functional region B from n' to c', and the polypeptide chain-3 and the polypeptide chain-4 are protein functional regions A;

(4) a polypeptide chain-1 of structure n'-[VH]-CH1-L1-{VL-L2-VH}-h-CH2-CH3-c' or n'-[VH]-CH1-L1-{VH-L2-VL}-h-CH2-CH3-c', a polypeptide chain-2 of structure n'-[VH]-CH1-h-CH2-CH3-c' and a polypeptide chain-3 of structure n'-[VL]-CL-c', wherein the polypeptide chain-1 comprises a protein functional region A and a protein functional region B, and the polypeptide chain-2 and the polypeptide chain-3 are protein functional regions B, or the polypeptide chain-1 comprises a protein functional region A and a protein functional region B, and the polypeptide chain-2 and the polypeptide chain-3 are protein functional regions A; preferably, the polypeptide chain-1 sequentially comprises a protein functional region B and a protein functional region A from n' to c' or sequentially comprises a protein functional region A and a protein functional region B from n' to c'; and

(5) a polypeptide chain-1 of structure n'-[VH]-CH1-h-CH2-CH3-Ll1{VL-L2-VH}-c' or n'-[VH]-CH1-h-CH2-CH3-L1-{VH-L2-VL}-c', a polypeptide chain-2 of structure n'-[VH]-CH1-h-CH2-CH3-c' and a polypeptide chain-3 of structure n'-[VL]-CL-c', wherein the polypeptide chain-1 comprises a protein functional region A and a protein functional region B, and the polypeptide chain-2 and the polypeptide chain-3 are protein functional regions B, or the polypeptide chain-1 comprises a protein functional region A and a protein functional region B, and the polypeptide chain-2 and the polypeptide chain-3 are protein functional regions A.

Among them, n' represents the amino-terminus (also designated N-terminus) of the polypeptide chain, c' represents the carboxyl-terminus (also designated C-terminus) of the polypeptide chain, h represents the hinge region, L, L1 or L2 represents a linker, and a suitable linker (L) in the prior art consists of a repeating $G_4S$ amino acid sequence or a variant thereof. For example, linkers with the amino acid sequence $(G4S)_4$ or $(G_4S)_3$ can be used, and variants thereof can also be used, e.g., one of the G of $G_4S$ is substituted with Q, e.g., the second or third G is substituted with Q. The preferred linker sequences of the present invention are set forth in SEQ ID NOs: 133-135. "-" represents a polypeptide bond linking different structural regions or is used to separate different structural regions.

In the present invention, the "[ ]" and "{ }" represent different functional regions or structures, respectively; for example, {VL-L-VH} and {VH-L-VL} represent the scFv structure, and [VH]/ {VH} and [VL]/ {VL} represent the heavy chain variable region and the light chain variable region of the Fab structure, respectively. When VH is protein functional region A or protein functional region B, it can also be expressed as VH_A (i.e., heavy chain variable region is protein functional region A) or VH_B (i.e., heavy chain variable region is protein functional region B); similarly, when VL is protein functional region A or protein functional region B, it can also be expressed as VL_A (i.e., light chain variable region is protein functional region A) or VL_B (i.e., light chain variable region is protein functional region B). In FIG. 28, "[ ]" and "{ }" are removed for convenience of drawing, so that VL_B-L-VH_A (light chain variable region is protein functional region B, heavy chain variable region is protein functional region A, and L is a linker linking VL_B and VH_A), VH_B-L-VL_A (heavy chain variable region is protein functional region B, light chain variable region is protein functional region A, and L is a linker linking VH_B and VL_A, see, e.g., polypeptide chain-3 of structure (1)), etc. also represent the scFv structure, while separate VL_B and VH_A (see, e.g., polypeptide chain-1 or polypeptide chain-2 of structure (1)) still represent the light chain variable region of the protein functional region B or the heavy chain variable region of the protein functional region A in the Fab structure, respectively. In the present invention, polypeptide chain-1, -2, -3 or -4 represents only the type of polypeptide chains, and the number of each polypeptide chain may be 1 or 2 when the bispecific antibody is actually composed of polypeptide chains, for example, in structures (5) and (6), the number of polypeptide chain-1, -2 or -3 is 1, and the number of polypeptide chain-4 is 2; in structures (7), (8), (9) and (10), the number of polypeptide chain-1 or -2 is 1, and the number of polypeptide chain-3 is 2. In the present invention, the polypeptide chain-1 is also referred to as a first polypeptide chain, the polypeptide chain-2 is also referred to as a second polypeptide chain, the polypeptide chain-3 is also referred to as a third polypeptide chain, and the polypeptide chain-4 is also referred to as a fourth polypeptide chain.

In some preferred embodiments, the bispecific antibody is selected from the group consisting of:

(1) the bispecific antibody comprises three polypeptide chains, wherein a first polypeptide chain comprises an amino acid sequence as set forth in SEQ ID NO: 109; a second polypeptide chain comprises an amino acid sequence as set forth in SEQ ID NO: 118; a third polypeptide chain comprises an amino acid sequence as set forth in SEQ ID NO: 119; or,

a first polypeptide chain comprises an amino acid sequence as set forth in SEQ ID NO: 114; a second polypeptide chain comprises an amino acid sequence as set forth in SEQ ID NO: 120; a third polypeptide chain comprises an amino acid sequence as set forth in SEQ ID NO: 119; or,
a first polypeptide chain comprises an amino acid sequence as set forth in SEQ ID NO: 114; a second polypeptide chain comprises an amino acid sequence as set forth in SEQ ID NO: 121; a third polypeptide chain comprises an amino acid sequence as set forth in SEQ ID NO: 119; or,
a first polypeptide chain comprises an amino acid sequence as set forth in SEQ ID NO: 115; a second polypeptide chain comprises an amino acid sequence as set forth in SEQ ID NO: 122; a third polypeptide chain comprises an amino acid sequence as set forth in SEQ ID NO: 119; or,
a first polypeptide chain comprises an amino acid sequence as set forth in SEQ ID NO: 126; a second polypeptide chain comprises an amino acid sequence as set forth in SEQ ID NO: 118; a third polypeptide chain comprises an amino acid sequence as set forth in SEQ ID NO: 109; or,
a first polypeptide chain comprises an amino acid sequence as set forth in SEQ ID NO: 127; a second polypeptide chain comprises an amino acid sequence as set forth in SEQ ID NO: 118; a third polypeptide chain comprises an amino acid sequence as set forth in SEQ ID NO: 109; or,
a first polypeptide chain comprises an amino acid sequence as set forth in SEQ ID NO: 110; a second polypeptide chain comprises an amino acid sequence as set forth in SEQ ID NO: 128; a third polypeptide chain comprises an amino acid sequence as set forth in SEQ ID NO: 129; or,
a first polypeptide chain comprises an amino acid sequence as set forth in SEQ ID NO: 110; a second polypeptide chain comprises an amino acid sequence as set forth in SEQ ID NO: 130; a third polypeptide chain comprises an amino acid sequence as set forth in SEQ ID NO: 129;

(2) the bispecific antibody comprises four polypeptide chains, wherein a first polypeptide chain comprises an amino acid sequence as set forth in SEQ ID NO: 97; a second polypeptide chain comprises an amino acid sequence as set forth in SEQ ID NO: 123; a third polypeptide chain comprises an amino acid sequence as set forth in SEQ ID NO: 118; a fourth polypeptide chain comprises an amino acid sequence as set forth in SEQ ID NO: 109; or,

a first polypeptide chain comprises an amino acid sequence as set forth in SEQ ID NO: 97; a second polypeptide chain comprises an amino acid sequence as set forth in SEQ ID NO: 123; a third polypeptide chain comprises an amino acid sequence as set forth in SEQ ID NO: 120; a fourth polypeptide chain comprises an amino acid sequence as set forth in SEQ ID NO: 114; or,

a first polypeptide chain comprises an amino acid sequence as set forth in SEQ ID NO: 97; a second polypeptide chain comprises an amino acid sequence as set forth in SEQ ID NO: 123; a third polypeptide chain comprises an amino acid sequence as set forth in SEQ ID NO: 121; a fourth polypeptide chain comprises an amino acid sequence as set forth in SEQ ID NO: 114; or,

a first polypeptide chain comprises an amino acid sequence as set forth in SEQ ID NO: 97; a second polypeptide chain comprises an amino acid sequence as set forth in SEQ ID NO: 123; a third polypeptide chain comprises an amino acid sequence as set forth in SEQ ID NO: 122; a fourth polypeptide chain comprises an amino acid sequence as set forth in SEQ ID NO: 115; or,

a first polypeptide chain comprises an amino acid sequence as set forth in SEQ ID NO: 97; a second polypeptide chain comprises an amino acid sequence as set forth in SEQ ID NO: 124; a third polypeptide chain comprises an amino acid sequence as set forth in SEQ ID NO: 118; a fourth polypeptide chain comprises an amino acid sequence as set forth in SEQ ID NO: 109; or,

a first polypeptide chain comprises an amino acid sequence as set forth in SEQ ID NO: 97; a second polypeptide chain comprises an amino acid sequence as set forth in SEQ ID NO: 125; a third polypeptide chain comprises an amino acid sequence as set forth in SEQ ID NO: 118; a fourth polypeptide chain comprises an amino acid sequence as set forth in SEQ ID NO: 109.

The protein functional region of the present invention may be Fab, scFv or VH in some cases, or $F(ab)_2$ or a full-length antibody in other cases, and is also referred to as an antibody, an antigen-binding protein or a binding protein in certain cases. In the present invention, an "Fab structure" or "Fab fragment" consists of one light chain and CH1 and the variable region of one heavy chain. The heavy chain of an Fab fragment cannot form a disulfide bond with another Fab heavy chain molecule. An "Fc" region contains two heavy chain fragments comprising the CH2 and CH3 domains of the antibody; the two heavy chain fragments are held together by two or more disulfide bonds and by the hydrophobic interaction of the CH3 domain. A "Fab' fragment" contains one light chain and part of one heavy chain comprising the VH domain and the CH1 domain and the region between the CH1 and CH2 domains, such that interchain disulfide bonds can be formed between the two heavy chains of two Fab' fragments to provide an $F(ab')_2$ fragment. A "$F(ab')_2$ fragment" contains two light chains and two heavy chains comprising part of the constant region between the CH1 and CH2 domains, such that interchain disulfide bonds are formed between the two heavy chains. Thus, an $F(ab')_2$ fragment consists of two Fab' fragments held together by disulfide bonds between the two heavy chains. The term "Fv" refers to an antibody fragment consisting of the VL and VH domains of a single arm of an antibody, but lacks the constant region.

In the present invention, the scFv (single chain antibody fragment) may be a conventional single chain antibody in the art, which comprises a heavy chain variable region, a light chain variable region, and a short peptide of 15-20 amino acids. The VL and VH domains are paired to form a monovalent molecule by a linker that enables them to be produced as a single polypeptide chain. Such scFv molecules may have a general structure: NH2-VL-linker-VH-COOH or NH2-VH-linker-VL-COOH or is expressed as n'-VL-L-VH-c' or n'-VH-L-VL-c'.

To solve the above technical problem, the third technical solution of the present invention is as follows: provided is a chimeric antigen receptor comprising the antibody according to the first technical solution of the present invention or the bispecific antibody according to the second technical solution of the present invention.

In the present application, the antibody or the bispecific antibody may be used to prepare a chimeric antigen receptor (CAR) or the like so as to modify it onto cells such as T cells or NK cells. The chimeric antigen receptor may be one that is conventional in the art, including, for example, one that utilizes the above antibody in the form of an scFv as an extracellular antigen-binding domain.

Therefore, to solve the above technical problem, a fourth technical solution of the present invention is as follows: provided is a genetically modified cell comprising the antibody according to the first technical solution of the present invention; the cell is preferably a eukaryotic cell, more preferably an isolated human cell, and even more preferably an immune cell such as a T cell (e.g., in the form of CAR-T), or an NK cell such as an NK92 cell line.

To solve the above technical problem, a fifth technical solution of the present invention is as follows: provided is an isolated nucleic acid encoding the antibody or the bispecific antibody as described above or the chimeric antigen receptor according to the third technical solution of the present invention.

The preparation method for the nucleic acid is a conventional preparation method in the art, and preferably comprises the following steps: obtaining a nucleic acid molecule encoding the above antibody by gene cloning technology, or obtaining a nucleic acid molecule encoding the above antibody by artificial complete sequence synthesis.

It is known to those skilled in the art that substitutions, deletions, alterations, insertions or additions may be appropriately introduced into the base sequence encoding the amino acid sequence of the above antibody to provide a polynucleotide homologue. The polynucleotide homologue of the present invention may be produced by substituting, deleting or adding one or more bases of a gene encoding the antibody sequence within a range in which the activity of the antibody is maintained.

To solve the above technical problem, a sixth technical solution of the present invention is as follows: provided is an expression vector comprising the isolated nucleic acid as described above.

The recombinant expression vector may be obtained by using conventional methods in the art, i.e., by linking the nucleic acid molecule of the present application to various expression vectors. The expression vector is any conventional vector in the art, provided that it can carry the aforementioned nucleic acid molecule.

Preferably, the expression vector comprises a eukaryotic cell expression vector and/or a prokaryotic cell expression vector.

To solve the above technical problem, a seventh technical solution of the present invention is as follows: provided is a transformant comprising the isolated nucleic acid or the expression vector as described above.

The transformant may be prepared by using conventional methods in the art, e.g., by transforming the above recombinant expression vector into a host cell. The host cell of the transformant is any conventional host cell in the art, provided that it can enable the stable replication of the above recombinant expression vector and the nucleic acid carried can be efficiently expressed. Preferably, the host cell is a prokaryotic/eukaryotic cell, wherein the prokaryotic cell is preferably an *E. coli* cell such as TG1 and BL21 (expressing a single chain antibody or Fab antibody), and the eukaryotic cell is preferably an HEK293 cell or a CHO cell (expressing a full-length IgG antibody). The preferred recombinant expression transformant of the present invention can be obtained by transforming the aforementioned recombinant expression plasmid into a host cell. The transformation method is a conventional transformation method in the art, preferably a chemical transformation method, a heat shock method or an electric transformation method.

To solve the above technical problem, an eighth technical solution of the present invention is as follows: provided is a method for preparing a B7H4-targeting antibody or bispecific antibody comprising the following steps: culturing the transformant according to the seventh technical solution of the present invention, and obtaining the B7H4-targeting antibody or bispecific antibody from the culture.

To solve the above technical problem, a ninth technical solution of the present invention is as follows: provided is an antibody-drug conjugate comprising an antibody moiety and a conjugate moiety, wherein the antibody moiety comprises the antibody according to the first technical solution of the present invention and the bispecific antibody according to the second technical solution of the present invention, and the conjugate moiety includes, but is not limited to, a detectable label, a drug, a toxin, a cytokine, a radionuclide, an enzyme, or a combination thereof, the antibody moiety and the conjugate moiety are conjugated via a chemical bond or a linker.

To solve the above technical problem, a tenth technical solution of the present invention is as follows: provided is a pharmaceutical composition comprising the antibody according to the first technical solution of the present invention or the bispecific antibody according to the second technical solution of the present invention and optionally a pharmaceutically acceptable carrier. Preferably, the pharmaceutical composition further comprises a pharmaceutically acceptable carrier.

More preferably, the pharmaceutical composition further comprises other anti-tumor antibodies as an active ingredient.

The pharmaceutically acceptable carrier may be a carrier conventional in the art, and the carrier may be any suitable physiologically or pharmaceutically acceptable auxiliary material. The pharmaceutically acceptable auxiliary material is one conventional in the art, and preferably comprises a pharmaceutically acceptable excipient, a filler, a diluent, or the like. More preferably, the pharmaceutical composition comprises 0.01%-99.99% of the above antibody and/or the bispecific antibody, and 0.01%-99.99% of a pharmaceutically acceptable carrier, the percentage being the mass percentage of the pharmaceutical composition.

The route of administration for the pharmaceutical composition of the present invention is preferably parenteral administration, injection administration or oral administration. The injection administration preferably includes intravenous injection, intramuscular injection, intraperitoneal injection, intradermal injection or subcutaneous injection. The pharmaceutical composition is in any conventional dosage form in the art, preferably in the form of a solid, semisolid or liquid, i.e., it may be an aqueous solution, a non-aqueous solution or a suspension, more preferably a tablet, capsule, granule, injection, infusion, or the like. More preferably, it is administered intravascularly, subcutaneously, intraperitoneally or intramuscularly. Preferably, the pharmaceutical composition may also be administered as an aerosol or a coarse spray, i.e., administered nasally; or administered intrathecally, intramedullarily or intraventricularly. More preferably, the pharmaceutical composition may also be administered transdermally, percutaneously, topically, enterally, intravaginally, sublingually or rectally. The pharmaceutical composition of the present invention may be formulated into various dosage forms as required, and can be administered by a physician in the light of the patient's type, age, weight, and general disease state, route of administration, etc. The administration may be performed, for example, by injection or other therapeutic modalities.

The dosage level at which the pharmaceutical composition of the present invention is administered can be adjusted depending on the amount of the composition to achieve the desired diagnostic or therapeutic outcome. The dosage regimen may also be a single injection or multiple injections, or an adjusted one. The selected dosage level and regimen is appropriately adjusted depending on a variety of factors including the activity and stability (i.e., half-life) of the pharmaceutical composition, the formulation, the route of administration, combination with other drugs or treatments, the disease or disorder to be detected and/or treated, and the health condition and past medical history of the subject to be treated.

A therapeutically effective dose for the pharmaceutical composition of the present invention may be estimated initially in cell culture experiments or animal models such as rodents, rabbits, dogs, pigs and/or primates. Animal models can also be used to determine the appropriate concentration range and route of administration, and subsequently an effective dose and a route of administration in humans. In general, the determination of and adjustment to the effective amount or dose to be administered and the assessment of when and how to make such adjustments are known to those skilled in the art.

For combination therapy, the above antibody, the bispecific antibody, and/or additional therapeutic or diagnostic agents may each be used as a single agent for use within any time frame suitable for performing the intended treatment or diagnosis. Thus, these single agents may be administered substantially simultaneously (i.e., as a single formulation or within minutes or hours) or sequentially.

For additional guidance regarding formulations, doses, dosage regimens, and measurable therapeutic outcomes, see Berkow et al. (2000) The Merck Manual of Medical Information and Merck & Co. Inc., Whitehouse Station, New Jersey; Ebadi (1998) CRC Desk Reference of Clinical Pharmacology*,* etc.

To solve the above technical problem, an eleventh technical solution of the present invention is as follows: provided is use of the antibody according to the first technical solution of the present invention, the bispecific antibody according to the second technical solution of the present invention, the chimeric antigen receptor according to the third technical solution of the present invention, the genetically modified cell according to the fourth technical solution of the present invention, the antibody-drug conjugate according to the ninth technical solution of the present invention or the pharmaceutical composition according to the tenth technical solution of the present invention in the manufacture of a medicament, a kit and/or an administration device for the treatment and/or prevention of a cancer.

Preferably, the cancer is a B7H4 positive tumor; the tumor is preferably breast cancer, ovarian cancer and endometrioma, the breast cancer being more preferably triple negative breast cancer.

To solve the above technical problem, a twelfth technical solution of the present invention is as follows: provided is a method for the detection of B7H4 in a sample, comprising conducting detection using the antibody or the bispecific antibody as described above. Preferably, the method is for non-diagnostic purposes.

The treatment or detection method for non-diagnostic purposes of the present invention includes, but is not limited to: screening of laboratory drugs, research of preventive medicine and formulation of public health policies, detection using kits, and the like. As known to those skilled in the art, modern medicine is divided into two parts: preventive medicine and clinical medicine. "The detection method for non-diagnosis purposes" of the present invention can detect samples (including human body secretion) collected in the environment in preventive medicine and determine whether the environment is polluted with an antigen. In the laboratory, laboratory reagents can also be detected by researchers using the antibody or the bispecific antibody of the present invention to ensure that the antigens used in experiments are not polluted with antigens other than B7H4 for further use in screening of new antibodies or screening of small molecule compounds as drug targets, etc.

To solve the above technical problem, a thirteen technical solution of the present invention is as follows: provided is a kit comprising the antibody according to the first technical solution of the present invention, the bispecific antibody according to the second technical solution of the present invention, the chimeric antigen receptor according to the third technical solution of the present invention, the genetically modified cell according to the fourth technical solution of the present invention, the antibody-drug conjugate according to the ninth technical solution of the present invention, and/or the pharmaceutical composition according to the tenth technical solution of the present invention, and optionally instructions for use.

To solve the above technical problem, a fourteen technical solution of the present invention is as follows: provided is an administration device comprising: (1) an infusion module for administering to a subject in need thereof the pharmaceutical composition according to the tenth technical solution of the present invention, and (2) optionally a pharmacodynamic monitoring module.

To solve the above technical problem, a fifteenth technical solution of the present invention is as follows: provided is use of the antibody according to the first technical solution of the present invention, the bispecific antibody according to the second technical solution of the present invention, the chimeric antigen receptor according to the third technical solution of the present invention, the genetically modified cell according to the fourth technical solution of the present invention, the antibody-drug conjugate according to the ninth technical solution of the present invention, and/or the pharmaceutical composition according to the tenth technical solution of the present invention in the diagnosis, prevention and/or treatment of a tumor. Preferably, the tumor is as described according to the eighth technical solution of the present invention.

To solve the above technical problem, a sixteenth technical solution of the present invention is as follows: provided is a kit of parts comprising a kit A and a kit B, wherein the kit A comprises the antibody according to the first technical solution of the present invention, the bispecific antibody according to the second technical solution of the present invention, the chimeric antigen receptor according to the third technical solution of the present invention, the genetically modified cell according to the fourth technical solution of the present invention, the antibody-drug conjugate according to the ninth technical solution of the present invention, and/or the pharmaceutical composition according to the tenth technical solution of the present invention, and the kit B comprises another anti-tumor antibody or a pharmaceutical composition comprising the another anti-tumor antibody. The kit A and the kit B may be used simultaneously, or the kit A may be used prior to the use of the kit B, or the kit B may be used prior to the use of the kit A. The sequence of use can be determined according to actual requirements in a specific application.

The three-letter codes and single-letter codes for amino acids used in the present application are known to those skilled in the art, or are described in J. Biol. Chem, 243, p3558 (1968). As used herein, the term "include/includes/including" or "comprise/comprises/comprising" is intended to mean that a composition and a method include the elements described but does not exclude other elements; but the case of "consist/consists/consisting of" is also included as the context dictates. Unless otherwise specifically stated in the content, the term "or" is used in the present invention to mean, and is interchangeable with, the term "and/or". The "about" and "approximately" shall generally mean an acceptable degree of error in the measured quantity in view of the nature or accuracy of the measurement. Exemplary degrees of error are typically within 10% thereof and more typically within 5% thereof or even within 2% or 1% thereof. As used herein, the term $EC_{50}$ refers to the concentration for 50% of maximal effect, i.e., the concentration that can cause 50% of the maximal effect.

The above preferred conditions may be combined arbitrarily to obtain preferred embodiments of the present invention on the basis of the general knowledge in the art.

The reagents and starting materials used in the present invention are commercially available.

The beneficial effects of the present invention are as follows:

1. The B7H4-targeting monoclonal antibody of the present invention is a naturally-occurring fully-human antibody with a binding activity to human B7H4 and cynomolgus monkey B7H4, without cross-reactivity with other B7 family members. After Fc modification, the B7H4-targeting monoclonal antibody has stronger ADCC effect, and shows good anti-tumor activity in the *in vivo* experiment. PR003369 has higher T cell activation activity and higher internalization activity after affinity maturation modification, and thus is more suitable for serving as an ADC drug.
2. The B7H4×CD3 bispecific antibody of the present invention has a bispecific antibody structure with a human Fc fragment, retains the binding effect of the Fc to an FcRn, and thus has a longer half-life. The B7H4 end are in the form of ScFv, which reduces the mismatching of light and heavy chains and retains good stability and hydrophilicity. The CD3-end activity is optimized, an anti-CD3 antibody with moderate strength is adopted, and thus the toxicity is reduced on the premise of ensuring the efficacy. The antibodies of B7H4 and CD3 ends have good activity of binding to cynomolgus monkeys.

## BRIEF DESCRIPTION OF THE DRAWINGS

FIG. 1 shows the results of the initial antibodies binding to human B7H4 on the cell surface;
FIG. 2 shows the results of expression supernatants of PR001476 and PR002037 and their PTM variants/DE mutant antibodies binding to human B7H4 on the cell surface;
FIG. 3 shows the results of the initial antibodies binding to cynomolgus monkey B7H4 on the cell surface;
FIG. 4 shows the results of PR001476 and PR002037 and their PTM variants/DE mutant antibodies binding to cynomolgus monkey B7H4 on the cell surface;
FIG. 5 shows the results of the initial antibodies binding to mouse B7H4 on the cell surface;
FIG. 6 shows the results of PR001476 and PR002037 and their PTM variants/DE mutant antibodies binding to mouse B7H4 on the cell surface;
FIG. 7 shows the results of the initial antibodies binding to B7H4 on the surface of tumor cells SK-BR-3;
FIG. 8 shows the results of expression supernatants of PR001476 and PR002037 and their PTM variants/DE mutant antibodies binding to B7H4 on the surface of tumor cells SK-BR-3;

FIG. 9 shows the results of PR001476 and PR002037 and their PTM variants/DE mutant antibodies binding to B7H4 on the surface of tumor cells SK-BR-3;
FIG. 10 shows the results of PR002418 and PR001476 with affinity maturation/DE mutant antibody PR003369 binding to B7H4 on the surface of tumor cells MDA-MB-468;
FIG. 11 shows ADCC killing activity of PR001476 and its PTM variant/DE mutant antibody against tumor cells SK-BR-3;
FIG. 12 shows ADCC killing activity of PR002037 and its PTM variant/DE mutant antibody against tumor cells SK-BR-3;
FIG. 13 shows the comparison of ADCC killing activity of PR002418 and PR002421 against tumor cells SK-BR-3 with the control antibodies from FivePrime;
FIG. 14 shows the comparison of ADCC killing activity of PR003369, PR002418 and RP002421 against tumor cells MDA-MB-468 and HCC-1954 with the control antibody 2 from FivePrime;
FIG. 15 shows the results of the anti-B7H4 antibodies blocking the immunosuppressive signal of B7H4 to activate T cells;
FIG. 16 shows the results of internalization of the anti-B7H4 antibodies on SK-BR-3 cells;
FIG. 17 shows the activity of internalization mediated cytotoxicity of the anti-B7H4 antibodies;
FIG. 18 shows the effect of crosslinking of MMAE groups on the activity of the antibodies binding to B7H4 on the surface of tumor cells;
FIG. 19 shows the effect of crosslinking of MMAE groups on the internalization activity of antibodies on tumor cells;
FIG. 20 shows the killing efficiency against tumor cells of the variant PR003369-ADC with affinity maturation and the control antibody PR000157-ADC;
FIG. 21 shows the analysis of affinity data of antibodies by using Biacore T200 analytical software 2.0;
FIG. 22 shows the cross-reactivity between the anti-B7H4 antibodies and other member proteins of the B7 family;
FIG. 23 shows the stability of anti-B7H4 antibodies PR002418, PR002037 and PR003369 and the control antibody in human serum at 37 °C for 14 days;
FIG. 24 shows the half-life of PR002418 and PR002421 and the control antibody 2 in mice;
FIG. 25 shows the *in vivo* anti-tumor effect of anti-B7H4 monoclonal antibody molecules in BALB/c nude mouse MDA-MB-468 tumor model;
FIG. 26 shows the *in vivo* anti-tumor effect of anti-B7H4 monoclonal antibody molecules in an NSG mouse MDA-MB-468 tumor model with reconstitution of human PBMC immune system;
FIG. 27 shows the expression of B7H4 in normal tissues (A) and tumors (B) and its IHC score statistics (C);
FIG. 28 shows schematic structural diagrams of B7H4×CD3 bispecific antibody molecules;
FIG. 29 shows the results of B7H4×CD3 bispecific antibody molecules binding to SK-BR-3 cells (A-D) and T cells (E-H);
FIG. 30 shows the results of B7H4×CD3 bispecific antibody molecules (A-L, "1 + 1" asymmetric structure; M-N, "2 + 1" asymmetric structure) activating T cells and killing target cells; and
FIG. 31 shows the tumor growth inhibition rate of B7H4×CD3 bispecific antibody molecules in NSG mouse tumor models (A is MDA-MB-468; B is HCC-1954) with reconstitution of human PBMC immune system.

## DETAILED DESCRIPTION

The present invention is further illustrated by the following examples, which are not intended to limit the present invention. Experimental procedures without specified conditions in the following examples are performed in accordance with conventional procedures and conditions, or in accordance with instructions.

### Example 1. Acquisition of Anti-B7H4 Antibody Molecules

Experimental animals, which may be mice, rats, rabbits, sheep and camels, can be immunized with the B7H4 recombinant protein or cells overexpressing B7H4 to obtain antibody molecules specifically binding to B7H4. Typically, the resulting antibody molecules are non-human antibodies. After obtaining non-human antibodies, these molecules need to be humanized by antibody engineering technology to reduce immunogenicity and improve druggability. However, the humanization of antibodies is complex in terms of the technology, and the humanized molecules tend to have reduced affinity for antigens. On the other hand, advances in transgenic technology have made it possible to develop genetically engineered mice that carry a human immunoglobulin immune repertoire and have the endogenous murine immune repertoire deleted. The Harbour H2L2 mice (Harbour Antibodies BV) are transgenic mice that carry human immunoglobulin immune repertoire, and the antibodies generated by the transgenic mice has a fully human sequence, thus eliminating the need for further humanization and greatly improving the efficiency of therapeutic antibody development.

### 1.1 Immunization of mice

Harbour H2L2 mice were subjected to multiple rounds of immunization with a soluble recombinant human B7H4-ECD-mFc fusion protein (Sino Biological, #10738-H05H) as an antigen. The antigenic protein was mixed with an immunoadjuvant to form an immunogenic reagent, which was then injected subcutaneously via the groin or intraperitoneally. In each round of immunization, each mouse received a total injection dose of 100 μL. In the first round of immunization, each mouse received the immunization with an immunogenic reagent prepared by mixing 50 μg of antigenic protein with complete Freund's adjuvant (Sigma, #F5881) in a 1:1 volume ratio. In each subsequent round of booster immunization, each mouse received an immunization with an immunogenic reagent prepared by mixing 25 μg of antigenic protein with Sigma Adjuvant System adjuvant (Sigma, #S6322). The interval between rounds of booster immunization was at least two weeks. In general, there are 6-7 rounds of booster immunizations. The immunization was performed at days 0, 14, 28, 42, 56, 70, 84 and 98; and the antibody titer in serum of mice was measured at days 49 and 77. The last round of booster immunization was performed at a dose of 25 μg of antigenic protein per mouse 5 days before the isolation of H2L2 mouse splenic B cells.

Or plasmids encoding mouse CD40L, after transfected with CHO-K1 cells (CHO-K1/hu B7H4, Harbour BioMed) overexpressing human B7H4, were mixed with an immunoadjuvant to obtain an immunogenic reagent, and the mice were then immunized with $5 \times 10^6$ cells per mouse, the immunization process being the same as protein immunization.

### 1.2 Serum titer assay

At specific time points, the sera of mice were collected, and the titer of antibody binding to B7H4 protein in the sera was determined by the ELISA method and the titer of antibody binding to B7H4-overexpressing cells in the sera was determined by the FACS method.

In the ELISA method, an ELISA plate (corning, 9018) was coated with 1 μg/mL hB7H4-ECD-his protein (Sino Biological, # 10738-H08H) at 100 μL/well and incubated overnight at 4 °C; after 2 rinses, the plate was blocked with 1% BSA in PBST for 2 hours at 37 °C; the plate was added with serially-diluted sera at 100 μL/well and incubated for 1 hour at 37 °C; after 3 rinses, the plate was added with anti-rat-HRP (sigma, # A5795) diluted at 1:5000 at 100 μL/well and incubated for 30 minutes at 37 °C. After 3 rinses, the plate was added with TMB substrate at 100 μL/well and incubated for about 10 minutes, and then added with 1N HCl at 50 μL/well for termination of the color development, and then the absorbance at 450 nm was read (Molecular Devices, Plus 384).

In the FACS method, serially-diluted mouse sera were incubated with HEK293-B7H4 cells for 1 hour at 4 °C; after 2 washes of the cells, a secondary antibody Anti-Rat IgG (H+L) (Life technologies, A1 1006) was added and incubated for 1 hour at 4 °C and after 2 washes, the cells were resuspended and detected by a flow cytometer (BD, Flibur). HEK293 cells served as background controls.

### 1.3 Screening of anti-B7H4 antibodies by hybridoma technology

Immunized mice with high serum titer were selected for one final immunization and then sacrificed. Spleen cells and SP2/0 myeloma cells (ATCC, CRL-1581) were electrofused at a cell ratio of 4:1 with the electrofusion parameters shown as follows: V1: 50V, 11: 15 s, V2: 600 V, t2: 20 μs, t3: 0.5 s, n: 1, t4: 7 s, V+/-: +, and fade: on. The cells were resuspended in a DMEM medium containing 20% FBS and HT at $1 \times 10^5$/100 μL/well. After 24 hours, DMEM containing 20% FBS and 2× HT was added at 100 μL/well for further culturing. The supernatant was subsequently collected and detected for the antibody titer. Generally, 9-15 days after fusion, supernatants of protein-immunized mice were taken and subjected to primary screening with Acumen, and detected for the binding to CHO-K1/huB7H4 cells; supernatants of cell-immunized mice were taken and subjected to primary screening with Mirrorball (SPT Labtech, mirrorball® fluorescence cytometer), and detected for the binding to HEK-293/huB7H4 cells. Positive clones were selected and then confirmed by ELISA and FACS to detect their binding ability to CHO-K1 cell line overexpressing human B7H4 (CHO-K1/huB7H4), CHO-K1 cell line overexpressing cynomolgus monkey B7H4 (CHO-K1/cynoB7H4), and CHO-K1 cell line overexpressing mouse B7H4 (CHO-K1/mB7H4). Positive wells were further subcloned by limiting dilution and further screened by ELISA and FACS methods. Clones with better binding to human and monkey B7H4 are selected for sequencing.

### 1.4. Screening of anti-B7H4 antibodies by *in vitro* cloning technique for B cells

The spleens of the mice were removed, ground and filtered through a 200-mesh filter, and the single cell suspensions were sorted according to the mouse memory B cell sorting kit (Miltenyi, #130-095-838). The sorted cells were subjected to immunofluorescence staining.

B200 positive cells (BioLegend, #103227), IgM negative cells (BioLegend, #406506) and B7H4 specific positive cells (BioLegend, #405207) were sorted using a flow cell sorter S3e. The cells obtained by sorting were cultured in a 96-well cell culture plate at a density of 5 cells per well, and irradiated EL4 cells were previously plated on the cell culture plate as feeder cells.

After 14 days of culture, culture supernatants were collected and subjected to ELISA assay, and for wells having binding activity to B7H4 protein, cells were taken and subjected to RT-PCR (SMART-Seq v4 Ultra Low Input RNA Kit for Sequencing (#634892), 1-5™ 2 × High-Fidelity Master Mix (#I5HM-5000)). The light and heavy chains obtained by amplification were spliced into an scFv by overlap PCR and expressed in *E.coli,* the expression supernatants were subjected to ELISA assay, and the positive clones were sequenced.

### 1.5. Sequence analysis and sequence optimization of anti-B7H4 antibodies

The nucleotide sequences encoding the variable domains of the antibody molecules and the corresponding amino acid sequences were obtained through conventional sequencing means. 3 monoclonal sequences were obtained. In this example, the sequences of the variable domains of the anti-B7H4 monoclonal antibody molecules obtained from immunized Harbour H2L2 mice were human antibody sequences, whose germline gene analysis and post-translational modification site (PTM) analysis are listed in Table 1-1.

Chemical modifications, sometimes introduced after amino acid chains of a protein or polypeptide is translated and synthesized in a cell, are called post-translational modifications (PTMs). For antibodies, some PTM sites are very conservative. For example, the conservative amino acid asparagine (Asn) at position 297 (EU numbering) of the constant domain of the human IgG1 antibody is often glycosylated to form a saccharide chain whose structure is critical for antibody structure and associated effector functions. However, PTMs may have a greater effect on antigen binding or result in changes in the physicochemical properties of the antibody, if they are present in the variable domains, particularly in the antigen binding regions (e.g., CDRs) of an antibody. For example, glycosylation, deamidation, isomerization, oxidation, and the like may increase the instability or heterogeneity of antibody molecules, thereby increasing the difficulty and risk of antibody development. Thus, it is very important for the development of therapeutic antibodies to avoid some potential PTMs. As experience has accumulated, it has been found that some PTMs are highly correlated with the composition of amino acid sequences, especially the "pattern" of the composition of adjacent amino acids, which makes it possible to predict potential PTMs from the primary amino acid sequences of a protein. For example, it can be predicted that there is an N-linked glycosylation site from the N-x-S/T sequence pattern (asparagine at the first position, any amino acid other than non-proline at the second position, and serine or threonine at the third position). The amino acid sequence patterns leading to PTMs may be derived from germline gene sequences, e.g., the human germline gene fragment IGHV3-33 naturally having a glycosylation pattern NST in the FR3 region; or they may also be derived from somatic hypermutations.

The amino acid sequence patterns of PTMs may be disrupted by amino acid mutations, thereby reducing or eliminating the formation of specific PTMs. There are different methods for designing mutations depending on the antibody sequences and PTM sequence patterns. One method is to replace a "hot spot" amino acid (e.g., N or S in the NS pattern) with an amino acid with similar physicochemical properties (e.g., to mutate N into Q). If the PTM sequence pattern is derived from somatic hypermutations and is not present in the germline gene sequence, the other method can be to replace the sequence pattern with the corresponding germline gene sequence. In practice, a variety of methods for designing mutations may be used for the same PTM sequence pattern.

The sequences of the new antibody molecules obtained from amino acid mutations on the sequences of antibodies PR001476 and PR002037 are listed in Table 1-2.

**Table 1-1. Germline gene analysis and post-translational modification site (PTM) analysis of anti-B7H4 antibodies**

| Clone No. | VH germline V gene | VL germline V gene | VH PTM | VL PTM | Recombinant antibody | Recombinant antibody subtype |
|---|---|---|---|---|---|---|
| **80C8-2E9** | IGHV3-30 | IGKV3-15 | DG (HCDR2) | NS (LCDR3) | PR001476 | Human IgG1 |
| **1025_B-1H11** | IGHV1-69 | IGKV3-15 | C (LFR3) | None | PR002037 | Human IgG1 |
| **1025_B-2A1** | IGHV1-69 | IGKV3-15 | None | None | PR002038 | Human IgG1 |

**Table 1-2. Mutation site designs of sequences of antigen-binding proteins**

| Initial antibody | Variant | Variable region mutations | Recombinant antibody subtype | Fc mutation |
|---|---|---|---|---|
| **PR001476** | PR002405 | H:D54E; L:N92Q | Human IgG1 | |
| **PR001476** | PR002408 | H:G55A; L:N92Q | Human IgG1 | |
| **PR001476** | PR002411 | | Human IgG1 | S239D, I332E |
| **PR001476** | PR002418 | H:G55A; L:N92Q | Human IgG1 | S239D, I332E |
| **PR001476** | PR003369 | H:GSSA,G101A; L:N92R | Human IgG1 | S239D, I332E |
| **PR002037** | PR002410 | L:C87Y | Human IgG1 | |
| **PR002037** | PR002420 | | Human IgG1 | S239D, I332E |
| **PR002037** | PR002421 | L:C87Y | Human IgG1 | S239D, I332E |

### 1.6. Affinity maturation of antibody PR001476

The molecule PR001476 was modified by site-directed mutagenesis to improve its affinity for binding to B7H4. This method of affinity maturation is divided into two rounds.

In the first round, amino acids of the heavy chain CDR3 and light chain CDR3 (as defined by Chothia CDRs) of the molecule PR001476 were scanned point-by-point to create single-site saturation mutagenesis libraries for multiple amino acid positions. Two saturation mutagenesis libraries were screened, positive molecules with a signal of 2-fold over those of wild type were picked out for sequencing and further identified, and a plurality of mutation hot sites were selected according to the binding ability of the positive molecules to human B7H4.

In the second round, the hot sites found by the saturation mutagenesis in the first round were randomly combined to create a library containing all mutation combinations. The combination library was then screened. Several mutants were selected by sequencing the positive molecules and detecting their binding ability to human B7H4. The selected mutants were represented by the corresponding clone numbers, for example, PR001476-R1-25B3 and PR001476-R1-26D7.

The mutants were constructed into mammalian expression vectors for the expression and purification of the proteins. The mutants were then detected for their binding ability to B7H4 using FACS and Fortebio Octet. PR003369 in Table 1-2 is a preferred mutant derived from PR001476.

### 1.7. Preparation of recombinant antibodies and physicochemical property characterization analysis

#### 1.7.1. Expression and purification of antibodies

This example describes a general method of antibody preparation in mammalian host cells (e.g., human embryonic kidney cell HEK293 or Chinese hamster ovary CHO cells and derived cells thereof) by using such techniques as transient transfection and expression, and affinity capture and separation. This method is applicable to an antibody of interest comprising an Fc region. The antibody of interest may consist of one or more protein polypeptide chains, and may be derived from one or more expression plasmids.

The amino acid sequences of the polypeptide chains of the antibody were converted into nucleotide sequences by codon optimization. The encoding nucleotide sequences were synthesized and cloned into expression vectors compatible with the host cell. The mammalian host cells were transfected simultaneously with plasmids encoding the polypeptide chains of the antibody in a particular ratio, and the recombinant antibody with correct folding and assembly of polypeptide chains could be obtained by the conventional recombinant protein expression and purification techniques. Specifically, FreeStyle™ 293-F cells (Thermo, #R79007) were expanded in FreeStyle™ F17 Expression Medium (Thermo, #A1383504). Before the transient transfection, the cells were adjusted to a concentration of $6 \times 10^5$ cells/mL to $8 \times 10^5$ cells/mL, and cultured in a shaker at 37 °C with 8% $CO_2$ for 24 hours at a concentration of $1.2 \times 10^6$ cells/mL. 30 mL of cultured cells were taken. Plasmids encoding the polypeptide chains of the antibody were mixed in a certain ratio, and a total of 30 μg of the plasmids (the ratio of the plasmids to cells was 1 μg : 1 mL) were dissolved in 1.5 mL of Opti-MEM reduced serum medium (Thermo, #31985088). The resulting mixture was filtered through a 0.22 μm filter membrane for sterilization. Then, 1.5 mL of Opti-MEM was dissolved in 120 μL of 1 mg/mL PEI (Polysciences, #23966-2), and the mixture was left to stand for 5 minutes. PEI was slowly added to the plasmids, and the mixture was incubated at room temperature for 10 min. The mixed solution of plasmids and PEI was slowly added dropwise while shaking the culture flask, and the cells were cultured in a shaker at 37 °C with 8% $CO_2$ for 5 days. Cell viability was measured after 5 days. The culture was collected and centrifuged at 3300 g for 10 min, and then the supernatant was collected and centrifuged at high speed to remove impurities. A gravity column (Bio-Rad, #7311550) containing MabSelect™ (GE Healthcare, #71-5020-91) was equilibrated with a PBS buffer (pH 7.4) and rinsed with 2-5 column volumes of PBS. The column was loaded with the supernatant sample, and rinsed with 5-10 column volumes of PBS buffer, followed by 0.1 M glycine at pH 3.5 to elute the target protein. The eluate was adjusted to neutrality with Tris-HCl at pH 8.0, and concentrated and buffer exchanged into PBS buffer or a buffer with other components with an ultrafiltration tube (Millipore, #UFC901024) to obtain a purified solution of the recombinant antibody. Finally, the purified antibody solution was determined for concentration using NanoDrop (Thermo, NanoDrop™ One), subpackaged and stored for later use.

### 1.7.2. Analysis of protein purity and polymers by SEC-HPLC

In this example, analytical size-exclusion chromatography (SEC) was used to analyze the protein sample for purity and polymer form. An analytical chromatography column TSKgel G3000SWxl (Tosoh Bioscience, #08541, 5 μm, 7.8 mm × 30 cm) was connected to a high-pressure liquid chromatograph HPLC (Agilent Technologies, Agilent 1260 Infinity II) and equilibrated with a PBS buffer at room temperature for at least 1 h. A proper amount of the protein sample (at least 10 μg) was filtered through a 0.22 μm filter membrane and then injected into the system, and an HPLC program was set: the sample was passed through the chromatography column with a PBS buffer at a flow rate of 1.0 mL/min for a maximum of 25 minutes. An analysis report was generated by the HPLC, with the retention time of the components with different molecular sizes in the sample reported.

### 1.7.3. Protein purity and hydrophobicity analysis by HIC-HPLC

Analytical hydrophobic interaction chromatography (HIC) was used to analyze the protein sample for purity and hydrophobicity. An analytical chromatography column TSKge1 Buty1-NPR (Tosoh Bioscience, 14947, 4.6 mm × 3.5 cm) was connected to a high-pressure liquid chromatograph HPLC (Agilent Technologies, Agilent 1260 Infinity II) and equilibrated with a PBS buffer at room temperature for at least 1 hour. The method consisted of a linear gradient from 100% mobile phase A (20 mM histidine, 1.8 M ammonium sulfate, pH 6.0) to 100% mobile phase B (20 mM histidine, pH 6.0) within 16 minutes, wherein the flow rate was set at 0.7 mL/min, the sample concentration was 1 mg/mL, the injection volume was 20 μL, and the detection wavelength was 280 nm. After being recorded, the chromatogram was integrated using ChemStation software and relevant data were calculated. An analysis was generated, with the retention time of the components with different molecular sizes in the sample reported.

### 1.7.4. Determination of thermostability of protein molecules by DSF

Differential scanning fluorimetry (DSF) is a commonly used high-throughput method for determining the thermostability of proteins. In this method, changes in the fluorescence intensity of the dye that binds to unfolded protein molecules were monitored using a real-time quantitative fluorescence PCR instrument to reflect the denaturation process of the protein and thus to reflect the thermostability of the protein. In this example, the thermal denaturation temperature (Tm) of a protein molecule was measured by DSF. 10 μg of protein was added to a 96-well PCR plate (Thermo, #AB-0700/W), followed by the addition of 2 μL of 100× diluted dye SYPROTM (Invitrogen, #2008138), and then the mixture in each well was brought to a final volume of 40 μL by adding buffer. The PCR plate was sealed, placed in a real-time quantitative fluorescence PCR instrument (Bio-Rad CFX96 PCR System), and incubated at 25 °C for 5 min, then at a temperature gradually increased from 25 °C to 95 °C at a gradient of 0.2 °C/0.2 min, and at a temperature decreased to 25 °C at the end of the test. The FRET scanning mode was used and data analysis was performed using Bio-Rad CFX Maestro software to calculate the Tm of the sample.

### 1.8. Preparation of anti-B7H4 fully human recombinant antibodies

Anti-B7H4 fully human IgG antibodies obtained in 1.3-1.6 and the optimized antibodies were prepared and analyzed using the method as described in 1.7.1. The results of transient expression and purification for small and large volumes are listed in Table 1-3 and Table 1-4, respectively. In addition, the sequences of anti-B7H4 antibodies (Table 1-5) were obtained from the prior documents and taken as controls in subsequent experiments.

**Table 1-3. Expression of anti-B7H4 antibodies**

| Antibody | Expression system and volume | Yield after first purification (mg/L) | SEC-HPLC purity (%) |
|---|---|---|---|
| **PR001476** | HEK293-F (50mL) | 80.6 | 99.18 |

(continued)

| Antibody | Expression system and volume | Yield after first purification (mg/L) | SEC-HPLC purity (%) |
|---|---|---|---|
| **PR002037** | HEK293-F (30mL) | 17.3 | 100 |
| **PR002038** | HEK293-F (30mL) | 34.0 | 100 |
| **PR002408** | HEK293-6E (40mL) | 37.0 | 99.03 |
| **PR002411** | HEK293-6E (40mL) | 64.8 | 99.3 |
| **PR002418** | HEK293-F (1.5mL) | 26.7 | n.d. |
| **PR003369** | HEK293-F (30mL) | 6.6 | 100 |
| **PR002410** | HEK293-6E (40mL) | 173.5 | 98.65 |
| **PR002420** | HEK293-6E (40mL) | 5.7 | 99.16 |
| **PR002421** | HEK293-F (1.5mL) | 73.3 | n.d. |

**Table 1-4. Expression of anti-B7H4 antibodies**

| Antibody | Expression system and volume | Yield after two-step purification (mg/L) | SEC-HPLC purity (%) |
|---|---|---|---|
| **PR002418** | Expi-CHOs (1000 mL) | 229 | 99.45% |
| **PR002421** | Expi-CHOs (1000 mL) | 276.9 | 99.01% |
| **PR003369** | Expi-CHOs (1000 mL) | 111.7 | 99.09% |
| **Control antibody 2** | Expi-CHOs (1000 mL) | 180.8 | 95.17% |

**Table 1-5. Information relating to control antibodies**

| Control antibodies | Antibody No. | Description |
|---|---|---|
| **Control antibody 1** | PR000157 | Anti-B7H4 IgG1 antibody (from Patent WO2016040724A1), also known as ID 11.v1.9varC2 |
| **Control antibody 2** | PR002962 | Anti-B7H4 IgG1 antibody (from Patent WO2019040780A1), with site mutation on the Fc region on the basis of PR002961 to enhance ADCC |
| **Control antibody 3** | PR002961 | Anti-B7H4 IgG1 antibody (from Patent WO2019040780A1) |

**1.9. Anti-B7H4 antibody sequences and numbers**

In the present invention, the amino acid sequences of the listed CDRs are shown according to the Chothia scheme. However, it is well known to those skilled in the art that the CDRs of an antibody can be defined in the art using a variety of methods, such as the Kabat scheme based on sequence variability (see Kabat et al., Sequences of Proteins of Immunological Interest, Fifth Edition, National Institutes of Health (U.S.), Bethesda, Maryland (1991)), and the Chothia scheme based on the location of the structural loop regions (see J Mol Biol 273: 927-48, 1997). In the technical solution of the present invention, the Combined scheme comprising the Kabat scheme and the Chothia scheme can also be used to determine the amino acid residues in a variable domain sequence. The Combined scheme combines the Kabat scheme with the Chothia scheme to obtain a larger range. See Table 1-6 for details. It will be understood by those skilled in the art that unless otherwise specified, the terms "CDR" and "complementary determining region" of a given antibody or a region (e.g., variable region) thereof are construed as encompassing complementary determining regions as defined by any one of the above known schemes described herein. Although the scope claimed in the present invention is the sequences shown based on the Chothia scheme, the amino acid sequences corresponding to the other schemes for numbering CDRs shall also fall within the scope of the present invention.

**Table 1-6. The scheme for numbering the CDRs of the antibody of the present application**

| | Kabat | Chothia | Combined |
|---|---|---|---|
| LCDR1 | L24-L34 | L24-L34 | L24-L34 |
| LCDR2 | L50-L56 | L50-L56 | L50-L56 |
| LCDR3 | L89-L97 | L89--L97 | L89-L97 |
| HCDR1 | H31--H35 | H26--H32 | H26-H35 |
| HCDR2 | H50--H65 | H52--H56 | H50-H65 |
| HCDR3 | H95--H102 | H95--H102 | H95-H102 |

Laa-Lbb can refer to an amino acid sequence from position aa (the Chothia scheme) to position bb (the Chothia scheme) beginning at the N-terminus of the light chain of the antibody; and Haa-Hbb can refer to an amino acid sequence from position aa (the Chothia scheme) to position bb (the Chothia scheme) beginning at the N-terminus of the heavy chain of the antibody. For example, L24-L34 can refer to the amino acid sequence from position 24 to position 34 according to the Chothia scheme beginning at the N-terminus of the light chain of the antibody; H26-H35 can refer to the amino acid sequence from position 26 to position 35 according to the Chothia scheme beginning at the N-terminus of the heavy chain of the antibody. It should be known to those skilled in the art that there are positions where insertion sites are present in numbering CDRs with the Chothia scheme (see http://bioinf.org.uk/abs/).

The sequence numbers of the CDRs, variable regions and light and heavy chains corresponding to the sequences of the anti-B7H4 antibodies of the present invention and the control antibody molecules are listed in Table 1-7. PR003366 is a single-chain variable region (scFv) homodimer molecule (scFv-Fc structure) constructed using the variable region sequence of PR002410.

**Table 1-7. Sequence numbers of anti-B7H4 antibodies**

| Antibody No. | Light chain | Heavy chain | VL | VH | LCDR1 | LCDR2 | LCDR3 | HCDR1 | HCDR2 | HCDR3 |
|---|---|---|---|---|---|---|---|---|---|---|
| **PR000157** | 108 | 94 | 85 | 75 | 45 | 52 | 62 | 7 | 19 | 33 |
| **PR002961** | 116 | 104 | 92 | 82 | 48 | 54 | 68 | 12 | 25 | 37 |
| **PR002962** | 116 | 105 | 92 | 82 | 48 | 54 | 68 | 12 | 25 | 37 |
| **PR001476** | 109 | 95 | 87 | 77 | 47 | 54 | 64 | 9 | 21 | 35 |
| **PR002037** | 112 | 98 | 88 | 79 | 48 | 54 | 65 | 11 | 22 | 36 |
| **PR002038** | 113 | 98 | 89 | 79 | 48 | 55 | 66 | 11 | 22 | 36 |
| **PR002405** | 114 | 99 | 90 | 80 | 47 | 54 | 67 | 9 | 23 | 35 |
| **PR002408** | 114 | 100 | 90 | 81 | 47 | 54 | 67 | 9 | 24 | 35 |
| **PR002411** | 109 | 101 | 87 | 77 | 47 | 54 | 64 | 9 | 21 | 35 |
| **PR002418** | 114 | 102 | 90 | 81 | 47 | 54 | 67 | 9 | 24 | 35 |
| **PR003369** | 117 | 106 | 93 | 83 | 47 | 54 | 69 | 9 | 24 | 38 |
| **PR002410** | 115 | 98 | 91 | 79 | 48 | 54 | 65 | 11 | 22 | 36 |
| **PR002420** | 112 | 103 | 88 | 79 | 48 | 54 | 65 | 11 | 22 | 36 |
| **PR002421** | 115 | 103 | 91 | 79 | 48 | 54 | 65 | 11 | 22 | 36 |
| **PR003366** | | 132 | 91 | 79 | 48 | 54 | 65 | 11 | 22 | 36 |

The sequence numbers of the framework regions and Fv corresponding to the sequences of the anti-B7H4 antibodies of the present invention and the control antibody molecules are listed in Table 1-8.

**Table 1-8. Sequence numbers of framework regions and Fv of anti-B7H4 antibodies**

| Antibody No. | Chain type | Fv | FWR1 | FWR2 | FWR3 | FWR4 |
|---|---|---|---|---|---|---|
| PR000157 | Heavy chain | 75 | 1 | 13 | 26 | 39 |
| | Light chain | 85 | 40 | 49 | 56 | 70 |
| PR002961 | Heavy chain | 82 | 6 | 18 | 31 | 39 |
| | Light chain | 92 | 42 | 51 | 58 | 72 |
| PR002962 | Heavy chain | 82 | 6 | 18 | 31 | 39 |
| | Light chain | 92 | 42 | 51 | 58 | 72 |
| PR001476 | Heavy chain | 77 | 3 | 15 | 28 | 39 |
| | Light chain | 87 | 42 | 51 | 58 | 72 |
| PR002037 | Heavy chain | 79 | 5 | 17 | 30 | 39 |
| | Light chain | 88 | 43 | 51 | 59 | 73 |
| PR002038 | Heavy chain | 79 | 5 | 17 | 30 | 39 |
| | Light chain | 89 | 44 | 51 | 60 | 74 |
| PR002405 | Heavy chain | 80 | 3 | 15 | 28 | 39 |
| | Light chain | 90 | 42 | 51 | 58 | 72 |
| PR002408 | Heavy chain | 81 | 3 | 15 | 28 | 39 |
| | Light chain | 90 | 42 | 51 | 58 | 72 |
| PR002411 | Heavy chain | 77 | 3 | 15 | 28 | 39 |
| | Light chain | 87 | 42 | 51 | 58 | 72 |
| PR002418 | Heavy chain | 81 | 3 | 15 | 28 | 39 |
| | Light chain | 90 | 42 | 51 | 58 | 72 |
| PR003369 | Heavy chain | 83 | 3 | 15 | 28 | 39 |
| | Light chain | 93 | 42 | 51 | 58 | 72 |
| PR002410 | Heavy chain | 79 | 5 | 17 | 30 | 39 |
| | Light chain | 91 | 43 | 51 | 61 | 73 |
| PR002420 | Heavy chain | 79 | 5 | 17 | 30 | 39 |
| | Light chain | 88 | 43 | 51 | 59 | 73 |
| PR002421 | Heavy chain | 79 | 5 | 17 | 30 | 39 |
| | Light chain | 91 | 43 | 51 | 61 | 73 |
| PR003366 | Heavy chain | 79 | 5 | 17 | 30 | 39 |
| | Light chain | 91 | 43 | 51 | 61 | 73 |
| PR000627 | Heavy chain | 76 | 2 | 14 | 27 | 39 |
| | Light chain | 86 | 41 | 50 | 57 | 71 |
| PR001924 | Heavy chain | 78 | 4 | 16 | 29 | 39 |
| | Light chain | 86 | 41 | 50 | 57 | 71 |
| PR001848 | Heavy chain | 78 | 4 | 16 | 29 | 39 |
| | Light chain | 86 | 41 | 50 | 57 | 71 |
| PR003886 | Heavy chain | 84 | 2 | 14 | 32 | 39 |
| | Light chain | 86 | 41 | 50 | 57 | 71 |

The CDR sequences corresponding to the sequences of the anti-B7H4 antibodies of the present invention and the control antibody molecules are listed in Table 1-9.

Table 1-9. CDR sequences of anti-B7H4 antibodies

| No. | HCDR1 | HCDR2 | HCDR3 | LCDR1 | LCDR2 | LCDR3 |
|---|---|---|---|---|---|---|
| PR000157 | GYTFTSY | YPGGGY | LAGSSYRGAMDS | KASQGFNKYVA | YTSTLQP | LQYGDLLYA |
| PR002961 | GGSIKSGSY | YYSGS | EGSYPNQFDP | RASQSVSSNLA | GASTRAT | QQYHSFPFT |
| PR002962 | GGSIKSGSY | YYSGS | EGSYPNQFDP | RASQSVSSNLA | GASTRAT | QQYHSFPFT |
| PR001476 | GFTFRSF | SYDGSN | GGGLRWYFAY | RASQSISSNLG | GASTRAT | QQYNSWPPLT |
| PR002037 | EDTFSSY | APIFGT | GGPYFDY | RASQSVSSNLA | GASTRAT | QQYKNWPFT |
| PR002038 | EDTFSSY | APIFGT | GGPYFDY | RASQSVSSNLA | GDYIRAT | QQYVDLPIT |
| PR002405 | GFTFRSF | SYEGSN | GGGLRWYFAY | RASQSISSNLG | GASTRAT | QQYQSWPPLT |
| PR002408 | GFTFRSF | SYDASN | GGGLRWYFAY | RASQSISSNLG | GASTRAT | QQYQSWPPLT |
| PR002411 | GFTFRSF | SYDGSN | GGGLRWYFAY | RASQSISSNLG | GASTRAT | QQYNSWPPLT |
| PR002418 | GFTFRSF | SYDASN | GGGLRWYFAY | RASQSISSNLG | GASTRAT | QQYQSWPPLT |
| PR003369 | GFTFRSF | SYDASN | GGALRWYFAY | RASQSISSNLG | GASTRAT | QQYRSWPPLT |
| PR002410 | EDTFSSY | APIFGT | GGPYFDY | RASQSVSSNLA | GASTRAT | QQYKNWPFT |
| PR002420 | EDTFSSY | APIFGT | GGPYFDY | RASQSVSSNLA | GASTRAT | QQYKNWPFT |
| PR002421 | EDTFSSY | APIFGT | GGPYFDY | RASQSVSSNLA | GASTRAT | QQYKNWPFT |
| PR003366 | EDTFSSY | APIFGT | GGPYFDY | RASQSVSSNLA | GASTRAT | QQYKNWPFT |
| PR000627 | GFTFNTY | RSKYNNYA | HGNFGNSYVSWFAY | RSSTGAVTTSNYAN | GTNKRAP | ALWYSNLWV |
| PR001924 | GFTFSTY | RSKYNNYA | HGNFGNSYVSWFAY | RSSTGAVTTSNYAN | GTNKRAP | ALWYSNLWV |
| PR001848 | GFTFSTY | RSKYNNYA | HGNFGNSYVSWFAY | RSSTGAVTTSNYAN | GTNKRAP | ALWYSNLWV |
| PR003886 | GFTFSTY | RSKYNNYA | HGNFGNSYVSWFAY | RSSTGAVTTSNYAN | GTNKRAP | ALWYSNLWV |

**Example 2. Detection of Binding Ability of Anti-B7H4 Antibodies to B7H4 by FACS**

This example is intended to investigate the *in vitro* binding activity of anti-human B7H4 H2L2 monoclonal antibody to human/cynomolgus monkey/mouse B7H4. Antibody binding experiments at the cellular level were performed using CHOK1 cell line overexpressing human B7H4 (CHOK1/hu B7H4, Harbour BioMed), CHOK1 cell line overexpressing cynomolgus monkey B7H4 (CHOK1/cyno B7H4, Harbour BioMed), CHOK1 cell line overexpressing mouse B7H4 (CHOK1/m B7H4, Harbour BioMed) and SK-BR-3 cell line (*ATCC*® HTB-30) highly expressing human B7H4. Briefly, CHOK1/hu B7H4 cells, CHOK1/cyno B7H4 cells, CHOK1/m B7H4 cells or SK-BR-3 cells were digested and resuspended with PBS containing 2% BSA. The cell density was adjusted to $1\times10^6$ cells/mL. The cells were seeded in a 96-well V-bottom plate (Corning, #3894) at 100 μL/well, followed by the addition of test antibodies diluted in a 3-fold concentration gradient of 2 times the final concentration, each at 100 μL/well. The cells were incubated at 4 °C for 2 h away from light. Thereafter, the cells in each well were rinsed twice with 100 μL of pre-cooled PBS containing 2% BSA, and centrifuged at 500 g at 4 °C for 5 min, and then the supernatant was discarded. Then 100 μL of a fluorescent secondary antibody (Alexa Fluor 488-conjugated AffiniPure Goat Anti-Human IgG, Fcγ Fragment Specific, Jackson, #109-545-098, 1:500 diluted) was added to each well. The plate was incubated away from light at 4 °C for 1 h. The cells in each well were washed twice with 100 μL of pre-cooled PBS containing 2% BSA, and centrifuged at 500 g at 4 °C for 5 minutes, and then the supernatant was discarded. Finally, the cells in each well were resuspended with 200 μL of pre-cooled PBS containing 2% BSA, and the fluorescence signal values were read using an ACEANovocyte3000 flow cytometer.

The antibodies binding to human B7H4, cynomolgus monkey B7H4 and mouse B7H4 on the cell surface and to B7H4 on the surface of tumor cells SK-BR-3 are summarized below (Table 2-1, Table 2-2 and Table 2-3). The variant PR003369 with affinity maturation showed a significant improvement in its binding to tumor cells compared with PR002418 (Table 2-3).

**Table 2-1. Anti-B7H4 antibodies (initial antibodies) binding to human B7H4, cynomolgus monkey B7H4 and mouse B7H4 on cell surface and to B7H4 on surface of tumor cells SK-BR-3**

| | CHOK1/hu B7H4 | | CHOK1/cyno B7H4 | | CHOK1/m B7H4 | | SK-BR-3 | |
|---|---|---|---|---|---|---|---|---|
| **Antibody** | **Maximum MFI** | **$EC_{50}$ (nM)** | **Maximum MFI** | **$EC_{50}$ (nM)** | **Maximum MFI** | **$EC_{50}$ (nM)** | **Maximum MFI** | **$EC_{50}$ (nM)** |
| **PR002037** | 1677000 | 3.196 | 1207000 | 3.301 | 750723 | 2.698 | 290692 | 3.527 |
| **PR002038** | 1146000 | 20.04 | 677326 | 24.76 | ~ | ~ | 82832 | 85.62 |
| **PR001476** | 1412000 | 0.7552 | 971785 | 0.8861 | 230031 | 3.018 | 277576 | 0.4706 |
| **Control antibody 1** | 1839000 | 1.943 | 1317000 | 2.057 | 857700 | 1.381 | 316232 | 1.175 |

**Table 2-2. Anti-B7H4 antibodies (variant molecules) binding to cynomolgus monkey B7H4 and mouse B7H4 on cell surface and to B7H4 on surface of tumor cells SK-BR-3**

| | CHOK1/cyno B7H4 | | CHOK1/m B7H4 | | SK-BR-3 | |
|---|---|---|---|---|---|---|
| **Antibody** | **Maximum MFI** | **$EC_{50}$ (nM)** | **Maximum MFI** | **$EC_{50}$ (nM)** | **Maximum MFI** | **$EC_{50}$ (nM)** |
| **RP001476** | 1822000 | 1.097 | 353378 | 3.614 | 238962 | 0.367 |
| **PR002408** | 1852000 | 1.029 | 398460 | 1.678 | 248060 | 0.3531 |
| **PR002418** | 1790000 | 0.7985 | 398407 | 1.306 | 251781 | 0.3112 |
| **PR002037** | 1922000 | 2.382 | 1686000 | 2.57 | 256727 | 2.22 |
| **PR002410** | 1862000 | 2.077 | 1554000 | 2.385 | 245482 | 1.872 |
| **PR002421** | 1873000 | 2.048 | 1465000 | 1.988 | 242474 | 1.92 |
| **Control antibody 1** | 1986000 | 2.095 | 1749000 | 1.728 | 265713 | 1.199 |

**Table 2-3. Binding of PR003369 and PR002418 to tumor cells**

| | SK-BR-3 | |
|---|---|---|
| **Antibody** | **$EC_{50}$ (nM)** | **Maximum MFI** |
| PR002418 | 1.116 | 162162 |
| PR003369 | 0.4342 | 170835 |

The results of the initial antibodies binding to human B7H4 on the cell surface are shown in FIG. 1, and the results showed that PR001476 and PR002037 had stronger binding activity to human B7H4, and PR002038 had weaker binding activity thereto; the results of expression supernatants of PR001476 and PR002037 and their PTM variants/DE mutant antibodies binding to human B7H4 on the cell surface are shown in FIG. 2, and the results showed that the PTM variants/DE mutant antibodies of PR002037 and PR001476 did not significantly affect the binding activity of the antibodies to human B7H4. The results of the initial antibodies binding to cynomolgus monkey B7H4 on the cell surface are shown in FIG. 3, and the results showed that PR002037 and PR001476 had stronger cross-binding activity to cynomolgus monkey B7H4, and PR002038 had weaker cross-binding activity thereto; the results of PR001476 and PR002037 and their PTM variants/DE mutant antibodies binding to cynomolgus monkey B7H4 on the cell surface are shown in FIG. 4, and the results showed that the PTM variants/DE mutant antibodies of PR002037 and PR001476 did not significantly affect the cross-binding activity of the antibodies to cynomolgus monkey B7H4. The results of the initial antibodies binding to mouse B7H4 on cell surface are shown in FIG. 5, and the results showed that PR002037 had stronger cross-binding activity to mouse B7H4, PR001476 had weaker cross-binding activity thereto, and PR002038 had no cross-binding activity thereto; the results of PR001476 and PR002037 and their PTM variants/DE mutant antibodies binding to mouse B7H4 on the cell surface are shown in FIG. 6, and the results showed that the PTM variants/DE mutant antibodies of PR002037 and PR001476 maintained similar cross-binding activity to mouse B7H4 as compared with their parents. The results of the initial antibodies binding to B7H4 on the surface of tumor cells SK-BR-3 are shown in FIG. 7, and the results showed that PR002037 and PR001476 had stronger binding activity to B7H4 on the surface of the tumor cells SK-BR-3, and PR002038 had weaker binding activity thereto; the results of expression supernatants of PR001476 and PR002037 and their PTM variants/DE mutant antibodies binding to B7H4 on the surface of tumor cells SK-BR-3 are shown in FIG. 8, and the results showed that the PTM variants/DE mutant antibodies of PR002037 and PR001476 did not significantly affect the binding activity of the antibodies to B7H4 on the surface of tumor cells SK-BR-3; the results of PR001476 and PR002037 and their PTM variants/DE mutant antibodies binding to B7H4 on the surface of tumor cells SK-BR-3 are shown in FIG. 9, and the results showed that the PTM variants/DE mutant antibodies of PR002037 and PR001476 maintained the binding activity to B7H4 on the surface of tumor cells SK-BR-3. The PTM variant/DE mutant antibody of PR001476 had stronger binding activity (lower $EC_{50}$) compared with control antibody 1; the results of PR002418 and PR001476 with affinity maturation/DE mutant antibody PR003369 binding to B7H4 on the surface of tumor cells MDA-MB-468 are shown in FIG. 10, and the results showed the variant PR003369 with affinity maturation had significantly improved binding to B7H4 on the surface of tumor cells MDA-MB-468 compared with the PTM variant PR002418.

## Example 3. ADCC Activity Assay

This example is intended to investigate the activity of anti-human B7H4 H2L2 monoclonal antibody in mediating NK cell killing of target cells through the ADCC effect *in vitro.* In this experiment, the human PBMC was used as an effector cell and cell lines SK-BR-3 and MDA-MB-468 highly expressing B7H4 and a cell line HCC-1954 moderately expressing B7H4 were used as target cells. The killing efficiency was reflected by the conductivity of the target cell measured using an RTCA instrument from ACEA. A 96-well plate E-plate was first equilibrated with 50 $\mu$L of complete medium. SK-BR-3, MDA-MB-468 or HCC-1954 cells were digested, resuspended in RPM1640 complete medium containing 10% fetal bovine serum and diluted to $4 \times 10^5$/mL. The cell suspension was plated on the 96-well E-plate at 50 $\mu$L/well, i.e., $2 \times 10^4$/well, and incubated overnight at 37 °C. The next day, 50 $\mu$L of fresh culture medium containing $2 \times 10^5$ PBMCs was added to each well, followed by the addition of 50 $\mu$L of antibodies diluted in a 4$\times$ concentration gradient with a maximum final concentration of 10 nM. A total of 8 concentrations were set in duplicate for each antibody. The conductivity of the target cells was measured in real time. Generally, the value at hour 4 was used to calculate the target cell killing efficiency = (1 - sample/blank control) $\times$ 100%.

The ADCC killing activity of PR001476 and its PTM variant/DE mutant antibody against tumor cells SK-BR-3 is shown in FIG. 11, and the results showed that the DE mutant antibody (PR002418) at the Fc end could significantly enhance the ADCC killing activity of the antibodies against the tumor cells SK-BR-3; the ADCC killing activity of PR002037 and its PTM variant/DE mutant antibody on tumor cells SK-BR-3 is shown in FIG. 12, and the results showed that the DE mutant antibody (PR002421) at the Fc end could significantly enhance the ADCC killing activity of the antibodies against the tumor cells SK-BR-3. The comparison of the ADCC killing activity of PR002418 and PR002421 on tumor cells SK-BR-3 with the control antibodies from FivePrime is shown in FIG. 13 (wherein A is donor 1 and B is donor 2), and the results showed that PR002418 had similar ADCC killing activity against tumor cells SK-BR-3 compared with the control antibodies from FivePrime, and PR002421 had weaker killing activity against tumor cells SK-BR-3. The comparison of ADCC killing activity of PR003369, PR002418 and RP002421 against tumor cells MDA-MB-468 and HCC-1954 with the control antibody 2 from FivePrime are shown in FIG. 14 (wherein A is MDA-MB-468 and B is HCC-1954), and the results showed that PR003369, PR002418 and RP002421 all had ADCC killing activity against tumor cells MDA-MB-468 and HCC-1954, and the killing activity was positively correlated with B7H4 expression, i.e., the killing activity was stronger on MDA-MB-468 cells highly expressing B7H4, and weaker on HCC-1954 cells moderately expressing B7H4. The variant PR003369 with affinity maturation further enhanced ADCC killing activity compared with PR002418 and PR002421.

**Example 4. Detection of Activation Activity of T cells**

To detect the function of the anti-B7H4 antibodies for blocking immune checkpoints of T cells and thus activating T cells, in this experiment, full-length B7H4 and anti-human CD3 antibody OKT3 in the form of scFv were overexpressed on HEK293T cells and taken as artificial antigen presenting cells (HEK 293T/OS8/hB7H4, KYinno), a human T cell isolation kit (Miltenyi, #130-096-535) was used to isolate T cells according to the method of the instruction, the artificial antigen presenting cells and the T cells were co-cultured, and the activation effect of the anti-B7H4 antibodies on T cells was detected. Specifically, HEK293T-OS8-hB7H4 was plated at a density of $1 \times 10^4$/well and incubated overnight. Human primary T cells were isolated and added to HEK293T/OS8/hB7H4 cells at a density of $2 \times 10^5$/100 μL/well, followed by the addition of test antibodies diluted in a 5-fold concentration gradient of 2 folds the final concentration, each at 100 μL/well, wherein the maximum final concentration of the antibody was 10 nM. A total of 6 concentrations were set in duplicate for each antibody. After 3 days of culture, the supernatant was collected and the concentration of IFN-γ was detected by ELISA method. The results showed that PR003369, PR002418, PR002421 and control antibodies all could promote the activation of T cells, wherein the variant PR003369 with affinity maturation had stronger T cell activation activity compared with the PTM variant PR002418 and the control antibody 2, and the mechanism of action of PR003369 may be blocking the interaction between B7H4 with its unknown receptor on T cells. The results of the anti-B7H4 antibodies blocking the immunosuppressive signal of B7H4 to activate T cells are shown in FIG. 15 (where A is donor 1 and B is donor 2).

**Example 5. Antibody Internalization Experiment**

Internalization of antibodies was detected using Zenon pHrodo iFL IgG Labeling Reagents kit (Invitrogen, #Z25611). The reagent is a secondary antibody with a fluorescent dye, does not emit fluorescence at neutral pH, can automatically emit bright fluorescence in an acidic pH environment after being combined with the primary antibody and internalized into lysosomes along with the antibody, and can be detected by FACS method. The specific method was as follows: SK-BR-3 cells were collected and centrifuged to discard the supernatant, and the cells were resuspended in a medium to adjust the cell concentration to $3 \times 10^6$/mL. The cell suspension was added to a 96-well plate at 50 μL/well, and then incubated overnight in a 37 °C thermostatic incubator. Test antibodies of 4× were prepared at a maximum concentration of 40 nM (4×), and diluted 3-fold for a total of 8 dilutions. A Zenon solution of 4× was prepared, and 25 μL of the test antibody and 25 μL of a Zenon labeling solution of 4× were mixed together and left to stand at room temperature for 5 minutes. Then 50 μL of the labeled antibody was added to a 96-well plate containing the cells, and incubated in a 37 °C thermostatic incubator for 24 hours. The cells were digested and fluorescence values were read on a flow cytometer. The results showed that PR003369 had the highest internalization activity compared with the control antibody RP000014 and other antibodies. The results of internalization of the anti-B7H4 antibodies on SK-BR-3 cells are shown in FIG. 16.

Internalization of the antibodies was detected using a-HFc-CL-MMAF reagent (Moradec, #AH-102-AF). The reagent is a secondary antibody carrying a toxic group MMAF, and the mechanism of the reagent being combined with the primary antibody and internalized along with the antibody to release the toxic group in the cells to kill the target cells is similar to that of ADC. The specific method was as follows: SK-BR-3 cells were collected and centrifuged to discard the supernatant, and the cells were resuspended in medium to a cell concentration of $1 \times 10^5$/mL. The cell suspension was added to a 96-well plate at 50 μL/well, and then incubated overnight in a 37 °C thermostatic incubator. Test antibodies of 4× were prepared at a maximum concentration of 40 nM (4×) in 5-fold dilutions for a total of 8 dilutions. An MMAF solution of 4× (4 μg/mL) was prepared. 25 μL of the test antibody of 4× and 25 μL of MMAF solution of 4× were added to a 96-well plate containing cells, and incubated at a 37 °C thermostatic incubator for 72 hours. 100 μL of CTG solution was added to the wells, and the luminescence signals of CTG were read using a microplate reader. The results in FIG.

17 showed that PR003369 had the highest internalization-mediated cytotoxic activity compared with the control antibody RP000014 and other antibodies.

**Example 6. Antibody-Drug Conjugate (ADC)**

In this example, an ADC was prepared using ADC conjugation technology by crosslinking a toxic group MMAE to an anti-B7H4 antibody (PR003369 antibody or control antibody 1). The purity parameters of the product are as follows, and the HPLC detection method is the same as that in 1.7.2.

**Table 6-1. ADC preparation of anti-B7H4 H2L2 antibody**

| Antibody ADC | Concentration (mg/mL) | DAR | Storage solution | SEC-HPLC Purity % | Endotoxin EU/mg |
|---|---|---|---|---|---|
| **PR003369-MMAE** | 2.66 | 4.4 | PBS, pH7.2 | 97.05 | <0.06 |
| **Control antibody 1-MMAE** | 2.65 | 4.1 | PBS, pH7.2 | 97.42 | 0.1 |

To investigate whether the binding activity of the antibody to the B7H4 target was affected after crosslinking of MMAE groups, antibody binding experiments at the cellular level were performed using the cell line MDA-MB-468 highly expressing human B7H4, and the method was the same as that in Example 2. The results in FIG. 18 showed that the crosslinking of MMAE groups did not substantially affect the binding activity of the antibody to B7H4 on the surface of tumor cells.

To investigate whether the internalization activity of the antibody was affected after crosslinking of MMAE groups, the internalization of the antibody was detected using Zenon pHrodo iFL IgG Labeling Reagents kit (Invitrogen, #Z25611). The experimental method was the same as that in Example 5. The results in FIG. 19 showed that the crosslinking of MMAE groups did not substantially affect the internalization activity of the antibody on tumor cells.

This example is to investigate the cell killing activity of the antibody with MMAE groups crosslinking ADCs. The cell line MDA-MB-468 highly expressing B7H4 was taken as a target cell, and the killing efficiency was reflected by the conductivity of the target cell measured using an RTCA instrument from ACEA. A 96-well plate E-plate was first equilibrated with 50 μL of complete medium. MDA-MB-468 cells were digested, resuspended in RPM1640 complete medium containing 10% fetal bovine serum and diluted to $1 \times 10^5$/mL. The cell suspension was plated on the 96-well E-plate at 50 μL/well, i.e., $5 \times 10^3$/well, and incubated overnight at 37 °C. The next day, 100 μL of antibodies diluted in a 2× concentration gradient was added to each well with a maximum final concentration of 50 nM. A total of 8 concentrations were set in duplicate for each antibody. The conductivity of the target cells was measured in real time. Generally, the value at hour 96 was used to calculate the target cell killing efficiency = (1 - sample/blank control) × 100%. The results in FIG. 20 showed that the variant PR003369-ADC with affinity maturation had a higher tumor cell killing efficiency than that of the control antibody 1-ADC.

**Example 7. Determination of Affinity of Anti-B7H4 Antibodies to Human B7H4 Recombinant Protein**

**7.1. Determination of affinity by SPR method**

10 × HBS-EP + (GE Healthcare, #BR-1006-69) was diluted 10-fold and then taken as an experimental buffer. The flow rate was set at 10 μL/min, and Protein A was coupled to 4 channels of a chip CM5 (GE Healthcare, #BR-1005-30) through the following procedures: 1) the injection time was set to 800 s, and a fresh mixture of 50 mM NHS and 200 mM EDC was injected into the 4 channels in a volume ratio of 1: 1; 2) Protein A was diluted to 20 μg/mL with sodium acetate (GE Healthcare, #BR-1003-50) at pH 4.5 and injected into each channel for 800 s; and 3) 1 M ethanolamine at pH 8.5 was injected for 800 s to block the remaining active carboxyl groups on the chip surface. After blocking, the instrument was equilibrated with 1 × HBS-EP + buffer for 2 hours, and the final coupling level of Protein A was about 2000 RU.

A multi-cycle kinetics mode was set at Biacore T200, and each cycle included antibody capture, analyte binding and chip regeneration. Antibodies PR002418 and PR002421, control antibody 1, and control antibody 2 were all diluted to 1 μg/mL, injected into channels 2, 3 and 4 at a flow rate of 10 μL/min for 30 s, and each antibody was captured by a pre-coupled Protein A at level 160 RU. Human B7-H4 (Sino biological, #10738-H08H) was injected into four channels (for control antibody 1, one maximum concentration of 100 nM was added) with a concentration gradient of 0 nM, 1.5625 nM, 3.125 nM, 6.25 nM, 12.5 nM, 25 nM and 50 nM sequentially, and the flow rate was set at 30 μL/min. The dissociation time was set to 200 s for PR002418, PR002421 and control antibody 2, and 500 s for control antibody 1, with 180 s for each injection. Finally, 10 mM glycine-hydrochloric acid at pH 1.5 (GE Healthcare, #BR-1003-54) was injected at the same flow rate for 30 s to regenerate the chip.

The experimental results were analyzed using the Biacore T200 analysis software 2.0, with channel 1 subtracted as a reference channel and a 1:1 kinetic fitting model selected as an analysis model. The results are shown in Table 7-1 and A-D of FIG. 21, which showed that PR002421 had the highest protein affinity.

**Table 7-1. Affinity of anti-B7H4 antibodies for binding to human B7H4 protein (SPR method)**

| Antibody | Antigen | Antibody concentration (nM) | ka (1/Ms) | kd (1/s) | KD (M) |
|---|---|---|---|---|---|
| **PR002418** | Human B7H4 (his tag) | 50 - 1.5625 | 2.01E+05 | 4.75E-03 | 2.37E-08 |
| **PR002421** | Human B7H4 (his tag) | 50 - 1.5625 | 3.50E+05 | 3.22E-04 | 9.21E-10 |
| **Control antibody 2** | Human B7H4 (his tag) | 50 - 1.5625 | 2.61E+05 | 1.16E-03 | 4.45E-09 |
| **Control antibody 1** | Human B7H4 (his tag) | 100 - 3.125 | 4.04E+04 | 1.89E-04 | 4.67E-09 |

### 7.2. Determination of affinity by BLI method

10× kinetics buffer (ForteBio, #18-1105) was diluted to 1× kinetics buffer for affinity assay and dilution of antigens and antibodies. The binding kinetics between the antigen and the antibody was analyzed by the Biolayer Interferometry (BLI) technique using an Octet Red 96e molecular interaction analyzer (Fortebio).

When the affinity of the antigen for the antibody was determined, the rotation speed of the sensor was set at 1000 rpm/min. The AHC sensors (Fortebio, #18-5060) placed in a row were equilibrated for 10 minutes in a test buffer before the AHC sensors were used to capture the B7-H4 antibodies at a capture height of 0.7 nm; the AHC sensors, after equilibrated in the buffer for 120 s, bound to 2-fold serially diluted human B7-H4 (concentrations were 50 nM-3.125 nM and 0 nM) for 180 s, followed by dissociation for 300 s. Finally, the AHC sensor was immersed in a 10 mM glycine-hydrochloric acid solution at pH 1.5 for regeneration to elute the proteins bound to the sensor.

When data analysis was performed using Octet Data Analysis software (Fortebio, version 11.0), 0 nM was taken as a reference well, and reference subtraction was performed; the "1:1 Global fitting" method was selected to fit the data, and the kinetics parameters of the binding of antigens to antigen-binding proteins were calculated, with $k_{on}$(1/Ms) values, $k_{dis}$(1/s) values and $K_D$(M) values obtained (see Table 7-2). The results showed that the variant PR003369 with affinity maturation had stronger protein affinity than PR002418.

**Table 7-2. Affinity of anti-B7H4 antibodies for binding to human B7H4 protein (BLI method)**

| Antibody | Concentration (nM) | KD (M) | kon(1/Ms) | kdis(1/s) | Full R^2 |
|---|---|---|---|---|---|
| **PR002418** | 50-12.5 | 1.03E-08 | 2.75E+05 | 2.84E-03 | 0.9926 |
| **PR003369** | 50-12.5 | 1.39E-09 | 2.83E+05 | 3.94E-04 | 0.9981 |

### Example 8. Determination of Epitope Competition of Anti-B7H4 Antibodies Binding to B7H4 by BLI Method

Epitope competition experiments were performed on B7-H4 antibodies PR002418 and PR002421, control antibody 1 and control antibody 2 by using ForteBio Octet Red96e platform, and the experimental buffer was the same as described above. Step one, acquisition of 100% signal of antibodies: B7-H4 (Acro Biosystems, #B74-H82E2-200μg) was captured at a capture height of 0.25 nm using an SA sensor (Fortebio, #18-5019). The sensor was equilibrated in a buffer for 120 s and then immersed in each antibody diluted to 100 nM for 240 s, and the final signal of the antibody binding to B7-H4 was recorded as the 100% signal of the antibody. Step two, epitope competition experiment: B7-H4 was captured using an SA sensor at a capture height of 0.25 nm. The sensor was immersed in a primary antibody (at a concentration of 100 nM) for 240 s, and then the SA sensor was immersed in a mixture of the primary and secondary antibodies (both at a final concentration of 100 nM) for 240 s, and the difference in signals after immersion of the sensor in the antibody mixture was recorded as the signal of the antibody as the secondary antibody. The inhibition rate was calculated according to the following formula:

$$\text{Inhibition (\%)} = (A - B)/A \times 100$$

A: 100% signal of an antibody (obtained from step one), B: the signal of the antibody as the secondary antibody (obtained from step two).

If the inhibition rate obtained was greater than 85 (%), it means that the epitopes of the two antibodies were completely overlapped; if the inhibition rate was less than 85 (%), it means that the epitopes to which the two antibodies bind were not completely overlapped.

The results in Table 8-1 showed that the epitopes of PR002418 and PR002421 binding to B7-H4 were different, and also different from the epitopes of control antibody 1 and control antibody 2, wherein PR002418 bound to one unique epitope (the first epitope), PR002421 bound to another epitope (the second epitope), and control antibody 1 and control antibody 2 bound to the same epitope (the third epitope).

**Table 8-1. Competitive analysis for antibodies binding to B7H4**

| Competitive inhibition rate (%) | | 2nd Ab | | | |
|---|---|---|---|---|---|
| | | PR002418 | PR002421 | Control antibody 2 | Control antibody 1 |
| 1st Ab | PR002418 | 94.91 | 7.23 | 8.15 | 11.19 |
| | PR002421 | 7.12 | 94.77 | 10.62 | 10.78 |
| | Control antibody 2 | 4.49 | 6.32 | 95.75 | 96.57 |
| | Control antibody 1 | 4.55 | 1.97 | 94.58 | 95.31 |

### Example 9. Cross-Reactivity with Other Members of B7 Family

The proteins of the B7 family (see Table 9-1 for details) were each diluted to 1 μg/mL with PBS, added to a 96-well plate (Corning, #9018) at 100 μL per well, and incubated overnight at 4 °C. After the liquid was discarded, the plate was washed 3 times with PBST buffer (pH 7.4, containing 0.05% tween-20), and 250 μL of 2% BSA blocking buffer was added. The plate was incubated at 37 °C for 1 hour. The blocking buffer was discarded, and the plate was washed 3 times with PBST buffer (pH 7.4, containing 0.05% Tween-20). The test antigen-binding protein was diluted to 2 concentrations: 10 nM and 1 nM, and added at 100 μL per well. The plate was incubated at 37 °C for 1 hour. An isotype antibody was taken as a control. After the plate was washed 3 times with PBST buffer (pH 7.4, containing 0.05% Tween-20), the plate was added with a 5000-fold diluted goat anti-human $F(ab')_2$ HRP secondary antibody (Jackson ImmunoResearch, 109-035-097), and incubated at 37 °C away from light for 1 hour. After the plate was washed 3 times with PBST buffer (pH 7.4, containing 0.05% Tween-20), TMB (Biopanda, #TMB-S-003) was added at 100 μL/well. The plate was left away from light at room temperature for about 30 minutes. The reactions were terminated by adding 50 μL of stop buffer (BBI life sciences, #E661006-0200) to each well, and the absorbance values at 450 nm (OD450) was measured using a microplate reader (PerkinElemer, #Enspire). FIG. 22 showed that the antibodies of the present invention did not cross-react with other member proteins of the B7 family.

**Table 9-1. Material information of B7 family proteins used in this example**

| Other members of B7 family | Supplier | Catalog number |
|---|---|---|
| **Human B7-1 / CD80 Protein, Fc Tag (HPLC-verified)** | Aero | B71-H5259 |
| **Human B7-2 / CD86 Protein, Fc Tag** | Aero | CD6-H5257 |
| **Human B7-DC/PD-L2/CD273 Protein, Recombinant (Fc Tag)** | Sino Biological | H10292-H02H |
| **Human PD-L1 / B7-H1 Protein, His Tag (HPLC verified)** | Aero | PD1-H5229 |
| **CD275/ICOS ligand Protein, Human, Recombinant (Fc Tag)** | Sino Biological | 11559-H02H |
| **B7-H3/CD276 Protein, Human, Recombinant (ECD, Fc Tag)** | Sino Biological | 11188-H02H |
| **Recombinant human B7H4 Fc Chimera** | R&D | 8870-B7-050 |
| **Recombinant human VISTA/B7H5/PD-1H Fc Chimera** | R&D | 7126-B7-050 |
| **B7-H6 /NCR3LG1 Protein, Human, Recombinant (Fc Tag)** | Sino Biological | 16140-H02H |
| **Recombinant human B7H7/HHLA2 Fc Chimera** | R&D | 8084-B7-050 |

**Example 10. Serum Stability Assay**

30 μL of antibody was diluted to 270 μL of normal human serum (serum concentration 90%), the antibody was divided into 5 parts which were incubated at 37 °C for 0 day, 1 day, 4 days, 7 days and 14 days, respectively, and then the antibodies were taken out, quick frozen with liquid nitrogen, and stored at -80 °C. The binding of antibodies to B7H4 on SK-BR-3 cells was detected by flow methods.

SK-BR-3 or CHOK1/H B7H4 cells were digested and resuspended with PBS containing 2% BSA. The cell density was adjusted to $1 \times 10^6$ cells/mL. The cells were seeded in a 96-well V-bottom plate (Corning, #3894) at 100 μL/well, followed by the addition of test antibodies diluted in a 3-fold gradient at a concentration that was 2 times the final concentration, each at 100 μL/well. The cells were incubated at 4 °C for 2 h away from light. Thereafter, the cells in each well were rinsed twice with 100 μL of pre-cooled PBS containing 2% BSA, and centrifuged at 500 g at 4 °C for 5 minutes, and then the supernatant was discarded. Then each well was added with 100 μL of fluorescent secondary antibody (Alexa Fluor 488-conjugated AffiniPure Goat Anti-Human IgG, Fcγ Fragment Specific, Jackson, #109-545-098, diluted in a 1:500 ratio), and the plate was incubated away from light at 4 °C for 60 minutes. The cells in each well were washed twice with 100 μL of pre-cooled PBS containing 2% BSA, and centrifuged at 500 g at 4 °C for 5 minutes, and then the supernatant was discarded. Finally, the cells in each well were resuspended with 200 μL of pre-cooled PBS containing 2% BSA, and the fluorescence signal values were read using an ACEA Novocyte3000 flow cytometer. The results in FIG. 23 showed that the anti-B7H4 antibodies PR002418, PR002037 and PR003369 and control antibody 1 and control antibody 2 had good stability in human serum at 37 °C for 14 days.

**Example 11. Pharmacokinetics in C57BL/6 Mice**

6 female Nu/Nu mice with a weight of 18-22 g were selected and administered via tail vein at a dose of 20 mg/kg; the whole blood of 3 mice in one group was collected prior to the administration and 15 minutes, 24 hours (1 day), 4 days and 10 days after the administration, and the whole blood of 3 mice in the other group was collected prior to the administration and 5 hours, 2 days, 7 days and 14 days after the administration. The whole blood was left to stand for 30 min for coagulation and centrifuged at 2,000 rpm for 5 min at 4 °C, and the isolated serum sample was cryopreserved at -80 °C until it was taken for analysis. In this example, the drug concentration in the serum of mice was quantitatively determined by ELISA method. The ELISA Fc end overall detection method was performed by capturing a fusion protein containing human Fc in the serum of mice using a goat anti-human Fc polyclonal antibody coating a 96-well plate, and then adding an HRP-labeled goat anti-human Fc secondary antibody. The plasma concentration data were analyzed using Phoenix WinNonlin software (version 8.2) by non-compartmental analysis (NCA) to evaluate the pharmacokinetics.

Table 11-1 shows the pharmacokinetic parameters of PR002418, PR002421 and control antibody 2 (PR002962). The results in FIG. 24 showed that the half-life of PR002418 in mice was about 6.56 days, the half-life of PR002421 in mice was about 6.64 days, and the half-life of control antibody 2 in mice was about 5.90 days, as calculated from the data of the first 14 days under the Fc end overall detection method. This result showed that PR002418 and PR002421 had slightly longer half-lives in mice than control antibody 2.

**Table 11-1. Pharmacokinetic parameters of anti-B7H4 antibodies**

| PK parameters | PR002421 | PR002418 | PR002962 |
|---|---|---|---|
| **Tl/2 (hr)** | 159.3 | 157.5 | 141.5 |
| **Vd (mL/kg)** | 93.3 | 89.3 | 93.3 |
| **AUCall (μg/mL hr)** | 36,710 | 37,786 | 33,913 |
| **C1 (mL/hr/kg)** | 0.42 | 0.41 | 0.48 |
| **C0 (μg/mL)** | 437.9 | 387.6 | 440.4 |

**Example 12. Anti-Tumor Efficacy of Anti-B7H4 Antibodies**

**12.1. BALB/c nude mouse MDA-MB-468 tumor model**

On the day of cell inoculation, each BALB/c nude mouse was inoculated subcutaneously with $1 \times 10^7$ tumor cells MDA-MB-468, the cells were resuspended in a PBS/Matrigel (1:1) mixture (0.1 mL/mouse) and inoculated subcutaneously. When the mean tumor volume of each group of mice reached 135 $mm^3$, 25 mice were divided into 5 groups, with an administration cycle being 2 times per week for 12 administrations via intraperitoneal administration. After the start of administration, the body weight and the tumor volume were measured twice a week. The tumor volume was calculated as follows: tumor volume ($mm^3$) = 0.5 × long diameter of tumor × short diameter of tumor$^2$. The experiment was terminated 39 days after administration and all mice were euthanized.

The *in vivo* anti-tumor efficacy of BALB/c nude mouse MDA-MB-468 tumor model is shown in FIG. 25, and specifically, the mice in the vehicle control group had a mean tumor volume of 1054 $mm^3$ at day 39 after administration. The mean tumor volume of the test drug PR002418 (5 mg/kg) treatment group at day 39 after administration was 606 $mm^3$, showing a significant difference (p value was 0.015) from that of the vehicle control group, with the tumor growth inhibition rate TGI (%) being 42.45%. The mean tumor volume at day 39 after administration of test drug PR002418 (15 mg/kg) treatment group was 532 $mm^3$, showing a significant difference from that of the vehicle control group (p value was 0.007), with the tumor inhibition rate TGI (%) being 49.47%. The mean tumor volume of the test drug PR002421 (5 mg/kg) treatment group at day 39 after administration was 665 $mm^3$, showing no significant difference (p value was 0.07) from that of the vehicle control group, with the tumor growth inhibition rate TGI (%) being 36.86%. The mean tumor volume of the test drug PR002421 (15 mg/kg) treatment group at day 39 after administration was 335 $mm^3$, showing a significant difference (p value was 0.018) from that of the vehicle control group, with the tumor growth inhibition rate TGI (%) being 68.23%.

### 12.2. NSG mouse MDA-MB-468 tumor model with reconstruction of human PBMC immune system

On the day of cell inoculation, each NCG mouse was inoculated subcutaneously with 5 × $10^6$ tumor cells MDA-MB-468, the cells were resuspended in a PBS/Matrigel (1:1) mixture (0.1 mL/mouse) and inoculated subcutaneously. When the mean tumor volume of each group of mice reached 126 $mm^3$, 30 mice were divided into 5 groups, each mouse was inoculated intravenously with 5 × $10^6$ human PBMCs, and the cells were resuspended in 200 μL of PBS. The administration was started the next day with an administration cycle of twice a week for 8 administrations via intraperitoneal administration. After the start of administration, the body weight and the tumor volume were measured twice a week. The tumor volume was calculated as follows: tumor volume ($mm^3$) = 0.5 × long diameter of tumor × short diameter of tumor$^2$. The experimental observation was terminated 36 days after administration and then all mice were euthanized.

The *in vivo* anti-tumor efficacy of the NSG mouse MDA-MB-468 tumor model with reconstitution of human PBMC immune system is shown in FIG. 26, and specifically, the mice in the vehicle control group had a mean tumor volume of 942 $mm^3$ at day 36 after administration. The mean tumor volume of the test drug PR002418 (15 mg/kg) treatment group at day 36 after administration was 585 $mm^3$, showing no significant difference (p value was 0.073) from that of the vehicle control group, with the tumor growth inhibition rate TGI (%) being 37.91%. The mean tumor volume of the test drug PR002421 (15 mg/kg) treatment group at day 36 after administration was 670 $mm^3$, showing no significant difference (p value was 0.200) from that of the vehicle control group, with the tumor growth inhibition rate TGI (%) being 28.87%. The mean tumor volume of the test drug PR003369 (15 mg/kg) treatment group at day 36 after administration was 354 $mm^3$, showing a significant difference (p value was 0.008) from that of the vehicle control group, with the tumor growth inhibition rate TGI (%) being 62.38%. The mean tumor volume of the test drug control antibody 2 (15 mg/kg) treatment group at day 36 after administration was 533 $mm^3$, showing a significant difference (p value was 0.028) from that of the vehicle control group, with the tumor growth inhibition rate TGI (%) being 43.41%.

## Example 13. Immunohistochemical Staining (IHC)

The pathological tissue chip was purchased from Guilin Fanpu Biotech, Inc. It comprises a BRC1021 breast cancer tissue chip, an EMC1021 endometrial cancer tissue chip, an OVC1021 ovarian cancer tissue chip and an MNO1021 normal tissue chip. Paraffin sections had a thickness of 4 μm and were taken with positive control tissue. Dewaxing and washing were performed; antigen retrieval was performed as follows: citric acid at pH 6 was added, the mixture was heated at 125 °C for 5 minutes, sealed for 10 minutes, and cooled at room temperature for 30 minutes; the mixture was washed with water and then with 0.3% hydrogen peroxide for 5 minutes, and then washed with TBST for 3 times for 5 minutes; Dako blocking buffer was used directly, and blocked in an incubation box at room temperature for 20 minutes; the blocking buffer was removed, the primary antibody was added, the antibody diluent Dako was used directly, the mixture was incubated for 60 minutes in an incubation box at room temperature, and a control was substituted with Rabbit IgG; the mixture was washed with TBST for three times, five minutes each time; a secondary antibody, Anti-Rabbit (EnVision + System-HRP Labelled Polymer) was incubated in the incubation box for 30 minutes at room temperature; the mixture was washed with TBST for three times, five minutes each time; after DAB color development, 0.85 mL of distilled water was added into reagents according to the sequence of the reagents A to B to C in an amount of 50 μL, and the mixture was incubated for 5 minutes in an incubation box at room temperature, then washed with distilled water, and counterstained with hematoxylin. The chip was observed under a microscope followed by sealing and reading.

The results in FIG. 27 showed that B7H4 was slightly expressed in adrenal gland, renal cortex, bladder, breast, fallopian tube, esophagus, ureter and endometrium of normal tissues, and not expressed in other tissues (FIG. 27, A).

In contrast, it was highly expressed in breast cancer, ovarian cancer and endometriomas (FIG. 27, B). For example, in ovarian cancer, among the 102 total samples, 67.65% had IHC scores of 2-4, and in endometrioma, among the 98 total samples, 62.24% had IHC scores of 2-4 (FIG. 27, C).

**Example 14. Structure and Design of B7H4×CD3 Bispecific Antibodies**

Selected anti-B7H4 and anti-CD3 antibodies were used to prepare a bispecific antibody. The prepared B7H4×CD3 bispecific antibody can bind to two targets simultaneously, with one terminus being capable of recognizing B7H4 specifically expressed on tumor cell surfaces and the other terminus being capable of binding to CD3 molecules on T cells. After binding to the surface of a tumor cell, the B7H4×CD3 bispecific antibody molecule can recruit and activate T cells in the vicinity of the tumor cell, thereby killing the tumor cell.

A and B of FIG. 28 show "1 + 1" Fab-Fc-scFv asymmetric structural molecules; C and D show B7H4×CD3 bispecific antibody molecules having "1 + 1" Fab-Fc-crossFab asymmetric structures. For the "1 + 1" asymmetric structural molecules, structures (1) and (2) involve three protein chains, which comprise the heavy and light chains of the corresponding anti-B7H4 antibody, and the scFv polypeptide chain of the anti-CD3 antibody described above (see FIG. 28, A and B), respectively. For the "1 + 1" asymmetric structural molecules, structures (3) and (4) involve four protein chains, which comprise the heavy and light chains of the corresponding anti-B7H4 antibody, and the heavy and light chains of the anti-CD3 antibody described above (see FIG. 28, C and D), respectively.

E and F, G and H, and I and J show B7H4×CD3 bispecific antibody molecules having "2 + 1" asymmetric structures. For the "2 + 1" asymmetric structural molecules, structures (5) and (6) involve four protein chains, which comprise the heavy and light chains of the corresponding anti-B7H4 antibody, and the heavy and light chains of the anti-CD3 antibody described above (see FIG. 28, E and F), respectively. For the "2 + 1" asymmetric structural molecules, structures (7), (8), (9) and (10) involve three protein chains, which comprise the heavy and light chains of the corresponding anti-B7H4 antibody, and the scFv polypeptide chain of the anti-B7H4 and anti-CD3 antibodies described above (see FIG. 28, G, H, I and J), respectively.

To minimize the formation of byproducts with mismatched heavy chains (e.g., mismatching of two heavy chains of the anti-CD3 antibody), a mutant heterodimeric Fc region carrying a "knob-hole" mutation and a modified disulfide bond was used, as described in WO2009080251 and WO2009080252. The B7H4×CD3 bispecific antibody has an Fc of IgG1 and carries L234A, L235A and P329G (numbered according to the EU index) mutations on CH3 of the Fc. Each bispecific antibody was generated by co-transfecting simultaneously three or four different mammalian expression vectors encoding: 1) the heavy chain of the corresponding anti-B7H4 antibody, which carries a "Hole" mutation in the Fc region so as to produce a heterodimeric antibody, CH3 of the Fc carrying L234A, L235A and P329G mutations; 2) the heavy chain of the corresponding anti-CD3 antibody, which carries a "knob" mutation in the Fc region so as to produce a heterodimeric antibody, CH3 of the Fc carrying L234A, L235A and P329G mutations; 3) the light chain of the corresponding anti-CD3 antibody. 4) the light chain of the corresponding anti-B7H4 antibody. The "knob" mutation in the Fc region of human IgG1 consists of: T366W, and the "Hole" mutation consists of: T366S, L368A, and Y407V. In addition, S354C in the "knob" Fc region and "Hole" Y349C may be included; they form a pair of disulfide bonds to increase the stability and the yield of the heterodimeric antibody.

The B7H4×CD3 bispecific antibody molecules constructed in this example are listed in Tables 14-1, 14-2, and 14-3, with the structure numbers in the tables corresponding to FIG. 28. Table 14-4 shows the sequences of the linker peptides. The corresponding sequence numbers of the CD3 monoclonal antibody molecules are listed in Table 14-5. The B7H4 monoclonal antibody molecules are derived from Table 1-7. The corresponding sequence numbers of the B7H4×CD3 bispecific antibody molecules are listed in Table 14-6. The sequence numbers of the corresponding CDR sequences of the first and second antigen-binding domains of the bispecific antibody molecules are listed in Table 14-7.

Table 14-1. B7H4×CD3 bispecific antibody molecules having "1 + 1" Fab-Fc-scFv asymmetric structures

| Structure No. | Bispecific antibody molecules | CD3 antibody | B7H4 antibody | Structure of CD3 end | Structure of B7H4 end | Linker peptide (between VL_B and VH_B) | Fc type of CD3 end (mutation) | Fc type of B7H4 end (mutation) |
|---|---|---|---|---|---|---|---|---|
| **1** | PR002849 | PR000627 | PR001476 | scFv(VL-VH) | Fab | GS_15 | Human IgG1 (knob, AAG) | Human IgG1 (hole, AAG) |
| **1** | PR002850 | PR000627 | PR002405 | scFv(VL-VH) | Fab | GS_15 | Human IgG1 (knob, AAG) | Human IgG1 (hole, AAG) |
| **1** | PR002851 | PR000627 | PR002408 | scFv(VL-VH) | Fab | GS_15 | Human IgG1 (knob, AAG) | Human IgG1 (hole, AAG) |
| **1** | PR002852 | PR000627 | PR002410 | scFv(VL-VH) | Fab | GS_15 | Human IgG1 (knob, AAG) | Human IgG1 (hole, AAG) |
| **1** | PR003733 | PR001848 | PR002037 | Fab | scFv(VL-VH) | GS_20 | Human IgG1 (knob, LALA) | Human IgG 1 (hole, LALA) |
| **1** | PR003899 | PR003886 | PR002037 | Fab | scFv(VL-VH) | GS_20 | Human IgG1 (knob, LALA) | Human IgG1 (hole, LALA) |

Table 14-2. B7H4×CD3 bispecific antibody molecules having "1 + 1" Fab-Fc-crossFab asymmetric structures

| Structure No. | Bispecific antibody molecules | CD3 antibody | B7H4 antibody | Structure of CD3 end | Structure of B7H4 end | Fc type of CD3 end (mutation) | Fc type of B7H4 end (mutation) |
|---|---|---|---|---|---|---|---|
| **4** | PR002855 | PR001924 | PR001476 | Fab(cross Fd/LC) | Fab | Human IgG1 (knob, AAG) | Human IgG1 (hole, AAG) |
| **4** | PR002856 | PR001924 | PR002405 | Fab(cross Fd/LC) | Fab | Human IgG1 (knob, AAG) | Human IgG1 (hole, AAG) |
| **4** | PR002857 | PR001924 | PR002408 | Fab(cross Fd/LC) | Fab | Human IgG1 (knob, AAG) | Human IgG1 (hole, AAG) |
| **4** | PR002858 | PR001924 | PR002410 | Fab(cross Fd/LC) | Fab | Human IgG1 (knob, AAG) | Human IgG1 (hole, AAG) |

Table 14-3. B7H4×CD3 bispecific antibody molecules having "2 + 1" asymmetric structures

| Structure No. | Bispecific antibody molecules | CD3 antibody | B7H4 antibody | Structure of CD3 end | Structure of B7H4 end | Code of B7H4 end in structure illustration (A/B) | Fc type of CD3 end (mutation) | Fc type of B7H4 end (mutation) |
|---|---|---|---|---|---|---|---|---|
| **6** | PR002994 | PR001924 | PR001476 | Fab | Fab | B | Human IgG1 (knob, AAG) | Human IgG1 (hole, AAG) |
| **5** | PR002987 | PR001924 | PR001476 | Fab(cross Fd/LC) | Fab | B | Human IgG1 (knob, AAG) | Human IgG1 (hole, AAG) |
| **7** | PR003001 | PR000627 | PR001476 | scFv(VL-VH) | Fab | B | Human | Human |
| | | | | | | | IgG1 (knob, AAG) | IgG1 (hole, AAG) |
| **9** | PR003008 | PR000627 | PR001476 | scFv(VL-VH) | Fab | B | Human IgG1 (knob, LALA) | Human IgG1 (hole, LALA) |

**Table 14-4. Linker peptide sequences**

| Name of linker peptide | Length | Sequence | |
|---|---|---|---|
| GS_5 | 5 | GGGGS | SEQ ID NO: 133 |
| GS_15 | 15 | GGGQSGQGGSGGGGS | SEQ ID NO: 134 |
| GS_20 | 20 | GGGGSGGGGSGGGGSGGGGS | SEQ ID NO: 135 |

**Table 14-5. Sequence numbers of CD3 antibodies of this example**

| Antibody No. | Light chain | Heavy chain | VL | VH | LCDR1 | LCDR2 | LCDR3 | HCDR1 | HCDR2 | HCDR3 |
|---|---|---|---|---|---|---|---|---|---|---|
| **PR000627** | | 131 | 86 | 76 | 46 | 53 | 63 | 8 | 20 | 34 |
| **PR001924** | 111 | 97 | 86 | 78 | 46 | 53 | 63 | 10 | 20 | 34 |
| **PR001848** | 110 | 96 | 86 | 78 | 46 | 53 | 63 | 10 | 20 | 34 |
| **PR003886** | 110 | 107 | 86 | 84 | 46 | 53 | 63 | 10 | 20 | 34 |

**Table 14-6. Sequence numbers of B7H4×CD3 bispecific antibody molecules of this example**

| Antibody No. | Polypeptide chain-1 | Polypeptide chain-2 | Polypeptide chain-3 | Polypeptide chain-4 |
|---|---|---|---|---|
| **PR002849** | 109 | 118 | 119 | |
| **PR002850** | 114 | 120 | 119 | |
| **PR002851** | 114 | 121 | 119 | |
| **PR002852** | 115 | 122 | 119 | |
| **PR002855** | 97 | 123 | 118 | 109 |
| **PR002856** | 97 | 123 | 120 | 114 |
| **PR002857** | 97 | 123 | 121 | 114 |
| **PR002858** | 97 | 123 | 122 | 115 |
| **PR002987** | 97 | 124 | 118 | 109 |
| **PR002994** | 97 | 125 | 118 | 109 |
| **PR003001** | 126 | 118 | 109 | |
| **PR003008** | 127 | 118 | 109 | |
| **PR003733** | 110 | 128 | 129 | |
| **PR003899** | 110 | 130 | 129 | |

**Table 14-7. Sequence numbers of CDRs of antigen-binding domains of B7H4×CD3 bispecific antibody molecules**

| Structure No. | Antibody No. | Antigen-binding domain No. | LCDR1 | LCDR2 | LCDR3 | HCDR1 | HCDR2 | HCDR3 |
|---|---|---|---|---|---|---|---|---|
| **1** | PR002849 | #1 | 46 | 53 | 63 | 8 | 20 | 34 |
| | | #2 | 47 | 54 | 64 | 9 | 21 | 35 |
| **1** | PR002850 | #1 | 46 | 53 | 63 | 8 | 20 | 34 |
| | | #2 | 47 | 54 | 67 | 9 | 23 | 35 |

(continued)

| Structure No. | Antibody No. | Antigen-binding domain No. | LCDR1 | LCDR2 | LCDR3 | HCDR1 | HCDR2 | HCDR3 |
|---|---|---|---|---|---|---|---|---|
| **1** | PR002851 | #1 | 46 | 53 | 63 | 8 | 20 | 34 |
| | | #2 | 47 | 54 | 67 | 9 | 24 | 35 |
| **1** | PR002852 | #1 | 46 | 53 | 63 | 8 | 20 | 34 |
| | | #2 | 48 | 54 | 65 | 11 | 22 | 36 |
| **1** | PR003733 | #1 | 46 | 53 | 63 | 10 | 20 | 34 |
| | | #2 | 48 | 54 | 65 | 11 | 22 | 36 |
| **1** | PR003899 | #1 | 46 | 53 | 63 | 10 | 20 | 34 |
| | | #2 | 48 | 54 | 65 | 11 | 22 | 36 |
| **4** | PR002855 | #1 | 46 | 53 | 63 | 10 | 20 | 34 |
| | | #2 | 47 | 54 | 64 | 9 | 21 | 35 |
| **4** | PR002856 | #1 | 46 | 53 | 63 | 10 | 20 | 34 |
| | | #2 | 47 | 54 | 67 | 9 | 23 | 35 |
| **4** | PR002857 | #1 | 46 | 53 | 63 | 10 | 20 | 34 |
| | | #2 | 47 | 54 | 67 | 9 | 24 | 35 |
| **4** | PR002858 | #1 | 46 | 53 | 63 | 10 | 20 | 34 |
| | | #2 | 48 | 54 | 65 | 11 | 22 | 36 |
| **5** | PR002987 | #1 | 46 | 53 | 63 | 10 | 20 | 34 |
| | | #2 | 47 | 54 | 64 | 9 | 21 | 35 |
| **6** | PR002994 | #1 | 46 | 53 | 63 | 10 | 20 | 34 |
| | | #2 | 47 | 54 | 64 | 9 | 21 | 35 |
| **7** | PR003001 | #1 | 46 | 53 | 63 | 8 | 20 | 34 |
| | | #2 | 47 | 54 | 64 | 9 | 21 | 35 |
| **9** | PR003008 | #1 | 46 | 53 | 63 | 8 | 20 | 34 |
| | | #2 | 47 | 54 | 64 | 9 | 21 | 35 |

**Table 14-8. Expression of B7H4×CD3 bispecific antibody molecule proteins**

| Structure No. | Bispecific antibody molecules | Expression system and volume | Yield after first purification (mg/L) | SEC-HPLC purity (%) |
|---|---|---|---|---|
| **1** | PR002849 | HEK293F (30mL) | 32.5 | 84.69 |
| **1** | PR002850 | HEK293F (30mL) | 19.7 | 84.1 |
| **1** | PR002851 | HEK293F (30mL) | 23.1 | 83.56 |
| **1** | PR002852 | HEK293F (30mL) | 20.7 | 86.73 |
| **1** | PR003733 | ExpiCHO (1000mL) | 361 | 86.33 |
| **1** | PR003899 | ExpiCHO (1000mL) | 375.4 | 80.94 |
| **4** | PR002855 | HEK293F (30mL) | 52.2 | 73.08 |
| **4** | PR002856 | HEK293F (30mL) | 28.8 | 72.03 |

(continued)

| Structure No. | Bispecific antibody molecules | Expression system and volume | Yield after first purification (mg/L) | SEC-HPLC purity (%) |
|---|---|---|---|---|
| 4 | PR002857 | HEK293F (30mL) | 27.2 | 78.06 |
| 4 | PR002858 | HEK293F (30mL) | 57.5 | 91.2 |
| 6 | PR002994 | HEK293F (30mL) | 20.1 | 51.76 |
| 5 | PR002987 | HEK293F (30mL) | 17.7 | 81.48 |
| 7 | PR003001 | HEK293F (30mL) | 21 | 90.49 |
| 9 | PR003008 | HEK293F (30mL) | 17 | 86.17 |

**Example 15. Detection of Binding Ability of B7H4×CD3 Bispecific Antibodies to B7H4 by FACS**

SK-BR-3 cells were digested. T cells were isolated using the human T cell isolation kit (Miltenyi, #130-096-535) as described in the method of the instruction, and resuspended with PBS containing 2% BSA. The cell density was adjusted to $1\times10^6$ cells/mL. The cells were seeded in a 96-well V-bottom plate (Corning, #3894) at 100 μL/well, followed by the addition of test antibodies diluted in a 3-fold gradient at a concentration that was 2 times the final concentration, each at 100 μL/well. The cells were incubated at 4 °C for 2 h away from light. Thereafter, the cells in each well were rinsed twice with 100 μL of pre-cooled PBS containing 2% BSA, and centrifuged at 500 g at 4 °C for 5 min, and then the supernatant was discarded. Then each well was added with 100 μL of fluorescent secondary antibody (Alexa Fluor 488-conjugated AffiniPure Goat Anti-Human IgG, Fcγ Fragment Specific, Jackson, #109-545-098, diluted in a 1:500 ratio), and the plate was incubated away from light at 4 °C for 1 h. The cells in each well were washed twice with 100 μL of pre-cooled PBS containing 2% BSA, and centrifuged at 500 g at 4 °C for 5 minutes, and then the supernatant was discarded. Finally, the cells in each well were resuspended with 200 μL of pre-cooled PBS containing 2% BSA, and the fluorescence signal values were read using an ACEA Novocyte3000 flow cytometer.

The results of the B7H4×CD3 bispecific antibody molecules having the "1 + 1" asymmetric structures binding to SK-BR-3 cells are shown in FIG. 29, A-C. The results showed that the bispecific antibody molecules had stronger binding activity to B7H4 on the surface of the tumor cells SK-BR-3. FIG. 29A shows the binding activity of the bispecific antibody molecules having an Fab structure at the anti-B7H4 end and an ScFv polypeptide chain at the anti-CD3 end, wherein the bispecific antibody molecules PR002849, PR002850 and PR002851 with the anti-B7H4 end being derived from an ScFv polypeptide chain of PR001476 and its PTM mutant antibody had stronger binding activity than the bispecific antibody molecule PR002852 with the anti-B7H4 end being derived from an ScFv polypeptide chain of PR002037 and its PTM mutant antibody; FIG. 29B shows the binding activity of the bispecific antibody molecules having an Fab structure at the anti-B7H4 end and a Cross Fab structure at the anti-CD3 end, wherein the bispecific antibody molecules PR002855, PR002856 and PR002857 with the anti-B7H4 end being derived from PR001476 and its PTM mutant antibody had stronger binding activity than the bispecific antibody molecule PR002858 with the anti-B7H4 end being derived from PR002037 and its PTM mutant antibody; FIG. 29C shows the binding activity of the bispecific antibody molecules having an ScFv polypeptide chain at the anti-B7H4 end and an Fab structure at the anti-CD3 end, wherein the binding activity of PR003733 and PR003899 is consistent because both the anti-B7H4 ends of these two antibodies are the same ScFv polypeptide chain. The results of the B7H4×CD3 bispecific antibody molecules having the "2 + 1" asymmetric structures binding to SK-BR-3 cells are shown in FIG. 29, D. The results showed that the 2-valent anti-B7H4 end also had high binding activity. The results of the B7H4×CD3 bispecific antibody molecules having the "1 + 1" asymmetric structures binding to human T cells are shown in FIG. 29, E-G; the results of the B7H4×CD3 bispecific antibody molecules having the "2 + 1" asymmetric structures binding to human T cells are shown in FIG. 29, H. The results showed that the antibodies having an Fab structure or an ScFv polypeptide chain at the anti-CD3 end can bind to the surface of T cells, whereas the binding of the bispecific antibody molecule PR003899 with the weak anti-CD3 end did not exist at FACS level.

**Example 16. T Cell Killing Experiment**

In this experiment, human primary T cells were used as effector cells, and a cell line SK-BR-3 or MDA-MB-468 highly expressing B7H4, a cell line HCC-1954 moderately expressing B7H4, or a B7H4 negative cell line MDA-MB-231 was used as target cells. The killing efficiency was reflected by the conductivity of the target cell measured using an RTCA instrument from ACEA. A 96-well plate E-plate was first equilibrated with 50 μL of complete medium. Target cells were digested, resuspended in RPM1640 complete medium containing 10% fetal bovine serum and diluted to $4\times10^5$/mL. The cell suspension was plated on the 96-well E-plate at 50 μL/well, i.e., $2\times10^4$/well, and incubated overnight at 37 °C. The next day, primary T cells were isolated using the T cell isolation kit (Miltenyi, #130-096-535) according to the method of the instruction. 50 $\mu$L of fresh culture medium containing 2 $\times$ $10^5$ T cells was added to each well, followed by the addition of 50 $\mu$L of antibodies diluted in a 4$\times$ concentration gradient with a maximum final concentration of 10 nM. A total of 8 concentrations were set in duplicate for each antibody. The conductivity of the target cells was measured in real time. The value at hour 24 was used to calculate the target cell killing efficiency = (1 - sample/blank control) $\times$ 100%. The supernatant was collected after 24 hours and the concentration of IFN-$\gamma$ was detected by ELISA method. The instructions of IFN gamma Human Uncoated ELISA Kit (Thermo, #88-7316-77) were referred to for the ELISA method.

The results of the B7H4$\times$CD3 bispecific antibody molecules having the "1 + 1" asymmetric structures activating T cells and killing target cells SK-BR-3 are shown in FIG. 30, A-K. A and B of FIG. 30 show the killing activity of the bispecific antibody molecules having an Fab structure at the anti-B7H4 end and an ScFv polypeptide chain at the anti-CD3 end and the generation of cytokine IFN-$\gamma$, wherein the bispecific antibody molecule PR002852 with the anti-B7H4 end being derived from the ScFv polypeptide chain of PR002037 and its PTM mutant antibody had the strongest killing activity and the most generated cytokine IFN-$\gamma$. C and D of FIG. 30 show the killing activity of the bispecific antibody molecules having an Fab structure at the anti-B7H4 end and a Cross Fab structure at the anti-CD3 end and the generation of cytokine IFN-$\gamma$, wherein the bispecific antibody molecule PR002852 with the anti-B7H4 end being derived from PR002037 and its PTM mutant antibody had the strongest killing activity and the most generated cytokine IFN-$\gamma$. E and F of FIG. 30 show the killing activity of the bispecific antibody molecules having an ScFv polypeptide chain at the anti-B7H4 end and an Fab structure at the anti-CD3 end and the generation of cytokine IFN-$\gamma$, wherein the bispecific antibody molecule PR003733 with the strong CD3 end had stronger killing activity and more generated cytokine IFN-$\gamma$, than the bispecific antibody molecule PR003899 with the weak CD3 end. G-L of FIG. 30 compare the killing activity and cytokine secretion of PR003733 and PR003899 on other tumor cells MDA-MB-468, HCC-1954, and MDA-MB-231, and the results showed that PR003733 had stronger *in vitro* killing and more generated cytokine IFN-$\gamma$ on cells MDA-MB-468 and HCC-1954 than PR003899; MDA-MB-231 was a negative control cell that did not express B7H4, and neither these two antibodies had any effect. The results of the B7H4$\times$CD3 bispecific antibody molecules having the "2 + 1" asymmetric structures activating T cells and killing target cells SK-BR-3 are shown in FIG. 30, M-N. The bispecific antibody molecules having the "2 + 1" asymmetric structures can kill SK-BR-3 cells and generate cytokine IFN-$\gamma$, and the bispecific antibody molecules PR003001 and PR003008 having a Cross Fab structure at the anti-CD3 end had stronger killing activity and more generated cytokine IFN-$\gamma$ than the bispecific antibody molecules PR002987 and PR002994 having an ScFv polypeptide chain at the anti-CD3 end.

### Example 17. NSG Mouse Tumor Model with Reconstruction of Human PBMC Immune System

On the day of cell inoculation, each NCG mouse was inoculated subcutaneously with 5 $\times$ $10^6$ tumor cells MDA-MB-468, the cells were resuspended in a PBS/Matrigel (1:1) mixture (0.1 mL/mouse) and inoculated subcutaneously. When the mean tumor volume of each group of mice reached 126 $mm^3$, 18 mice were divided into 3 groups, each mouse was inoculated intravenously with 5 $\times$ $10^6$ human PBMCs, and the cells were resuspended with 200 $\mu$L of PBS. The administration was started the next day with an administration cycle of once a week for a total of 3 administrations via intravenous administration. After the start of administration, the body weight and the tumor volume were measured twice a week. The tumor volume was calculated as follows: tumor volume ($mm^3$) = 0.5 $\times$ long diameter of tumor $\times$ short diameter of tumor$^2$. The experimental observation was terminated 36 days after administration and then all mice were euthanized.

The mean tumor volume of the mice in the vehicle control group at day 36 after administration was 942 $mm^3$. The mean tumor volume of the test drug PR003733 (2 mg/kg) treatment group at day 36 after administration was 590 $mm^3$, showing a significant difference (p value was 0.048) from that of the vehicle control group, with the tumor growth inhibition rate TGI (%) being 37.31%. The mean tumor volume of the test drug PR003899 (2 mg/kg) treatment group at day 36 after administration was 0 $mm^3$, with complete regression of tumor, showing a significant difference (p value was 0.0001) from that of the vehicle control group, with the tumor growth inhibition rate TGI (%) being 100% (see FIG. 31, A).

In the HCC-1954 model, on the day of cell inoculation, each NCG mouse was inoculated subcutaneously with 5 $\times$ $10^6$ tumor cells HCC-1954, the cells were resuspended in a PBS/Matrigel (1:1) mixture (0.1 mL/mouse) and inoculated subcutaneously. When the mean tumor volume of each group of mice reached 102 $mm^3$, 15 mice were divided into 3 groups, each mouse was inoculated intravenously with 3 $\times$ $10^6$ human PBMCs, and the cells were resuspended with 200 $\mu$L of PBS. The administration was started the next day with a dosing period of once a week for a total of 2 administrations, via intravenous administration. After the start of administration, the body weight and the tumor volume were measured twice a week. The tumor volume was calculated as follows: tumor volume ($mm^3$) = 0.5 $\times$ long diameter of tumor $\times$ short diameter of tumor$^2$. The experiment was terminated 16 days after administration for observation and then all mice were euthanized.

The mean tumor volume of the mice in the vehicle control group at day 16 after administration was 622 $mm^3$. The mean tumor volume of the test drug PR003733 (0.5 mg/kg) treatment group at day 16 after administration was 450

$mm^3$, showing no significant difference (p value was 0.1) from that of the vehicle control group, with the tumor growth inhibition rate TGI (%) being 27.64%. The mean tumor volume of the test drug PR003899 (0.5 mg/kg) treatment group at day 16 after administration was 322 $mm^3$, showing a significant difference (p value was 0.0028) from that of the vehicle control group, with the tumor growth inhibition rate TGI (%) being 48.27% (see FIG. 31, B).

Pharmacodynamic experiments of the two tumor models showed that PR003899 had better efficacy than PR003733.

## Claims

1. A B7H4-targeting antibody or a variant thereof, wherein the antibody comprises a light chain variable region and a heavy chain variable region, wherein:

   the light chain variable region comprises a LCDR1, a LCDR2 and a LCDR3 with amino acid sequences as set forth in SEQ ID NOs: 47, 54 and 64, respectively; the heavy chain variable region comprises a HCDR1, a HCDR2 and a HCDR3 with amino acid sequences as set forth in SEQ ID NOs: 9, 21 and 35, respectively; or
   the light chain variable region comprises a LCDR1, a LCDR2 and a LCDR3 with amino acid sequences as set forth in SEQ ID NOs: 48, 54 and 65, respectively; the heavy chain variable region comprises a HCDR1, a HCDR2 and a HCDR3 with amino acid sequences as set forth in SEQ ID NOs: 11, 22 and 36, respectively;
   the variant has an amino acid mutation in the light chain variable region and/or the heavy chain variable region of the antibody and maintains the function of the antibody; preferably, the amino acid mutation is an amino acid substitution, and the number of the amino acid substitutions is 1 to 3.

2. The antibody or the variant thereof according to claim 1, wherein the variant has an amino acid substitution at position 3 or 4 of the HCDR2 and position 3 of the HCDR3 in the heavy chain variable region of the antibody, and/or an amino acid substitution at position 4 of the LCDR3 in the light chain variable region of the antibody; preferably,

   the light chain variable region comprises a LCDR1, a LCDR2 and a LCDR3 with amino acid sequences as set forth in SEQ ID NOs: 48, 55 and 66, respectively; the heavy chain variable region comprises a HCDR1, a HCDR2 and a HCDR3 with amino acid sequences as set forth in SEQ ID NOs: 11, 22 and 36, respectively; or
   the light chain variable region comprises a LCDR1, a LCDR2 and a LCDR3 with amino acid sequences as set forth in SEQ ID NOs: 47, 54 and 67, respectively; the heavy chain variable region comprises a HCDR1, a HCDR2 and a HCDR3 with amino acid sequences as set forth in SEQ ID NOs: 9, 23 and 35, respectively; or
   the light chain variable region comprises a LCDR1, a LCDR2 and a LCDR3 with amino acid sequences as set forth in SEQ ID NOs: 47, 54 and 67, respectively; the heavy chain variable region comprises a HCDR1, a HCDR2 and a HCDR3 with amino acid sequences as set forth in SEQ ID NOs: 9, 24 and 35, respectively; or
   the light chain variable region comprises a LCDR1, a LCDR2 and a LCDR3 with amino acid sequences as set forth in SEQ ID NOs: 47, 54 and 69, respectively; the heavy chain variable region comprises a HCDR1, a HCDR2 and a HCDR3 with amino acid sequences as set forth in SEQ ID NOs: 9, 24 and 38, respectively.

3. The antibody or the variant thereof according to claim 1 or 2, wherein the heavy chain variable region further comprises a heavy chain variable region framework region HFWR, and/or the light chain variable region further comprises a light chain variable region framework region LFWR, wherein the HFWR is a heavy chain variable region framework region of a human antibody and the LFWR is a light chain variable region framework region of a human antibody; preferably,

   the light chain variable region comprises an amino acid sequence as set forth in SEQ ID NO: 87; the heavy chain variable region comprises an amino acid sequence as set forth in SEQ ID NO: 77; or,
   the light chain variable region comprises an amino acid sequence as set forth in SEQ ID NO: 88; the heavy chain variable region comprises an amino acid sequence as set forth in SEQ ID NO: 79; or,
   the light chain variable region comprises an amino acid sequence as set forth in SEQ ID NO: 89; the heavy chain variable region comprises an amino acid sequence as set forth in SEQ ID NO: 79; or,
   the light chain variable region comprises an amino acid sequence as set forth in SEQ ID NO: 90; the heavy chain variable region comprises an amino acid sequence as set forth in SEQ ID NO: 80; or,
   the light chain variable region comprises an amino acid sequence as set forth in SEQ ID NO: 90; the heavy chain variable region comprises an amino acid sequence as set forth in SEQ ID NO: 81; or,
   the light chain variable region comprises an amino acid sequence as set forth in SEQ ID NO: 93; the heavy chain variable region comprises an amino acid sequence as set forth in SEQ ID NO: 83; or,

the light chain variable region comprises an amino acid sequence as set forth in SEQ ID NO: 91; the heavy chain variable region comprises an amino acid sequence as set forth in SEQ ID NO: 79.

**4.** The antibody or the variant thereof according to any one of claims 1 to 3, wherein the antibody further comprises a heavy chain constant region and/or a light chain constant region; preferably, the heavy chain constant region of the antibody is selected from that of hIgG1, hIgG2, hIgG3 and hIgG4, and the light chain constant region is selected from that of a κ chain and a λ chain; more preferably, the variant has an amino acid substitution at position 239 and/or 332 of an Fc of the antibody, and preferably, the amino acid substitution is S239D and/or I332E.

**5.** The antibody or the variant thereof according to any one of claims 1 to 4, wherein the antibody is a full-length antibody, an Fab, an Fab', an $F(ab')_2$, an Fv, an scFv, or a monoclonal or polyclonal antibody prepared from an antibody as above.

**6.** The antibody according to claim 5, comprising

(1) a heavy chain and a light chain, wherein the heavy chain comprises an amino acid sequence as set forth in SEQ ID NO: 95; the light chain comprises an amino acid sequence as set forth in SEQ ID NO: 109; or, the heavy chain comprises an amino acid sequence as set forth in SEQ ID NO: 98; the light chain comprises an amino acid sequence as set forth in SEQ ID NO: 112; or,

the heavy chain comprises an amino acid sequence as set forth in SEQ ID NO: 98; the light chain comprises an amino acid sequence as set forth in SEQ ID NO: 113; or,
the heavy chain comprises an amino acid sequence as set forth in SEQ ID NO: 99; the light chain comprises an amino acid sequence as set forth in SEQ ID NO: 114; or,
the heavy chain comprises an amino acid sequence as set forth in SEQ ID NO: 100; the light chain comprises an amino acid sequence as set forth in SEQ ID NO: 114; or,
the heavy chain comprises an amino acid sequence as set forth in SEQ ID NO: 101; the light chain comprises an amino acid sequence as set forth in SEQ ID NO: 109; or,
the heavy chain comprises an amino acid sequence as set forth in SEQ ID NO: 102; the light chain comprises an amino acid sequence as set forth in SEQ ID NO: 114; or,
the heavy chain comprises an amino acid sequence as set forth in SEQ ID NO: 106; the light chain comprises an amino acid sequence as set forth in SEQ ID NO: 117; or,
the heavy chain comprises an amino acid sequence as set forth in SEQ ID NO: 98; the light chain comprises an amino acid sequence as set forth in SEQ ID NO: 115; or,
the heavy chain comprises an amino acid sequence as set forth in SEQ ID NO: 103; the light chain comprises an amino acid sequence as set forth in SEQ ID NO: 112; or,
the heavy chain comprises an amino acid sequence as set forth in SEQ ID NO: 103; the light chain comprises an amino acid sequence as set forth in SEQ ID NO: 115; or,

(2) a heavy chain, wherein the heavy chain comprises an amino acid sequence as set forth in SEQ ID NO: 132.

**7.** A B7H4-targeting bispecific antibody comprising a protein functional region A and a protein functional region B, wherein the protein functional region A is the B7H4-targeting antibody according to any one of claims 1 to 6; the protein functional region B is an antibody not targeting B7H4; preferably, the antibody not targeting B7H4 is a CD3-targeting antibody; more preferably, the CD3-targeting antibody comprises a light chain variable region and a heavy chain variable region, wherein the light chain variable region comprises a LCDR1, a LCDR2 and a LCDR3 with amino acid sequences as set forth in SEQ ID NOs: 46, 53 and 63, respectively; the heavy chain variable region comprises a HCDR1, a HCDR2 and a HCDR3 with amino acid sequences as set forth in SEQ ID NOs: 8, 20 and 34, respectively; or the light chain variable region comprises a LCDR1, a LCDR2 and a LCDR3 with amino acid sequences as set forth in SEQ ID NOs: 46, 53 and 63, respectively; the heavy chain variable region comprises a HCDR1, a HCDR2 and a HCDR3 with amino acid sequences as set forth in SEQ ID NOs: 10, 20 and 34, respectively; and even more preferably, in the CD3-targeting antibody, the light chain variable region comprises an amino acid sequence as set forth in SEQ ID NO: 86; the heavy chain variable region comprises an amino acid sequence as set forth in SEQ ID NO: 76; or the light chain variable region VL comprises an amino acid sequence as set forth in SEQ ID NO: 86; the heavy chain variable region VH comprises an amino acid sequence as set forth in SEQ ID NO: 78; or the light chain variable region VL comprises an amino acid sequence as set forth in SEQ ID NO: 86; the heavy chain variable region VH comprises an amino acid sequence as set forth in SEQ ID NO: 84.

**8.** The bispecific antibody according to claim 7, wherein the protein functional region B comprises a light chain variable region and a heavy chain variable region, and the protein functional region A comprises a light chain variable region and a heavy chain variable region; wherein,

in the protein functional region B, the light chain variable region comprises a LCDR1, a LCDR2 and a LCDR3 with amino acid sequences as set forth in SEQ ID NOs: 46, 53 and 63, respectively, and the heavy chain variable region comprises a HCDR1, a HCDR2 and a HCDR3 with amino acid sequences as set forth in SEQ ID NOs: 8, 20 and 34, respectively; in the protein functional region A, the light chain variable region comprises a LCDR1, a LCDR2 and a LCDR3 with amino acid sequences as set forth in SEQ ID NOs: 47, 54 and 64, respectively, and the heavy chain variable region comprises a HCDR1, a HCDR2 and a HCDR3 with amino acid sequences as set forth in SEQ ID NOs: 9, 21 and 35, respectively; or,
in the protein functional region B, the light chain variable region comprises a LCDR1, a LCDR2 and a LCDR3 with amino acid sequences as set forth in SEQ ID NOs: 46, 53 and 63, respectively, and the heavy chain variable region comprises a HCDR1, a HCDR2 and a HCDR3 with amino acid sequences as set forth in SEQ ID NOs: 8, 20 and 34, respectively; in the protein functional region A, the light chain variable region comprises a LCDR1, a LCDR2 and a LCDR3 with amino acid sequences as set forth in SEQ ID NOs: 47, 54 and 67, respectively, and the heavy chain variable region comprises a HCDR1, a HCDR2 and a HCDR3 with amino acid sequences as set forth in SEQ ID NOs: 9, 23 and 35, respectively; or,
in the protein functional region B, the light chain variable region comprises a LCDR1, a LCDR2 and a LCDR3 with amino acid sequences as set forth in SEQ ID NOs: 46, 53 and 63, respectively, and the heavy chain variable region comprises a HCDR1, a HCDR2 and a HCDR3 with amino acid sequences as set forth in SEQ ID NOs: 8, 20 and 34, respectively; in the protein functional region A, the light chain variable region comprises a LCDR1, a LCDR2 and a LCDR3 with amino acid sequences as set forth in SEQ ID NOs: 47, 54 and 67, respectively, and the heavy chain variable region comprises a HCDR1, a HCDR2 and a HCDR3 with amino acid sequences as set forth in SEQ ID NOs: 9, 24 and 35, respectively; or,
in the protein functional region B, the light chain variable region comprises a LCDR1, a LCDR2 and a LCDR3 with amino acid sequences as set forth in SEQ ID NOs: 46, 53 and 63, respectively, and the heavy chain variable region comprises a HCDR1, a HCDR2 and a HCDR3 with amino acid sequences as set forth in SEQ ID NOs: 8, 20 and 34, respectively; in the protein functional region A, the light chain variable region comprises a LCDR1, a LCDR2 and a LCDR3 with amino acid sequences as set forth in SEQ ID NOs: 48, 54 and 65, respectively, and the heavy chain variable region comprises a HCDR1, a HCDR2 and a HCDR3 with amino acid sequences as set forth in SEQ ID NOs: 11, 22 and 36, respectively; or,
in the protein functional region B, the light chain variable region comprises a LCDR1, a LCDR2 and a LCDR3 with amino acid sequences as set forth in SEQ ID NOs: 46, 53 and 63, respectively, and the heavy chain variable region comprises a HCDR1, a HCDR2 and a HCDR3 with amino acid sequences as set forth in SEQ ID NOs: 10, 20 and 34, respectively; in the protein functional region A, the light chain variable region comprises a LCDR1, a LCDR2 and a LCDR3 with amino acid sequences as set forth in SEQ ID NOs: 47, 54 and 64, respectively, and the heavy chain variable region comprises a HCDR1, a HCDR2 and a HCDR3 with amino acid sequences as set forth in SEQ ID NOs: 9, 21 and 35, respectively; or,
in the protein functional region B, the light chain variable region comprises a LCDR1, a LCDR2 and a LCDR3 with amino acid sequences as set forth in SEQ ID NOs: 46, 53 and 63, respectively, and the heavy chain variable region comprises a HCDR1, a HCDR2 and a HCDR3 with amino acid sequences as set forth in SEQ ID NOs: 10, 20 and 34, respectively; in the protein functional region A, the light chain variable region comprises a LCDR1, a LCDR2 and a LCDR3 with amino acid sequences as set forth in SEQ ID NOs: 47, 54 and 67, respectively, and the heavy chain variable region comprises a HCDR1, a HCDR2 and a HCDR3 with amino acid sequences as set forth in SEQ ID NOs: 9, 23 and 35, respectively; or,
in the protein functional region B, the light chain variable region comprises a LCDR1, a LCDR2 and a LCDR3 with amino acid sequences as set forth in SEQ ID NOs: 46, 53 and 63, respectively, and the heavy chain variable region comprises a HCDR1, a HCDR2 and a HCDR3 with amino acid sequences as set forth in SEQ ID NOs: 10, 20 and 34, respectively; in the protein functional region A, the light chain variable region comprises a LCDR1, a LCDR2 and a LCDR3 with amino acid sequences as set forth in SEQ ID NOs: 47, 54 and 67, respectively, and the heavy chain variable region comprises a HCDR1, a HCDR2 and a HCDR3 with amino acid sequences as set forth in SEQ ID NOs: 9, 24 and 35, respectively; or,
in the protein functional region B, the light chain variable region comprises a LCDR1, a LCDR2 and a LCDR3 with amino acid sequences as set forth in SEQ ID NOs: 46, 53 and 63, respectively, and the heavy chain variable region comprises a HCDR1, a HCDR2 and a HCDR3 with amino acid sequences as set forth in SEQ ID NOs: 10, 20 and 34, respectively; in the protein functional region A, the light chain variable region comprises a LCDR1, a LCDR2 and a LCDR3 with amino acid sequences as set forth in SEQ ID NOs: 48, 54 and 65, respectively, and the heavy chain variable region comprises a HCDR1, a HCDR2 and a HCDR3 with amino acid sequences as set forth in SEQ ID NOs: 11, 22 and 36, respectively.

**9.** The bispecific antibody according to claim 8, wherein the protein functional region B comprises a light chain variable region and a heavy chain variable region, and the protein functional region A comprises a light chain variable region and a heavy chain variable region; wherein,

in the protein functional region B, the light chain variable region comprises an amino acid sequence as set forth in SEQ ID NO: 86, and the heavy chain variable region comprises an amino acid sequence as set forth in SEQ ID NO: 76; in the protein functional region A, the light chain variable region comprises an amino acid sequence as set forth in SEQ ID NO: 87, and the heavy chain variable region comprises an amino acid sequence as set forth in SEQ ID NO: 77; or,
in the protein functional region B, the light chain variable region comprises an amino acid sequence as set forth in SEQ ID NO: 86, and the heavy chain variable region comprises an amino acid sequence as set forth in SEQ ID NO: 76; in the protein functional region A, the light chain variable region comprises an amino acid sequence as set forth in SEQ ID NO: 90, and the heavy chain variable region comprises an amino acid sequence as set forth in SEQ ID NO: 80; or,
in the protein functional region B, the light chain variable region comprises an amino acid sequence as set forth in SEQ ID NO: 86, and the heavy chain variable region comprises an amino acid sequence as set forth in SEQ ID NO: 76; in the protein functional region A, the light chain variable region comprises an amino acid sequence as set forth in SEQ ID NO: 90, and the heavy chain variable region comprises an amino acid sequence as set forth in SEQ ID NO: 81; or,
in the protein functional region B, the light chain variable region comprises an amino acid sequence as set forth in SEQ ID NO: 86, and the heavy chain variable region comprises an amino acid sequence as set forth in SEQ ID NO: 76; in the protein functional region A, the light chain variable region comprises an amino acid sequence as set forth in SEQ ID NO: 91, and the heavy chain variable region comprises an amino acid sequence as set forth in SEQ ID NO: 79; or,
in the protein functional region B, the light chain variable region comprises an amino acid sequence as set forth in SEQ ID NO: 86, and the heavy chain variable region comprises an amino acid sequence as set forth in SEQ ID NO: 78; in the protein functional region A, the light chain variable region comprises an amino acid sequence as set forth in SEQ ID NO: 87, and the heavy chain variable region comprises an amino acid sequence as set forth in SEQ ID NO: 77; or,
in the protein functional region B, the light chain variable region comprises an amino acid sequence as set forth in SEQ ID NO: 86, and the heavy chain variable region comprises an amino acid sequence as set forth in SEQ ID NO: 78; in the protein functional region A, the light chain variable region comprises an amino acid sequence as set forth in SEQ ID NO: 90, and the heavy chain variable region comprises an amino acid sequence as set forth in SEQ ID NO: 80; or,
in the protein functional region B, the light chain variable region comprises an amino acid sequence as set forth in SEQ ID NO: 86, and the heavy chain variable region comprises an amino acid sequence as set forth in SEQ ID NO: 78; in the protein functional region A, the light chain variable region comprises an amino acid sequence as set forth in SEQ ID NO: 90, and the heavy chain variable region comprises an amino acid sequence as set forth in SEQ ID NO: 81; or,
in the protein functional region B, the light chain variable region comprises an amino acid sequence as set forth in SEQ ID NO: 86, and the heavy chain variable region comprises an amino acid sequence as set forth in SEQ ID NO: 78; in the protein functional region A, the light chain variable region comprises an amino acid sequence as set forth in SEQ ID NO: 91, and the heavy chain variable region comprises an amino acid sequence as set forth in SEQ ID NO: 79; or,
in the protein functional region B, the light chain variable region comprises an amino acid sequence as set forth in SEQ ID NO: 86, and the heavy chain variable region comprises an amino acid sequence as set forth in SEQ ID NO: 84; in the protein functional region A, the light chain variable region comprises an amino acid sequence as set forth in SEQ ID NO: 91, and the heavy chain variable region comprises an amino acid sequence as set forth in SEQ ID NO: 79.

**10.** The bispecific antibody according to claim 9, wherein the bispecific antibody is selected from the group consisting of:

(1) the bispecific antibody comprises three polypeptide chains, wherein:

a first polypeptide chain comprises an amino acid sequence as set forth in SEQ ID NO: 109; a second polypeptide chain comprises an amino acid sequence as set forth in SEQ ID NO: 118; a third polypeptide chain comprises an amino acid sequence as set forth in SEQ ID NO: 119; or,
a first polypeptide chain comprises an amino acid sequence as set forth in SEQ ID NO: 114; a second polypeptide chain comprises an amino acid sequence as set forth in SEQ ID NO: 120; a third polypeptide chain comprises an amino acid sequence as set forth in SEQ ID NO: 119; or,
a first polypeptide chain comprises an amino acid sequence as set forth in SEQ ID NO: 114; a second polypeptide chain comprises an amino acid sequence as set forth in SEQ ID NO: 121; a third polypeptide chain comprises an amino acid sequence as set forth in SEQ ID NO: 119; or,
a first polypeptide chain comprises an amino acid sequence as set forth in SEQ ID NO: 115; a second polypeptide chain comprises an amino acid sequence as set forth in SEQ ID NO: 122; a third polypeptide chain comprises an amino acid sequence as set forth in SEQ ID NO: 119; or,
a first polypeptide chain comprises an amino acid sequence as set forth in SEQ ID NO: 126; a second polypeptide chain comprises an amino acid sequence as set forth in SEQ ID NO: 118; a third polypeptide chain comprises an amino acid sequence as set forth in SEQ ID NO: 109; or,
a first polypeptide chain comprises an amino acid sequence as set forth in SEQ ID NO: 127; a second polypeptide chain comprises an amino acid sequence as set forth in SEQ ID NO: 118; a third polypeptide chain comprises an amino acid sequence as set forth in SEQ ID NO: 109; or,
a first polypeptide chain comprises an amino acid sequence as set forth in SEQ ID NO: 110; a second polypeptide chain comprises an amino acid sequence as set forth in SEQ ID NO: 128; a third polypeptide chain comprises an amino acid sequence as set forth in SEQ ID NO: 129; or,
a first polypeptide chain comprises an amino acid sequence as set forth in SEQ ID NO: 110; a second polypeptide chain comprises an amino acid sequence as set forth in SEQ ID NO: 130; a third polypeptide chain comprises an amino acid sequence as set forth in SEQ ID NO: 129;

(2) the bispecific antibody comprises four polypeptide chains, wherein:

a first polypeptide chain comprises an amino acid sequence as set forth in SEQ ID NO: 97; a second polypeptide chain comprises an amino acid sequence as set forth in SEQ ID NO: 123; a third polypeptide chain comprises an amino acid sequence as set forth in SEQ ID NO: 118; a fourth polypeptide chain comprises an amino acid sequence as set forth in SEQ ID NO: 109; or,
a first polypeptide chain comprises an amino acid sequence as set forth in SEQ ID NO: 97; a second polypeptide chain comprises an amino acid sequence as set forth in SEQ ID NO: 123; a third polypeptide chain comprises an amino acid sequence as set forth in SEQ ID NO: 120; a fourth polypeptide chain comprises an amino acid sequence as set forth in SEQ ID NO: 114; or,
a first polypeptide chain comprises an amino acid sequence as set forth in SEQ ID NO: 97; a second polypeptide chain comprises an amino acid sequence as set forth in SEQ ID NO: 123; a third polypeptide chain comprises an amino acid sequence as set forth in SEQ ID NO: 121; a fourth polypeptide chain comprises an amino acid sequence as set forth in SEQ ID NO: 114; or,
a first polypeptide chain comprises an amino acid sequence as set forth in SEQ ID NO: 97; a second polypeptide chain comprises an amino acid sequence as set forth in SEQ ID NO: 123; a third polypeptide chain comprises an amino acid sequence as set forth in SEQ ID NO: 122; a fourth polypeptide chain comprises an amino acid sequence as set forth in SEQ ID NO: 115; or,
a first polypeptide chain comprises an amino acid sequence as set forth in SEQ ID NO: 97; a second polypeptide chain comprises an amino acid sequence as set forth in SEQ ID NO: 124; a third polypeptide chain comprises an amino acid sequence as set forth in SEQ ID NO: 118; a fourth polypeptide chain comprises an amino acid sequence as set forth in SEQ ID NO: 109; or,
a first polypeptide chain comprises an amino acid sequence as set forth in SEQ ID NO: 97; a second polypeptide chain comprises an amino acid sequence as set forth in SEQ ID NO: 125; a third polypeptide chain comprises an amino acid sequence as set forth in SEQ ID NO: 118; a fourth polypeptide chain comprises an amino acid sequence as set forth in SEQ ID NO: 109.

**11.** A chimeric antigen receptor comprising the antibody according to any one of claims 1 to 6 or the bispecific antibody according to any one of claims 7 to 10.

**12.** A genetically modified cell comprising the chimeric antigen receptor according to claim 11, wherein the cell is preferably a eukaryotic cell, more preferably an isolated human cell, and even more preferably an immune cell such as a T cell or an NK cell.

**13.** An isolated nucleic acid encoding the antibody according to any one of claims 1 to 6, the bispecific antibody according to any one of claims 7 to 10, or the chimeric antigen receptor according to claim 11.

**14.** An expression vector comprising the isolated nucleic acid according to claim 13.

**15.** A transformant comprising the expression vector according to claim 14, wherein the transformant is prepared by transforming the recombinant expression vector into a host cell, wherein the host cell is preferably a prokaryotic cell or a eukaryotic cell.

**16.** A method for preparing a B7H4-targeting antibody or bispecific antibody, comprising the following steps: culturing the transformant according to claim 15, and obtaining the B7H4-targeting antibody or bispecific antibody from the culture.

**17.** An antibody-drug conjugate comprising an antibody moiety and a conjugate moiety, wherein the antibody moiety comprises the antibody according to any one of claims 1 to 6 or the bispecific antibody according to any one of claims 7 to 10, and the conjugate moiety includes, but is not limited to, a detectable label, a drug, a toxin, a cytokine, a radionuclide, an enzyme, or a combination thereof, wherein the antibody moiety and the conjugate moiety are conjugated via a chemical bond or a linker.

**18.** A pharmaceutical composition comprising the antibody according to any one of claims 1 to 6 or the bispecific antibody according to any one of claims 7 to 10 and optionally a pharmaceutically acceptable carrier.

**19.** Use of the antibody according to any one of claims 1 to 6, the bispecific antibody according to any one of claims 7 to 10, the chimeric antigen receptor according to claim 11, the immune cell according to claim 12, the antibody-drug conjugate according to claim 17 or the pharmaceutical composition according to claim 18 in the manufacture of a medicament, a kit and/or an administration device for the treatment and/or prevention of a cancer, wherein preferably, the cancer is a B7H4 positive tumor, and more preferably breast cancer, ovarian cancer and endometrioma, the breast cancer being, for example, triple negative breast cancer.

**20.** A method for the detection of B7H4 in a sample, comprising conducting detection using the antibody according to any one of claims 1 to 6 or the bispecific antibody according to any one of claims 7 to 10, wherein preferably, the method is for non-diagnostic purposes.

**21.** A kit comprising the antibody according to any one of claims 1 to 6, the bispecific antibody according to any one of claims 7 to 10, the chimeric antigen receptor according to claim 11, the immune cell according to claim 12, the antibody-drug conjugate according to claim 17 and/or the pharmaceutical composition according to claim 18, and optionally instructions for use.

**22.** An administration device comprising: (1) an infusion module for administering to a subject in need thereof the pharmaceutical composition according to claim 18, and (2) optionally a pharmacodynamic monitoring module.

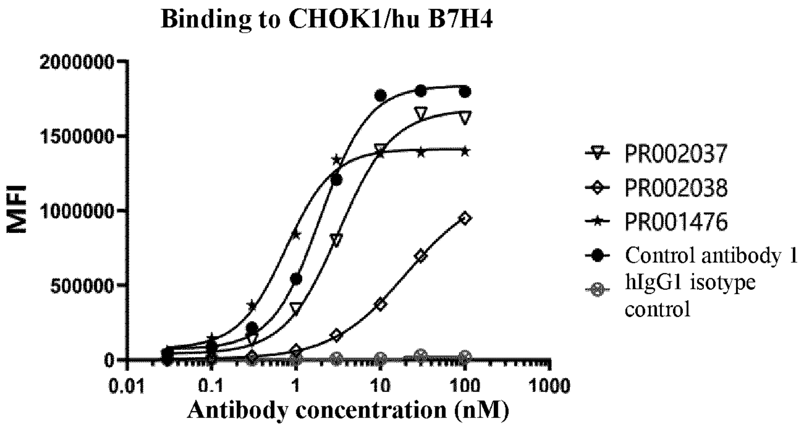


| Antibody | EC50 (nM) | Maximum MFI |
|---|---|---|
| PR002037 | 3.196 | 1677000 |
| PR002038 | 20.04 | 1146000 |
| PR001476 | 0.7552 | 1412000 |
| Control antibody 1 | 1.943 | 1839000 |

FIG. 1

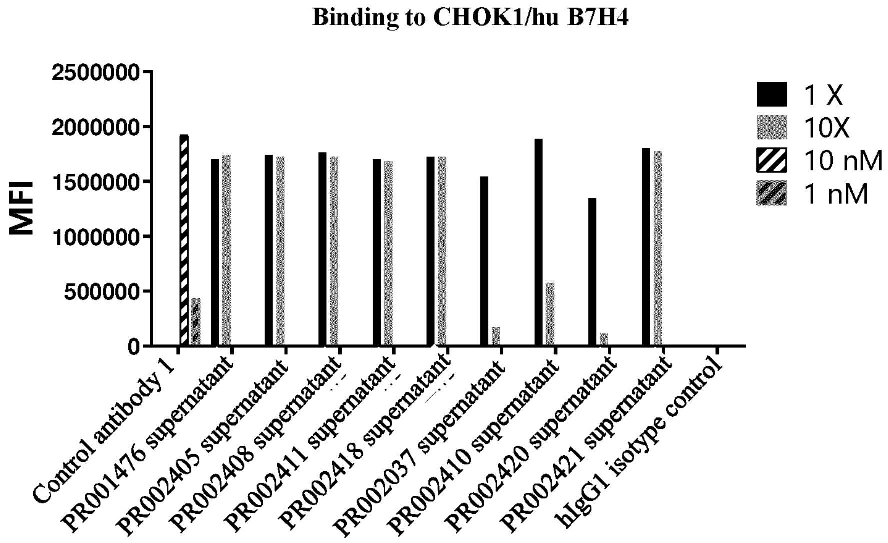


FIG. 2

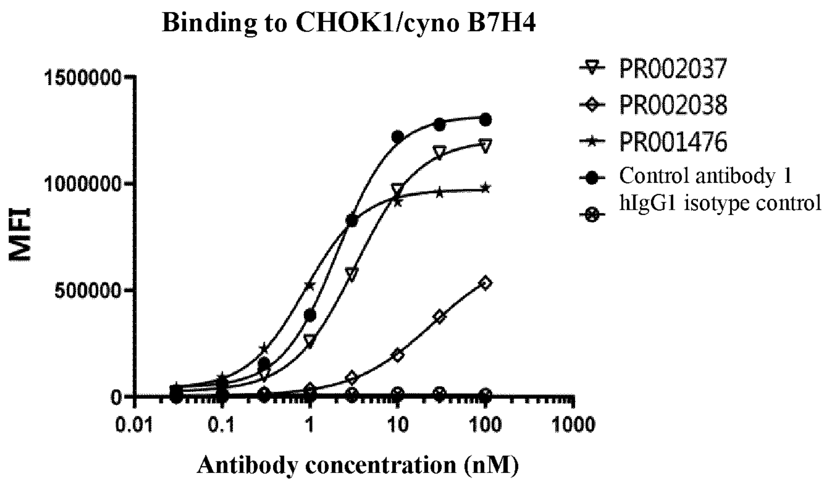


| Antibody | EC50 (nM) | Maximum MFI |
|---|---|---|
| PR002037 | 3.301 | 1207000 |
| PR002038 | 24.76 | 677326 |
| PR001476 | 0.8861 | 971785 |
| Control antibody 1 | 2.057 | 1317000 |

FIG. 3

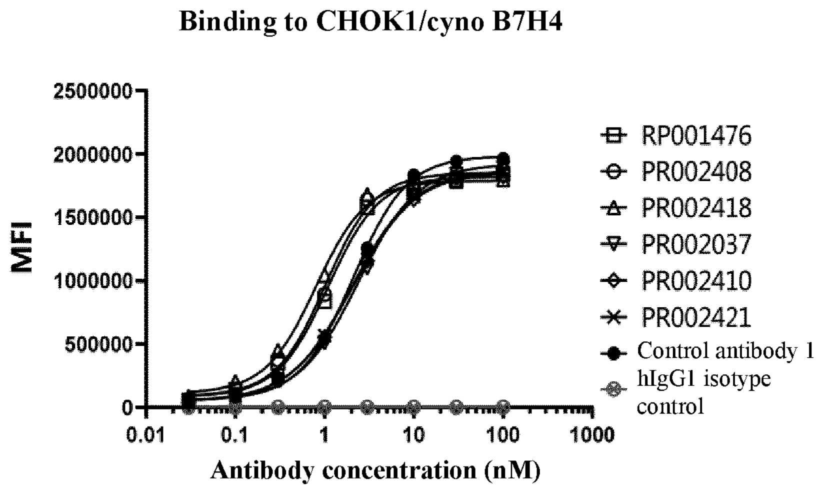


| Antibody | EC50 (nM) | Maximum MFI |
|---|---|---|
| RP001476 | 1.097 | 1822000 |
| PR002408 | 1.029 | 1852000 |
| PR002418 | 0.7985 | 1790000 |
| PR002037 | 2.382 | 1922000 |
| PR002410 | 2.077 | 1862000 |
| PR002421 | 2.048 | 1873000 |
| Control antibody 1 | 2.095 | 1986000 |

FIG. 4

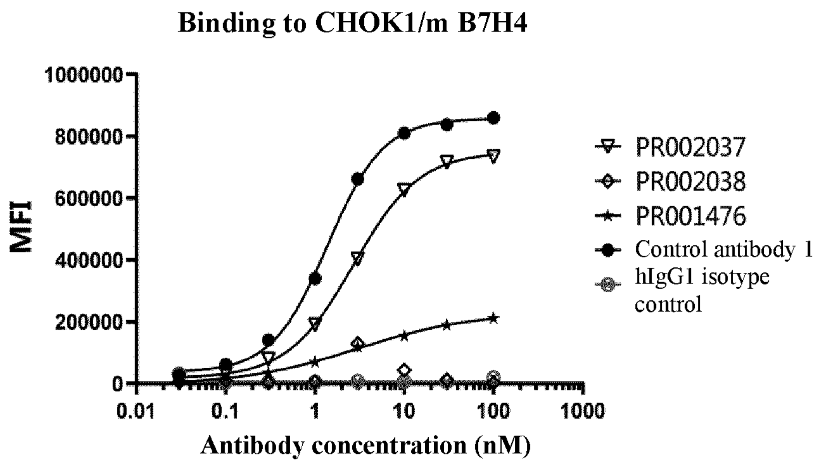


| Antibody | EC50 (nM) | Maximum MFI |
|---|---|---|
| PR002037 | 2.698 | 750723 |
| PR002038 | n.d. | 3147 |
| PR001476 | 3.018 | 230031 |
| Control antibody 1 | 1.381 | 857700 |

FIG. 5

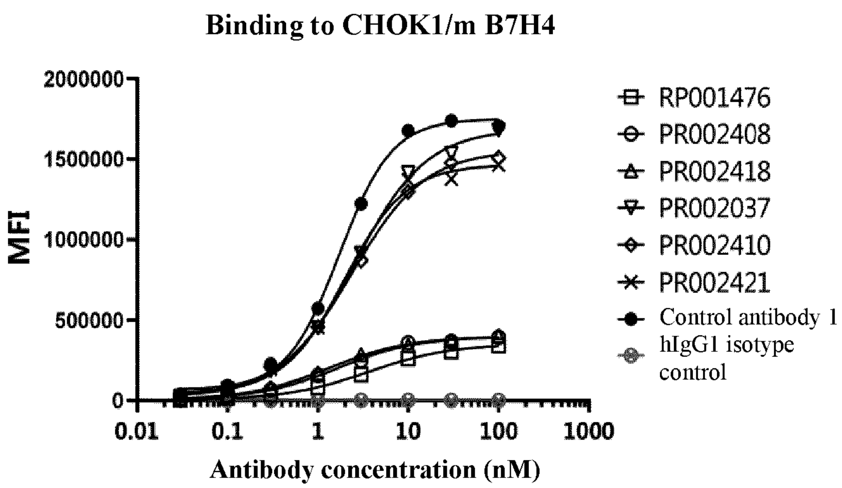


| Antibody | EC50 (nM) | Maximum MFI |
|---|---|---|
| RP001476 | 3.614 | 353378 |
| PR002408 | 1.678 | 398460 |
| PR002418 | 1.306 | 398407 |
| PR002037 | 2.57 | 1686000 |
| PR002410 | 2.385 | 1554000 |
| PR002421 | 1.988 | 1465000 |
| Control antibody 1 | 1.728 | 1749000 |

FIG. 6

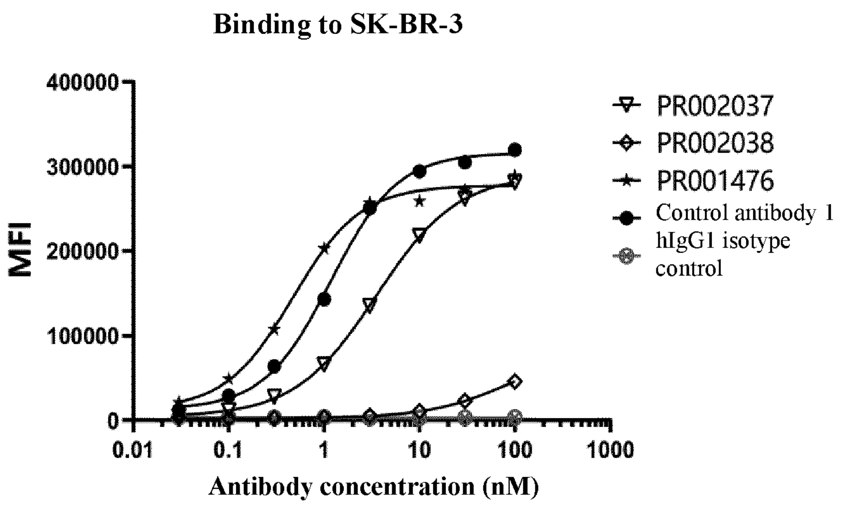


| Antibody | EC50 (nM) | Maximum MFI |
|---|---|---|
| PR002037 | 3.527 | 290692 |
| PR002038 | 85.62 | 82832 |
| PR001476 | 0.4706 | 277576 |
| Control antibody 1 | 1.175 | 316232 |

FIG. 7

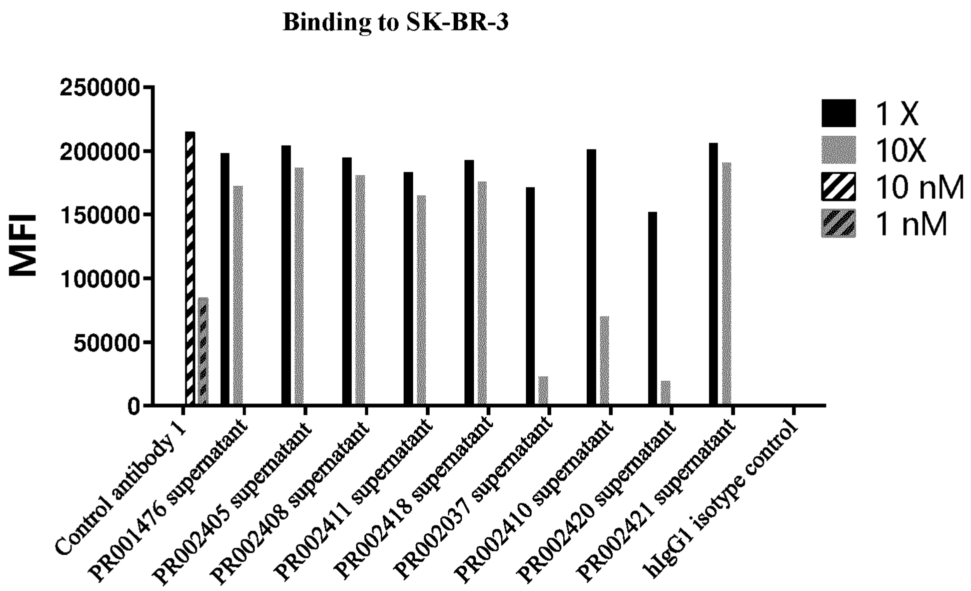


FIG. 8

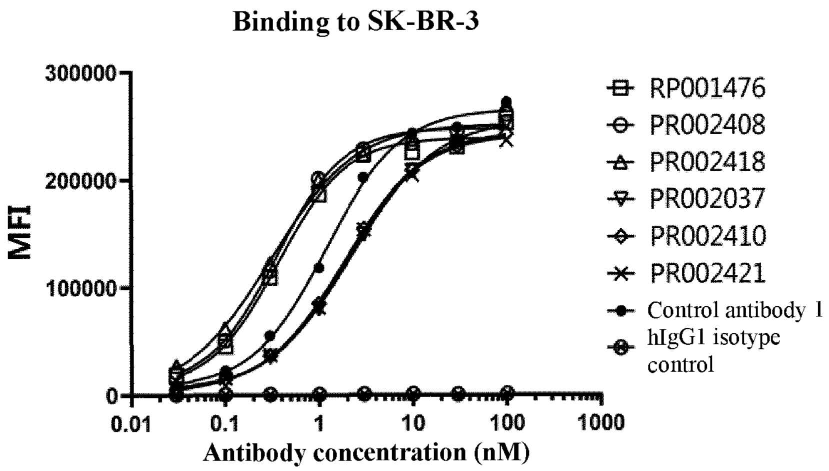


| Antibody | EC50 (nM) | Maximum MFI |
|---|---|---|
| RP001476 | 0.367 | 238962 |
| PR002408 | 0.3531 | 248060 |
| PR002418 | 0.3112 | 251781 |
| PR002037 | 2.22 | 256727 |
| PR002410 | 1.872 | 245482 |
| PR002421 | 1.92 | 242474 |
| Control antibody 1 | 1.199 | 265713 |

FIG. 9

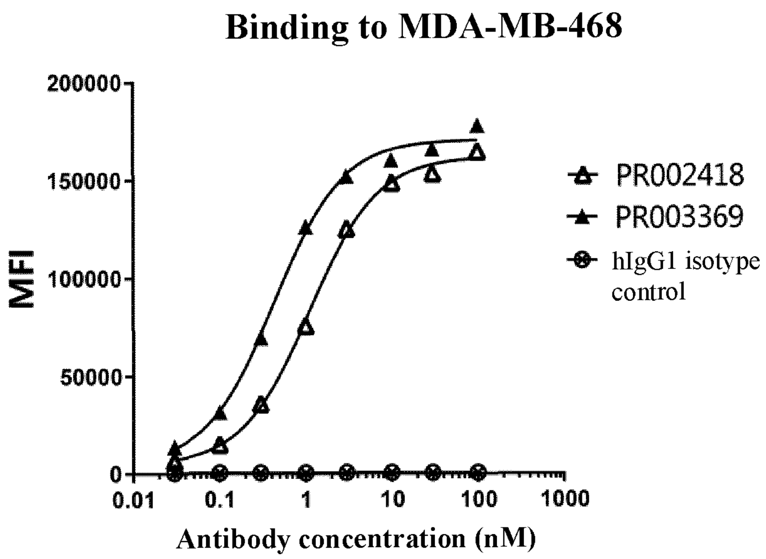


| Antibody | EC50 (nM) | Maximum MFI |
|---|---|---|
| PR002418 | 1.116 | 162162 |
| PR003369 | 0.4342 | 170835 |

FIG. 10

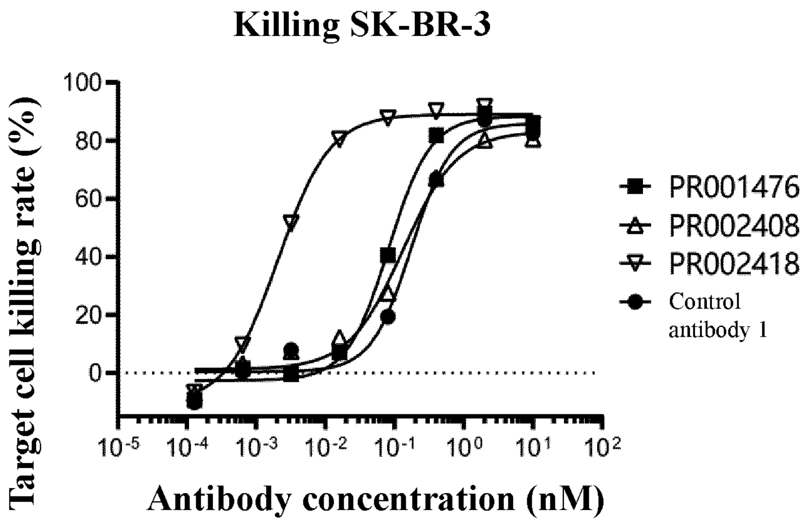


| Antibody | Maximum killing rate % | EC50 (nM) |
|---|---|---|
| PR002418 | 89.09 | 0.00209 |
| PR001476 | 88.41 | 0.08241 |
| PR002408 | 83.17 | 0.132 |
| Control antibody 1 | 85.92 | 0.1757 |

FIG. 11

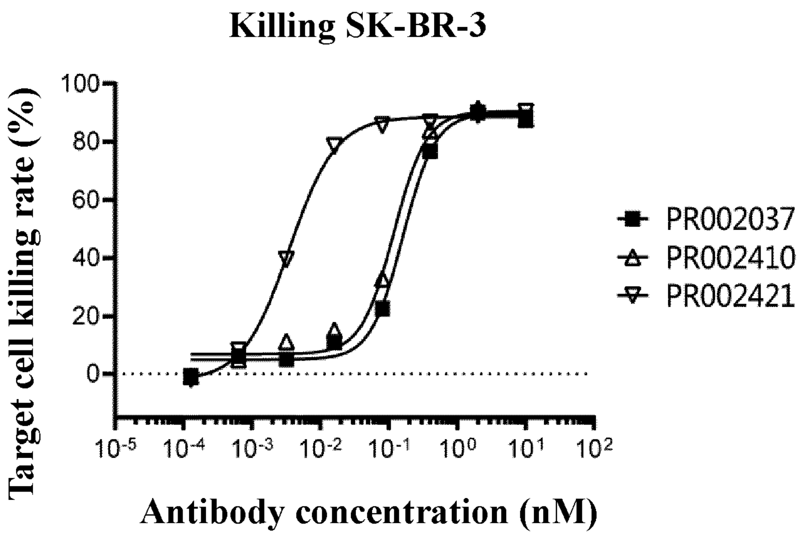


| Antibody | Maximum killing rate % | EC50 (nM) |
|---|---|---|
| PR002037 | 89.73 | 0.1607 |
| PR002410 | 90.41 | 0.1184 |
| PR002421 | 88.55 | 0.003519 |

FIG. 12

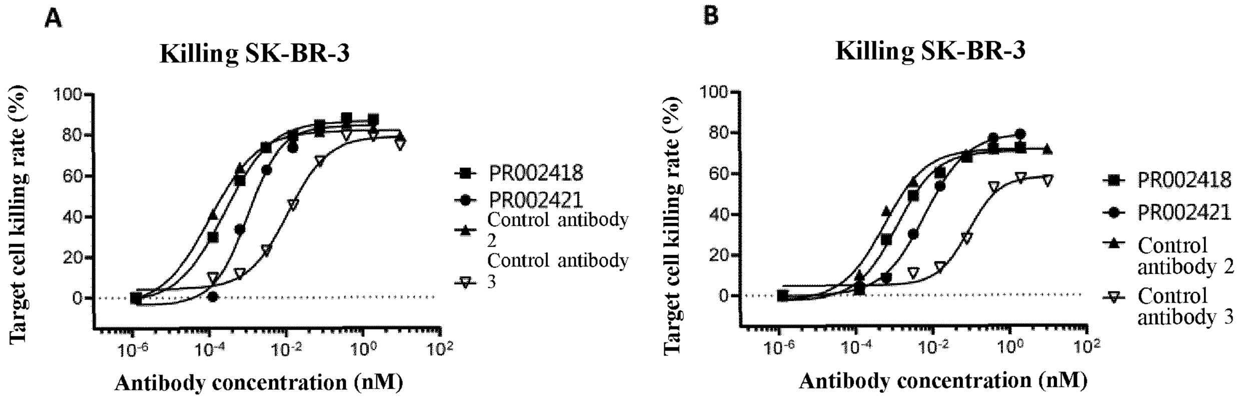


| | Donor 1 | | Donor 2 | |
|---|---|---|---|---|
| Antibody | Maximum killing rate % | EC50 (nM) | Maximum killing rate % | EC50 (nM) |
| PR002418 | 86.73 | 0.000267 | 70.83 | 0.001218 |
| PR002421 | 84.33 | 0.001045 | 79.24 | 0.006439 |
| Control antibody 2 | 82.01 | 0.000109 | 71.76 | 0.000576 |
| Control antibody 3 | 78.98 | 0.01159 | 58.27 | 0.08705 |

FIG. 13

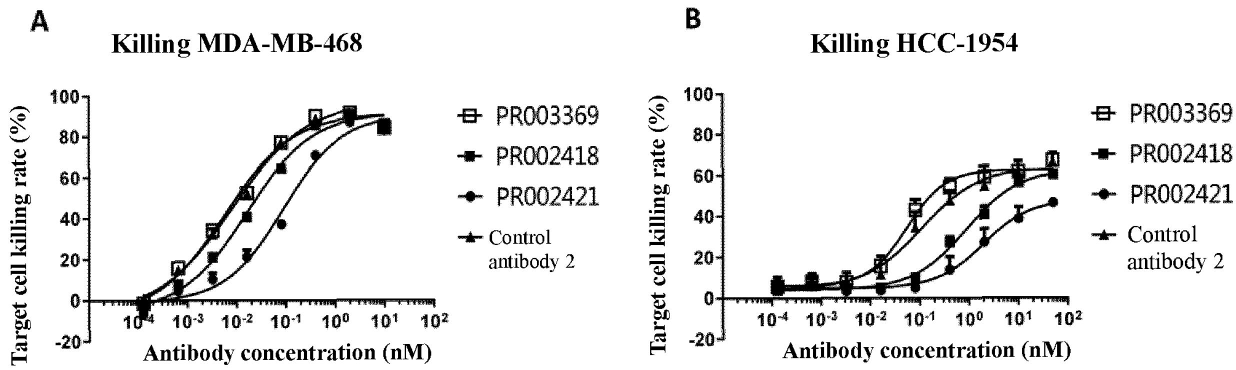


| | MDA-MB-468 | | HCC-1954 | |
|---|---|---|---|---|
| Antibody | Maximum killing rate % | EC50 (nM) | Maximum killing rate % | EC50 (nM) |
| PR003369 | 91.38 | 0.0058 | 62.72 | 0.054 |
| PR002418 | 91.67 | 0.0162 | 62.8 | 0.858 |
| PR002421 | 90.57 | 0.0876 | 48.05 | 1.944 |
| Control antibody 2 | 97.28 | 0.0082 | 63.78 | 0.107 |

FIG. 14

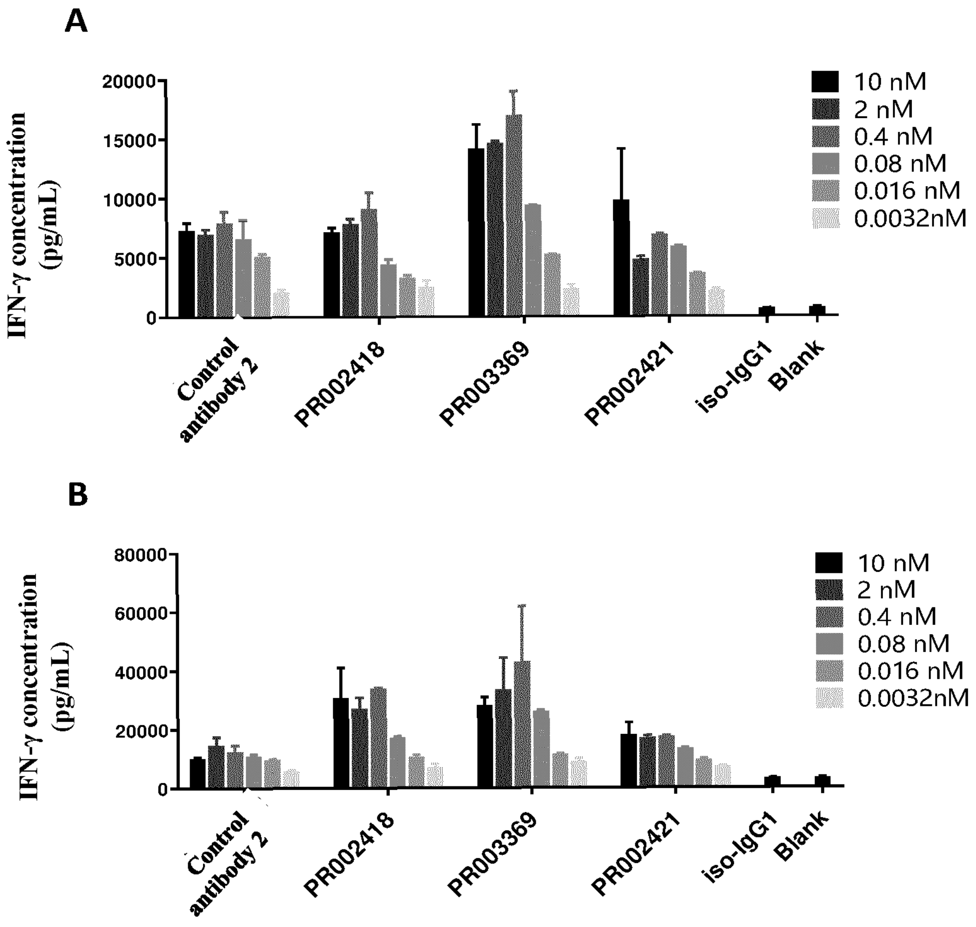


FIG. 15

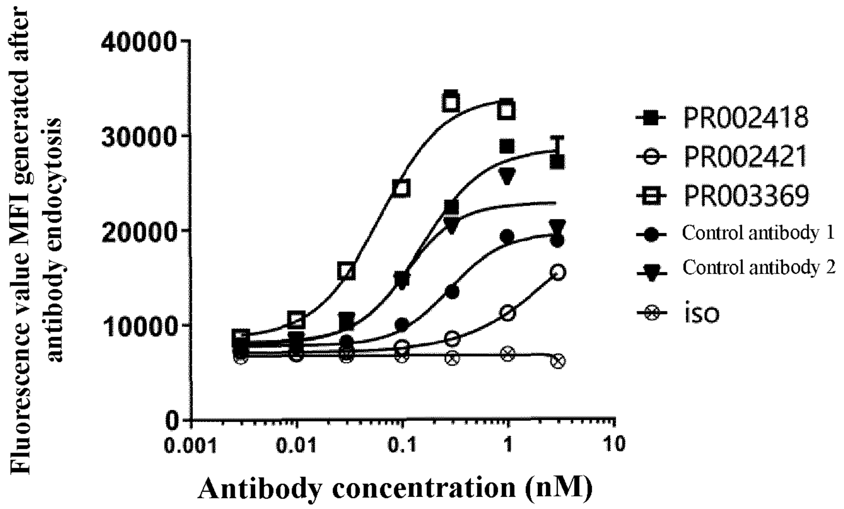


| Antibody | Maximum MFI | EC50 (nM) |
|---|---|---|
| PR002418 | 28455 | 0.1556 |
| PR002421 | 22289 | 2.518 |
| PR003369 | 34014 | 0.06262 |
| Control antibody 1 | 19578 | 0.2833 |
| Control antibody 2 | 22746 | 0.11 |

FIG. 16

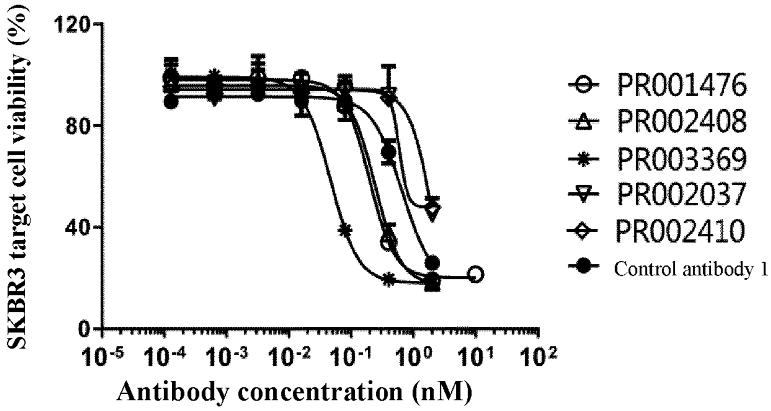


| Antibody | Minimum cell viability % | EC50 (nM) |
|---|---|---|
| PR001476 | 20.07 | 0.1957 |
| PR002408 | 17.3 | 0.2423 |
| PR003369 | 18.06 | 0.04668 |
| PR002037 | ~-20.36 | ~2.287 |
| PR002410 | ~47.86 | ~0.5919 |
| Control antibody 1 | 16.85 | 0.659 |

FIG. 17

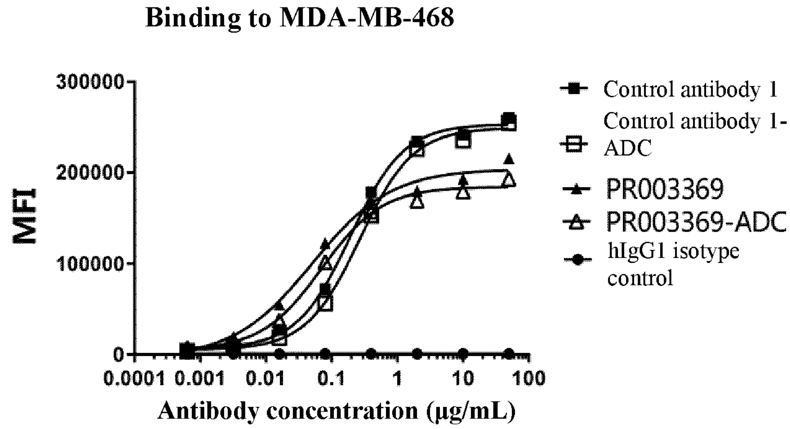


| Antibody | EC50 (nM) | Maximum MFI |
|---|---|---|
| Control antibody 1 | 253552 | 0.1941 |
| Control antibody 1-ADC | 249359 | 0.2697 |
| PR003369 | 204113 | 0.05077 |
| PR003369-ADC | 184504 | 0.07071 |

FIG. 18

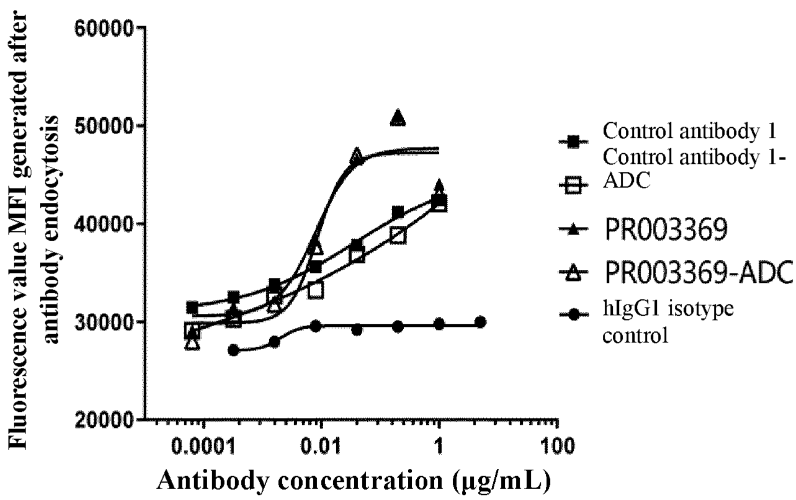


| Antibody | EC50 (nM) | Maximum MFI |
|---|---|---|
| Control antibody 1 | 45229 | 0.03666 |
| Control antibody 1-ADC | ~ | ~ |
| PR003369 | 47711 | 0.007967 |
| PR003369-ADC | 47236 | 0.008489 |

FIG. 19

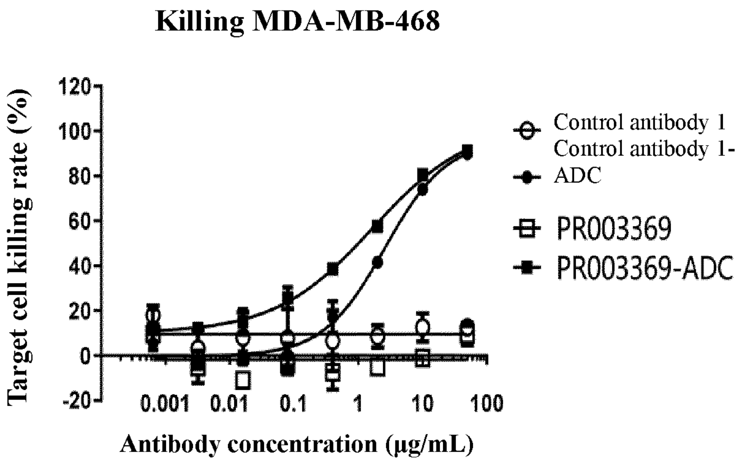


| Antibody | Maximum killing rate % | EC50 (nM) |
|---|---|---|
| Control antibody 1-ADC | 96.12 | 2.602 |
| PR003369-ADC | 103.3 | 1.671 |

FIG. 20

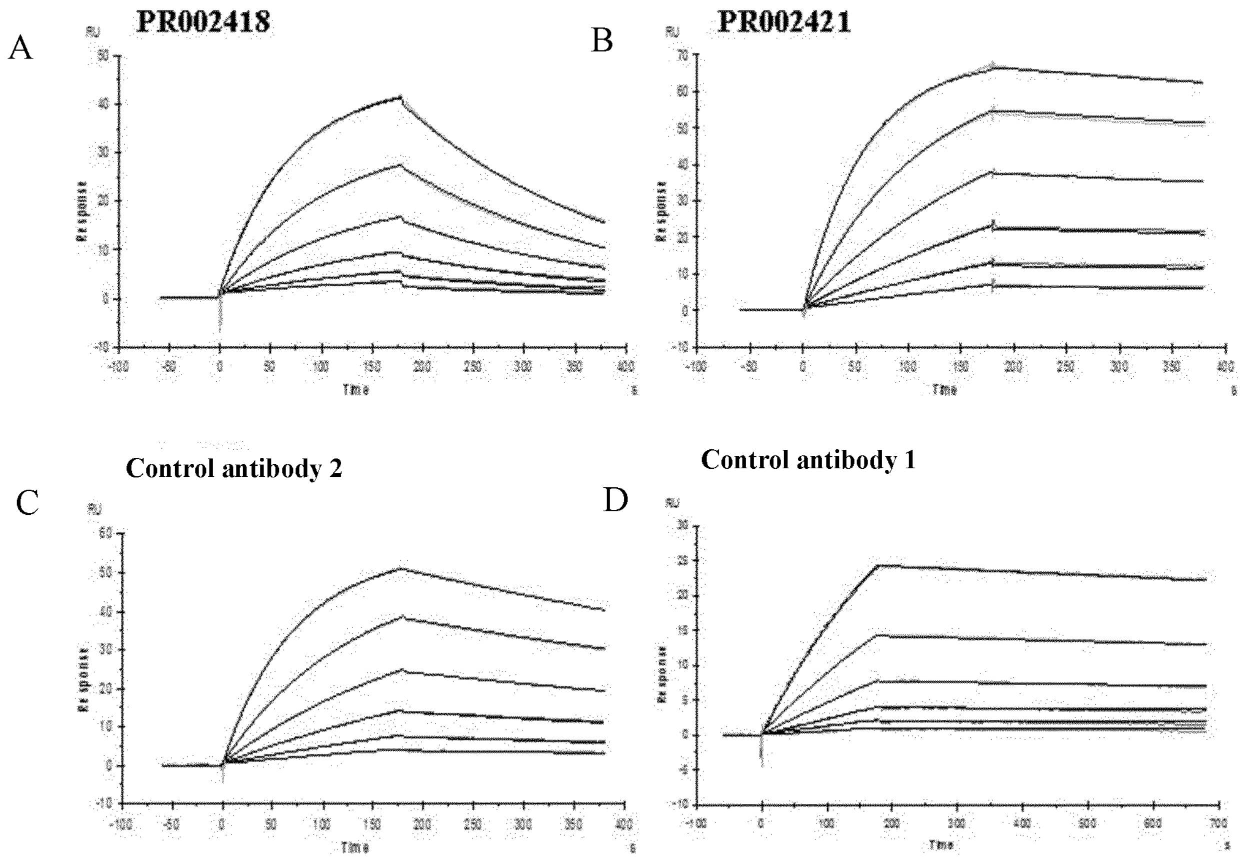


FIG. 21

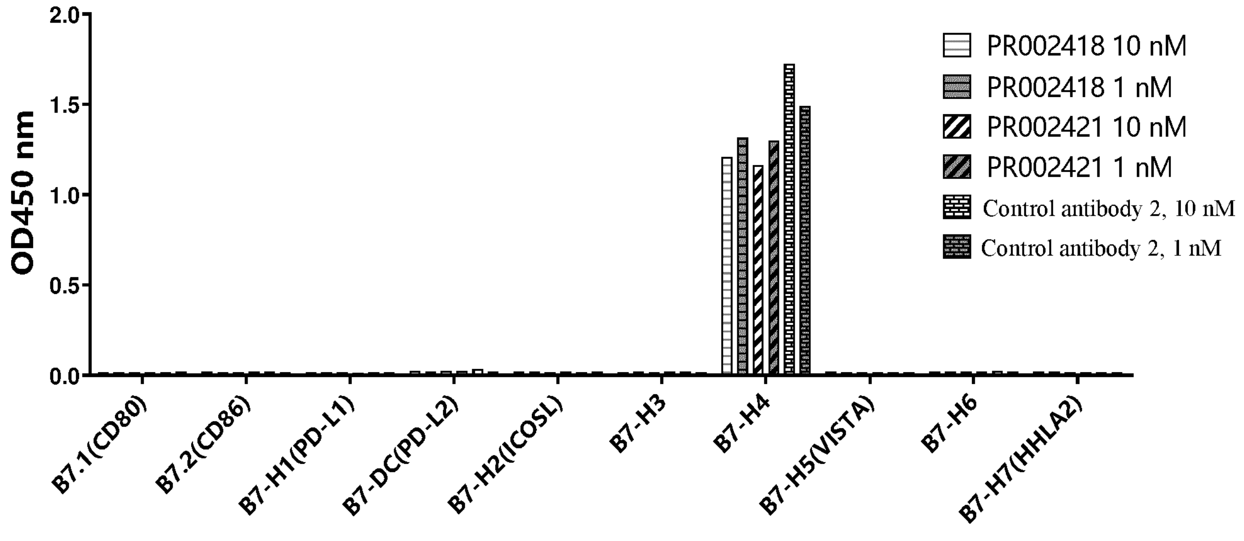
OD450 nm
2.0
1.5
1.0
0.5
0.0
PR002418 10 nM
PR002418 1 nM
PR002421 10 nM
PR002421 1 nM
Control antibody 2, 10 nM
Control antibody 2, 1 nM
B7.1(CD80)
B7.2(CD86)
B7-H1(PD-L1)
B7-DC(PD-L2)
B7-H2(ICOSL)
B7-H3
B7-H4
B7-H5(VISTA)
B7-H6
B7-H7(HHLA2)

FIG. 22

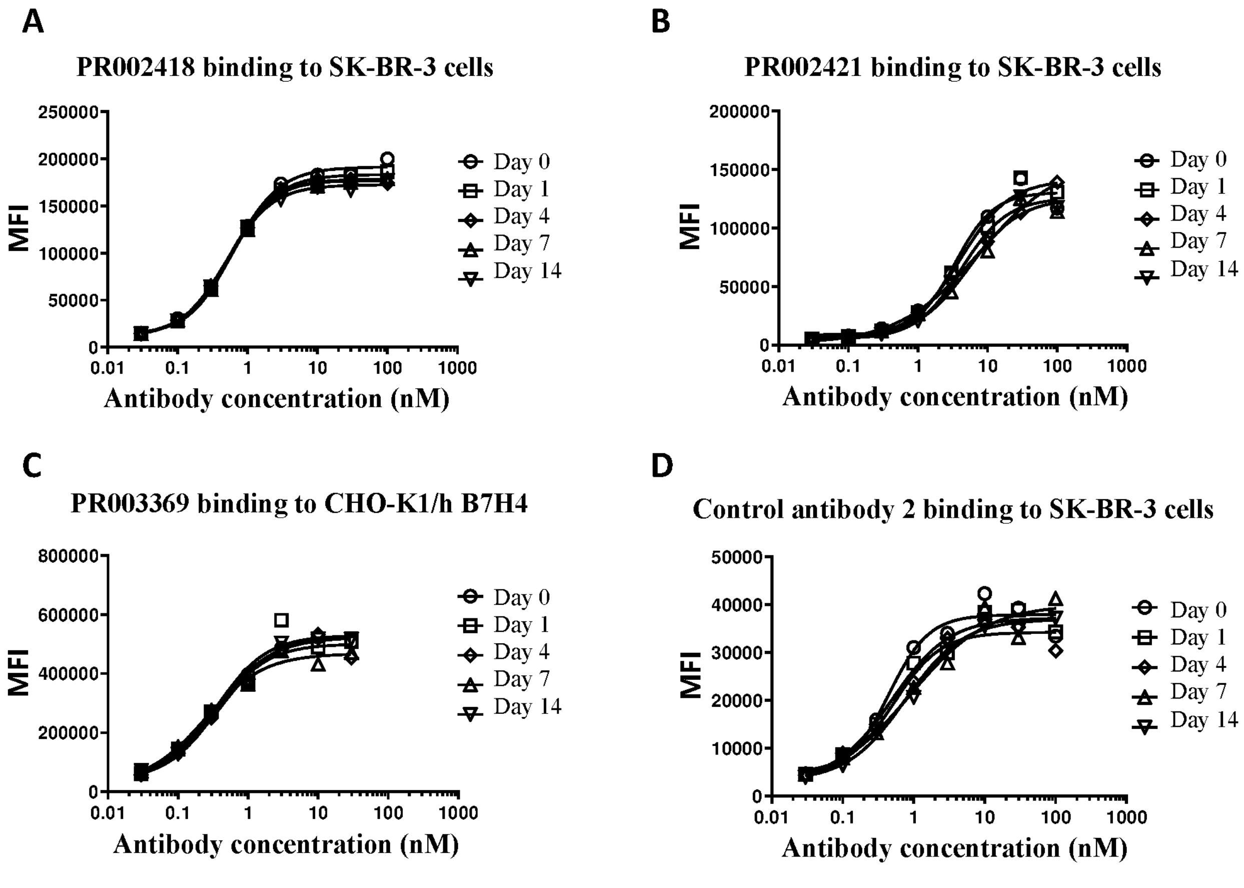
A
PR002418 binding to SK-BR-3 cells
MFI
Antibody concentration (nM)
B
PR002421 binding to SK-BR-3 cells
MFI
Antibody concentration (nM)
C
PR003369 binding to CHO-K1/h B7H4
MFI
Antibody concentration (nM)
D
Control antibody 2 binding to SK-BR-3 cells
MFI
Antibody concentration (nM)
Day 0
Day 1
Day 4
Day 7
Day 14

FIG. 23

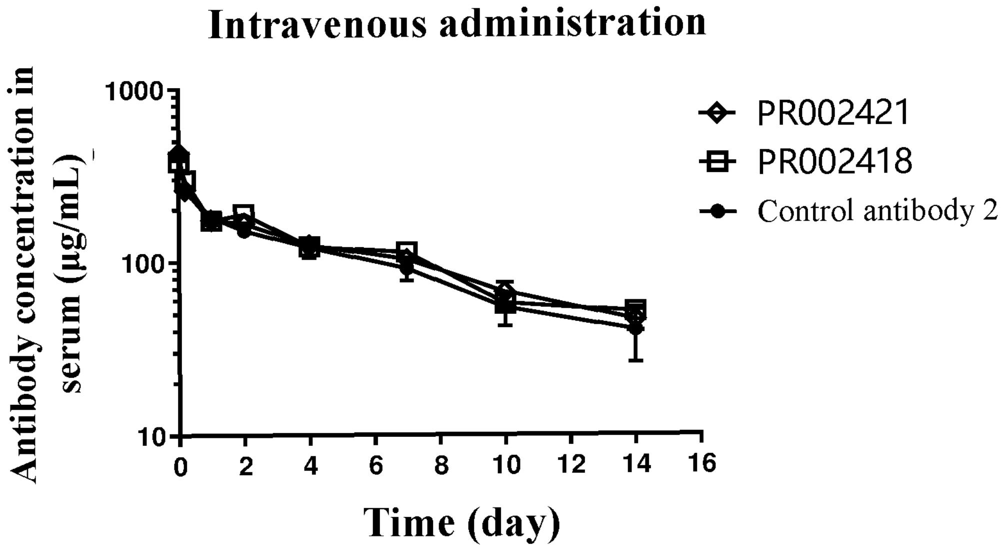
Intravenous administration
Antibody concentration in serum (µg/mL)
1000
100
10
0
2
4
6
8
10
12
14
16
Time (day)
PR002421
PR002418
Control antibody 2

FIG. 24

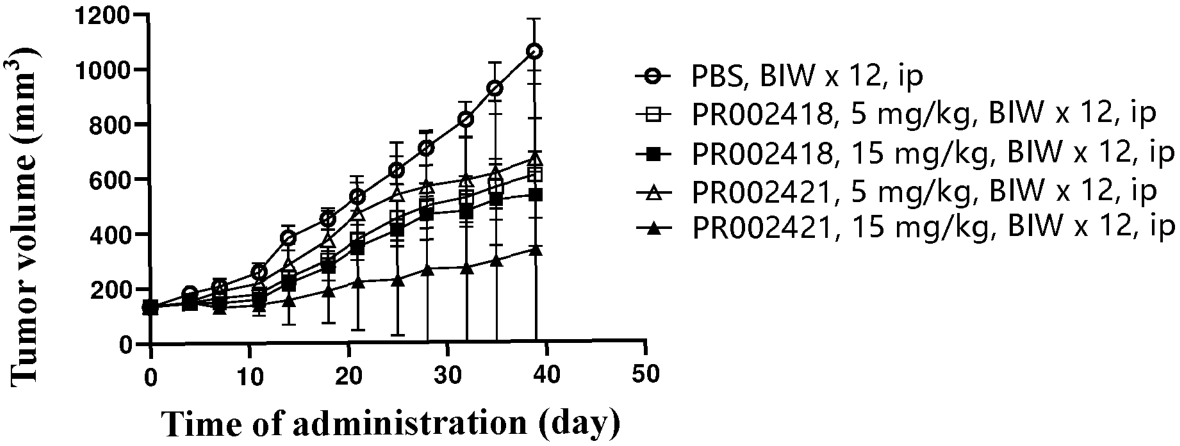
Tumor volume (mm3)
1200
1000
800
600
400
200
0
0
10
20
30
40
50
Time of administration (day)
PBS, BIW x 12, ip
PR002418, 5 mg/kg, BIW x 12, ip
PR002418, 15 mg/kg, BIW x 12, ip
PR002421, 5 mg/kg, BIW x 12, ip
PR002421, 15 mg/kg, BIW x 12, ip

FIG. 25

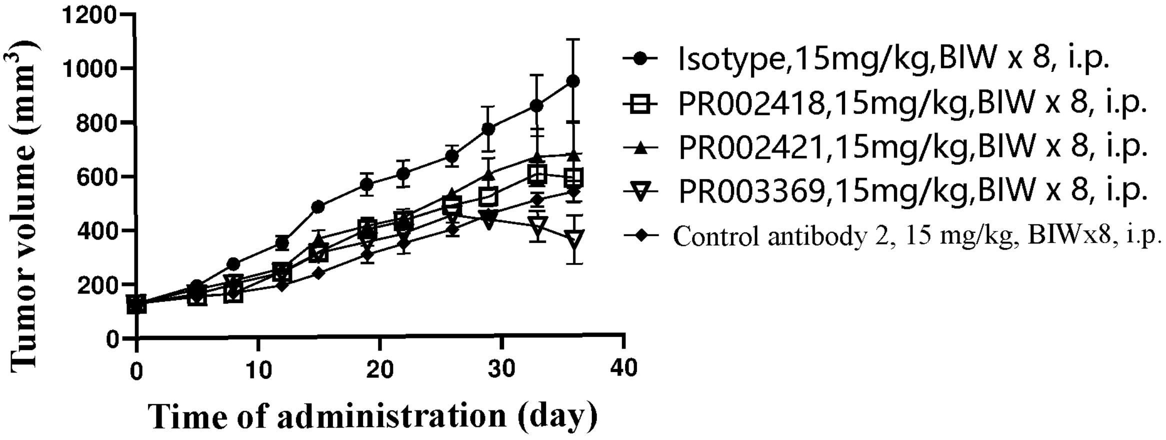


FIG. 26

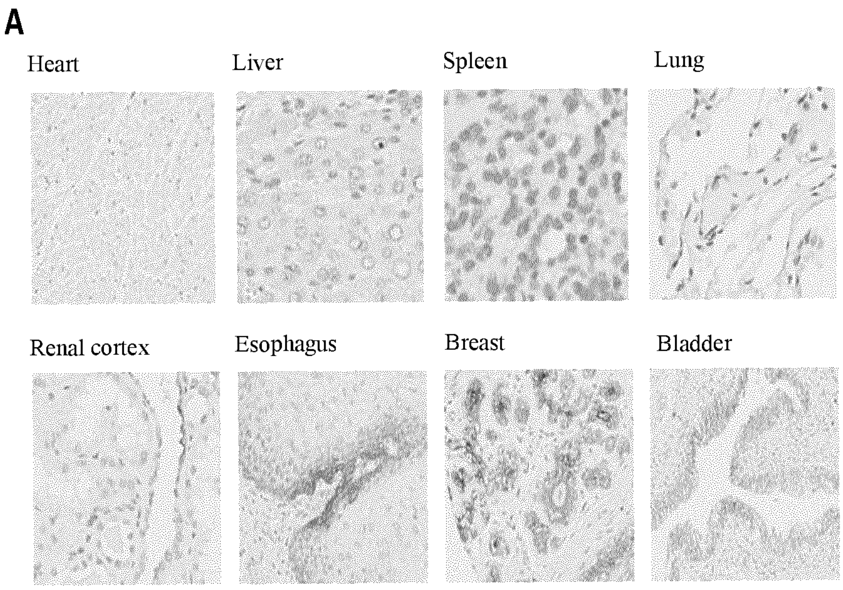


| | | | |
|---|---|---|---|
| Adrenal gland | Mild | Liver | - |
| Bladder | Mild | Lung | - |
| Bone marrow | - | Ovary | - |
| Salivary gland | - | Pancreas | - |
| Eye | - | Parathyroid | - |
| Breast | Mild | Pituitary | - |
| Cerebellum | - | Placenta | - |
| Cerebral cortex | - | Prostate gland | - |
| Fallopian tube | Mild | Skin | - |
| Esophagus | Mild | Spinal cord | - |
| Stomach | - | Spleen | - |
| Small intestine | - | Striated muscle | - |
| Colon | - | Testicle | |
| Rectum | - | Thymus | - |
| Heart | - | Thyroid | - |
| Renal cortex | Mild | Tonsil | - |
| Renal medulla | - | Cervix uteri | - |
| Peripheral nerve | - | Endometrium | Mild |
| Ureter | Mild | | |

(continued on next figure)

(continued from previous figure)

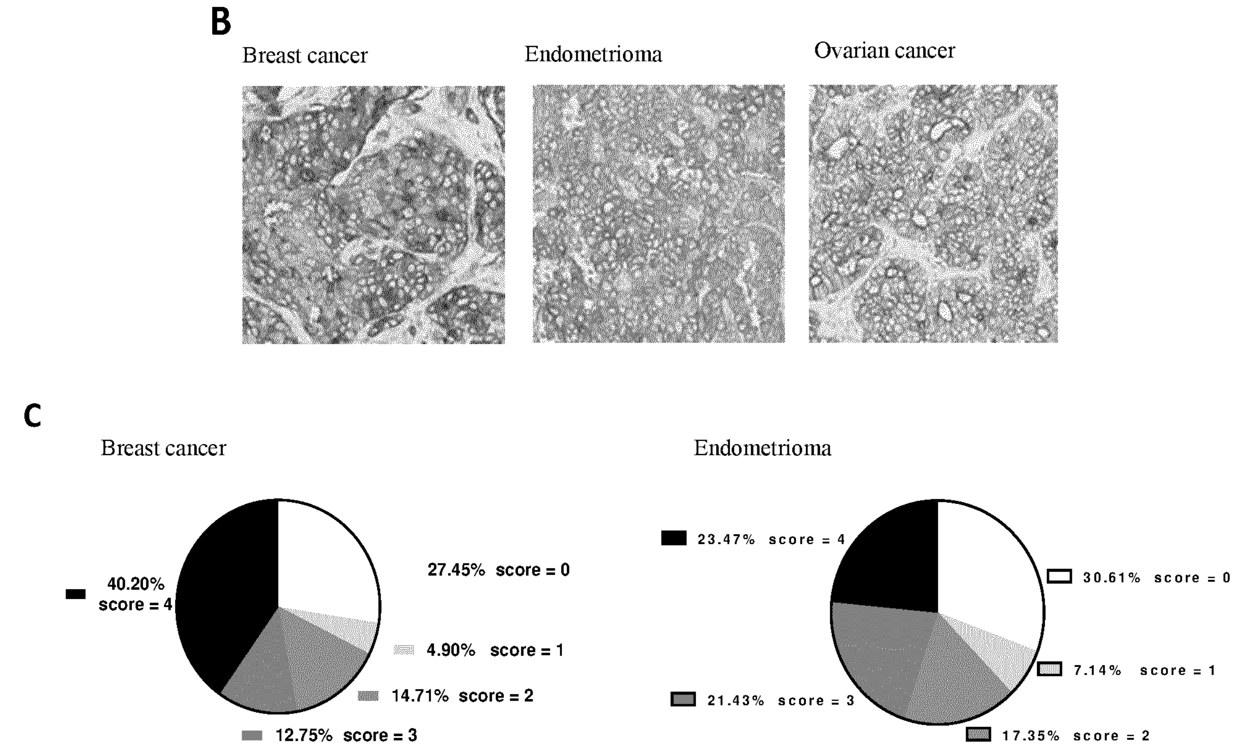


FIG. 27

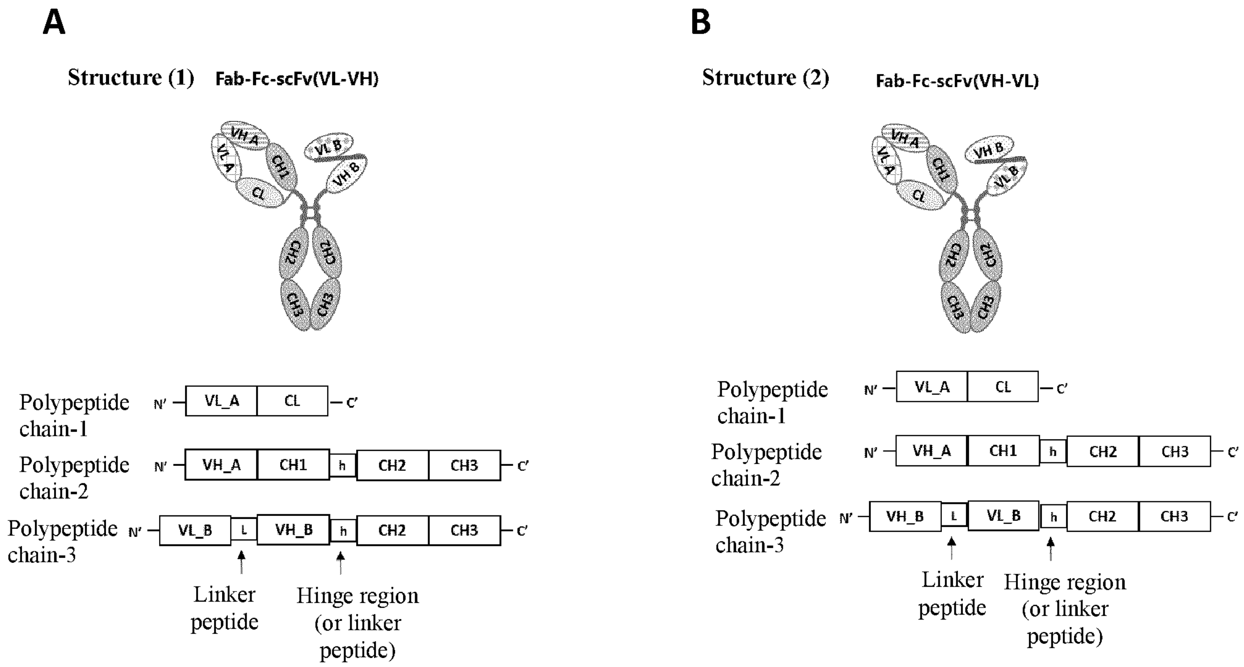


(continued on next figure)

(continued from previous figure)

C

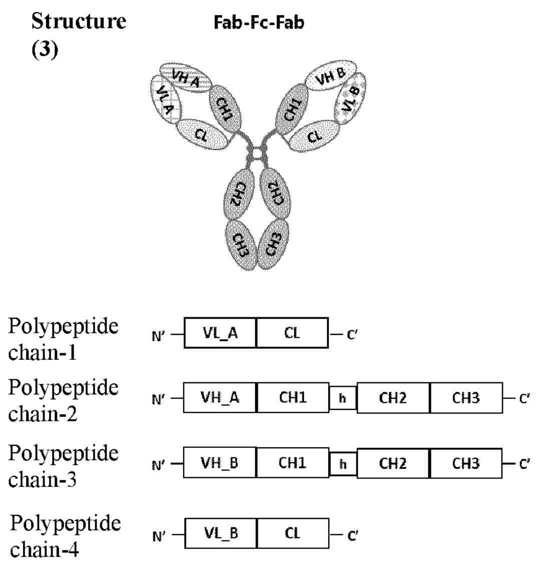


D

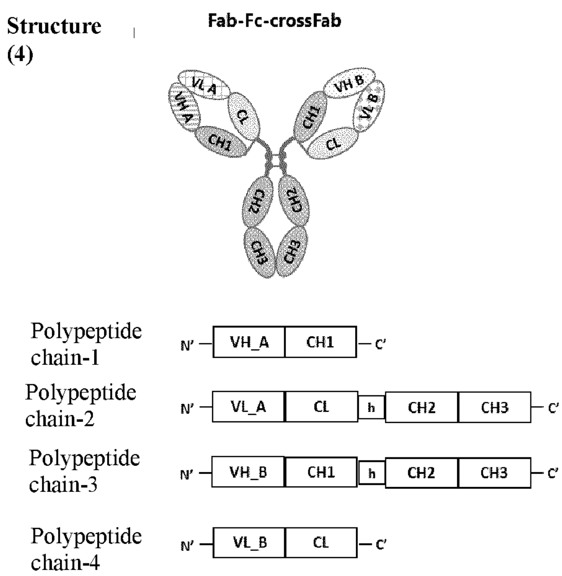


E

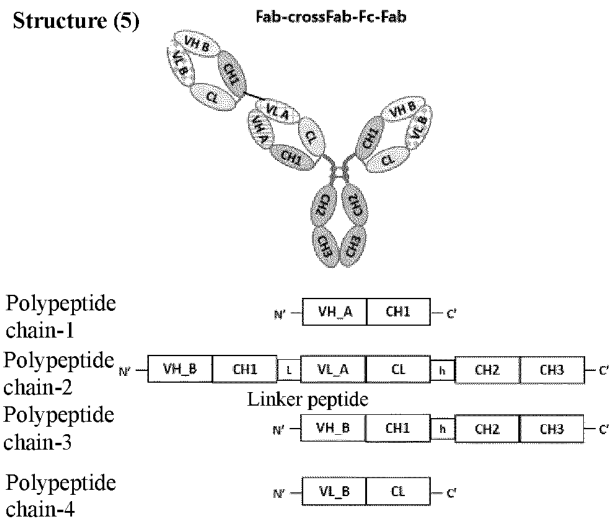


F

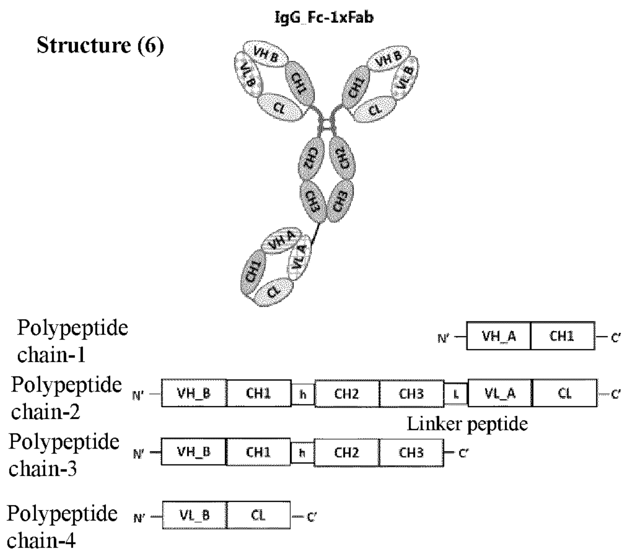


(continued on next figure)

(continued from previous figure)

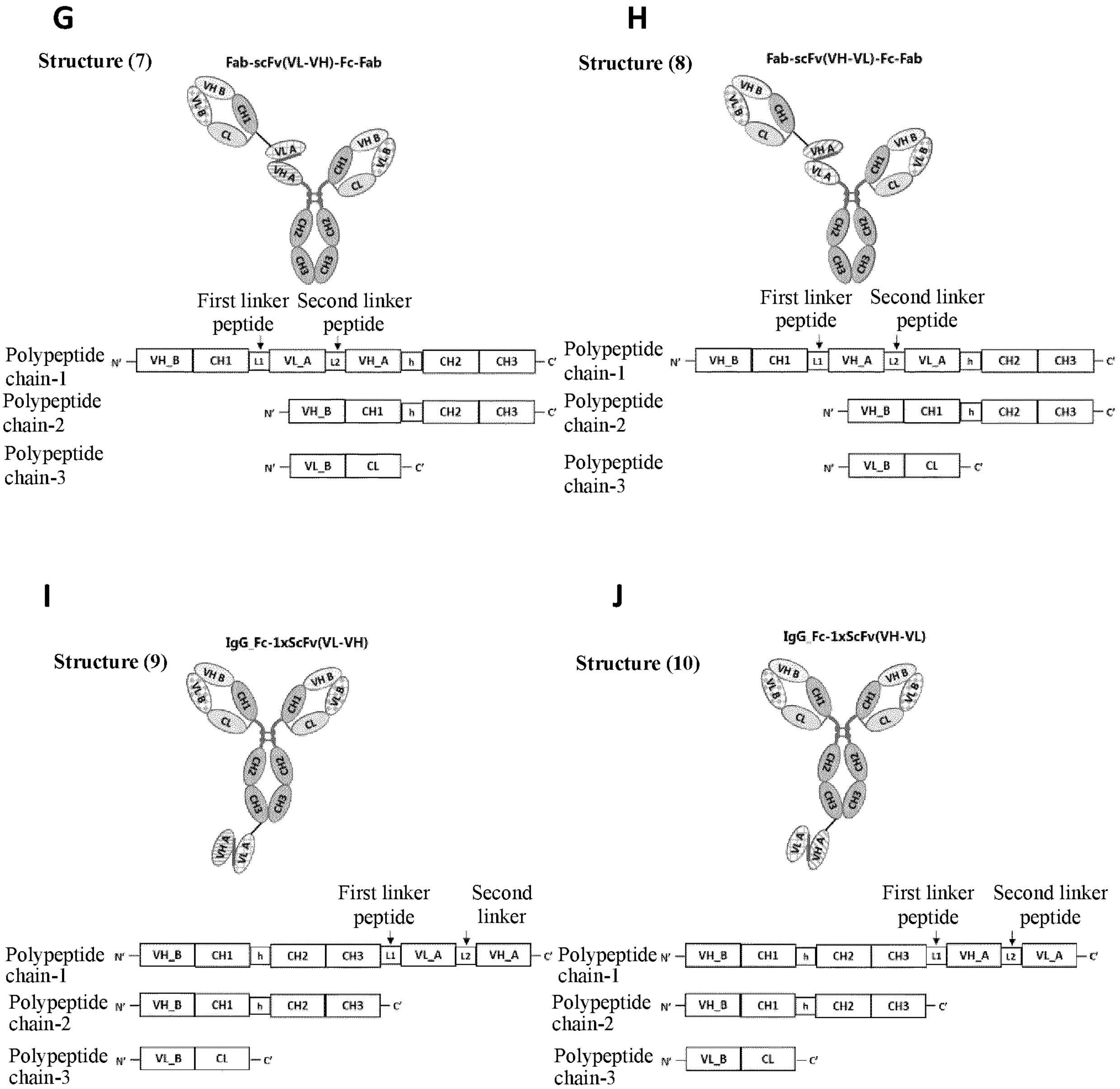


FIG. 28

A

Binding to SK-BR-3

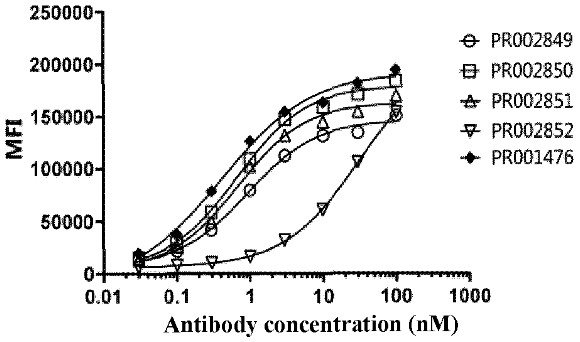


| Antibody | EC50 (nM) | Maximum MFI |
|---|---|---|
| PR002849 | 0.933 | 146289 |
| PR002850 | 0.676 | 179159 |
| PR002851 | 0.694 | 162972 |
| PR002852 | 32.230 | 153882 |
| PR001476 (αB7H4 IgG) | 0.428 | 191590 |

B

Binding to SK-BR-3

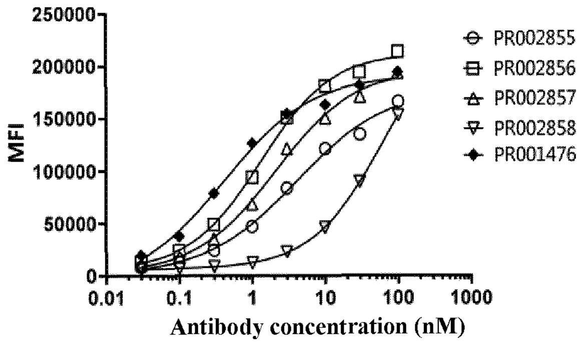


| Antibody | EC50 (nM) | Maximum MFI |
|---|---|---|
| PR002855 | 4.050 | 178586 |
| PR002856 | 1.317 | 212320 |
| PR002857 | 2.095 | 197029 |
| PR002858 | 67.820 | 153055 |
| PR001476 (αB7H4 IgG) | 0.428 | 191590 |

C

Binding to SK-BR-3

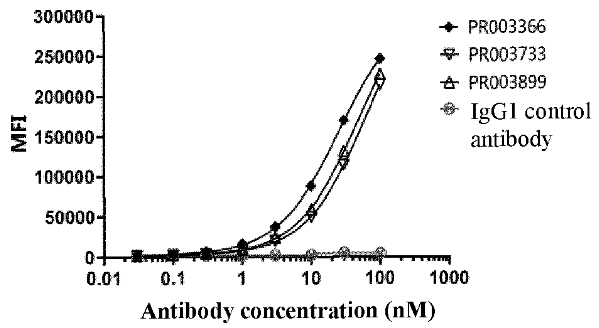


| Antibody | EC50 (nM) | Maximum MFI |
|---|---|---|
| PR003733 | 71.15 | 214270 |
| PR003899 | 51.20 | 227375 |
| PR003366 (αB7H4 scFv-Fc) | 26.21 | 246319 |

D

Binding to SK-BR-3

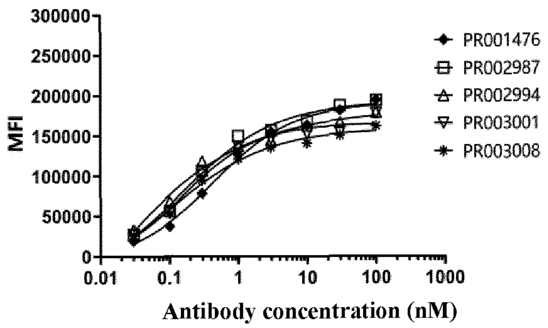


| Antibody | EC50 (nM) | Maximum MFI |
|---|---|---|
| PR002987 | 0.145 | 192702 |
| PR002994 | 0.006 | 183041 |
| PR003001 | 0.162 | 165503 |
| PR003008 | 0.108 | 158980 |
| PR001476 | 0.428 | 191590 |

(continued on next figure)

(continued from previous figure)

E

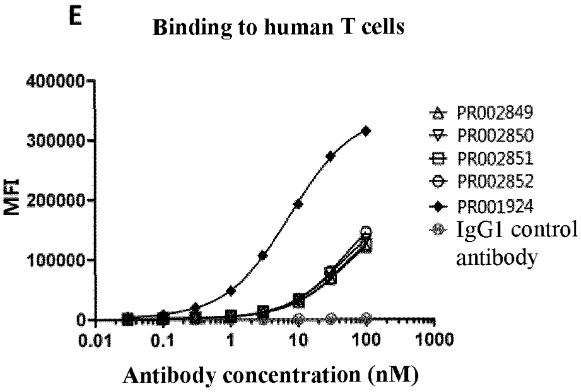


| Antibody | EC50 (nM) | Maximum MFI |
|---|---|---|
| PR002849 | n.d. | 121205 |
| PR002850 | n.d. | 132538 |
| PR002851 | n.d. | 124665 |
| PR002852 | n.d. | 145036 |
| PR001924 (αCD3 IgG) | 7.498 | 314185 |

F

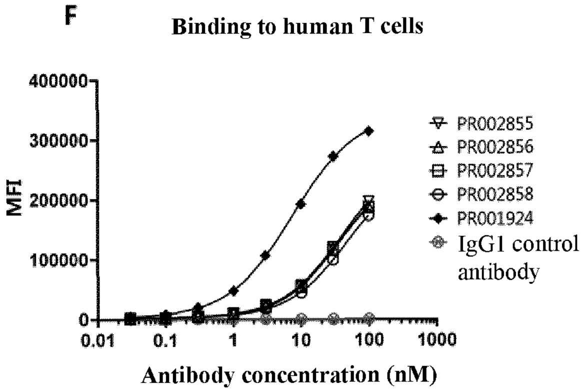


| Antibody | EC50 (nM) | Maximum MFI |
|---|---|---|
| PR002855 | n.d. | 196778 |
| PR002856 | n.d. | 188570 |
| PR002857 | n.d. | 186998 |
| PR002858 | n.d. | 173711 |
| PR001924 (αCD3 IgG) | 7.498 | 314185 |

G

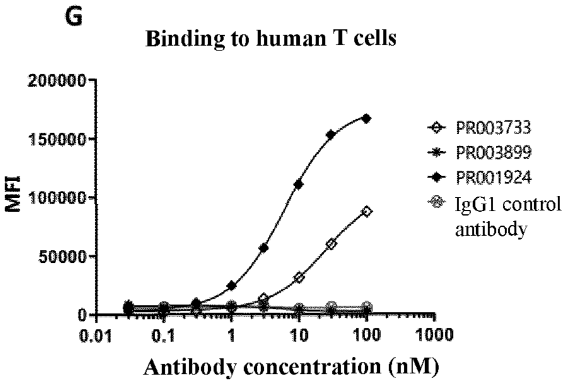


| Antibody | EC50 (nM) | Maximum MFI |
|---|---|---|
| PR003733 | n.d. | 87142 |
| PR003899 | n.d. | 2497 |
| PR001924 (αCD3 IgG) | 6.101 | 166289 |

H

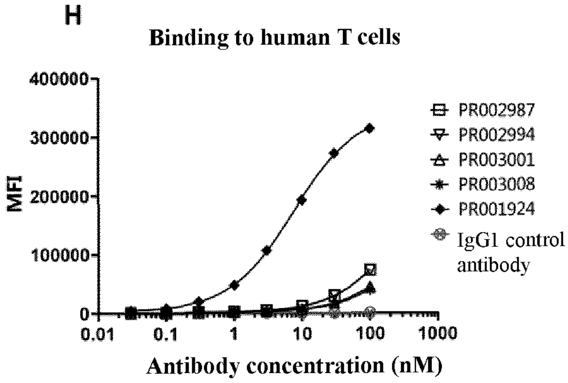


| Antibody | EC50 (nM) | Maximum MFI |
|---|---|---|
| PR002987 | n.d. | 73615 |
| PR002994 | n.d. | 73024 |
| PR003001 | n.d. | 44362 |
| PR003008 | n.d. | 39387 |
| PR001924 (αCD3 IgG) | 7.498 | 314185 |

FIG. 29

A

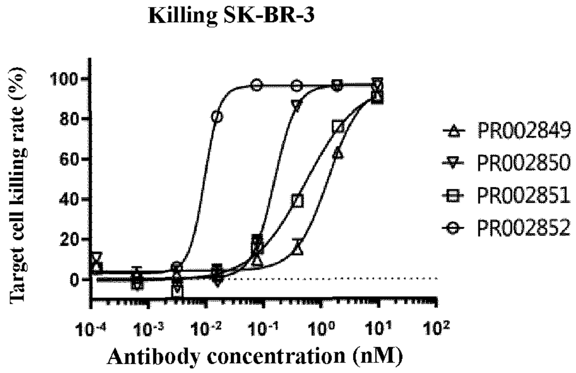


| Antibody | Maximum killing rate % | EC50 (nM) |
|---|---|---|
| PR002849 | 95.2 | 1.367 |
| PR002850 | 96.6 | 0.1585 |
| PR002851 | 96.5 | 0.5481 |
| PR002852 | 96.3 | 0.009729 |

B

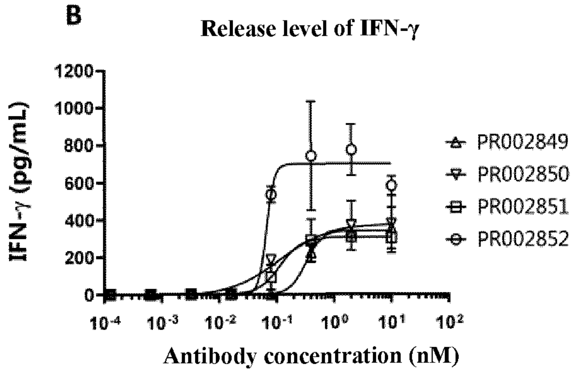


| Antibody | Maximum IFN-γ release value (pg/mL) | EC50 (nM) |
|---|---|---|
| PR002849 | 340.8 | 0.3233 |
| PR002850 | 377 | 0.1116 |
| PR002851 | 305.4 | 0.114 |
| PR002852 | 698.5 | 0.06667 |

C

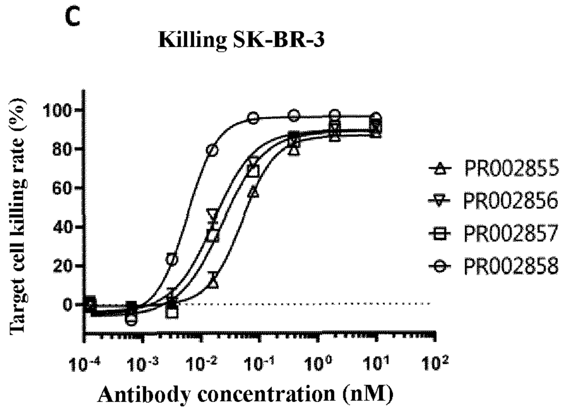


| Antibody | Maximum killing rate % | EC50 (nM) |
|---|---|---|
| PR002855 | 86.4 | 0.05101 |
| PR002856 | 89.1 | 0.01681 |
| PR002857 | 88.9 | 0.02327 |
| PR002858 | 96.3 | 0.00595 |

D

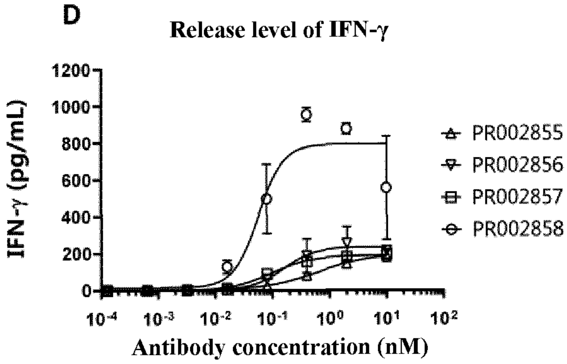


| Antibody | Maximum IFN-γ release value (pg/mL) | EC50 (nM) |
|---|---|---|
| PR002855 | 200 | 0.6329 |
| PR002856 | 234.6 | 0.1549 |
| PR002857 | 190.2 | 0.09531 |
| PR002858 | 794.7 | 0.05708 |

(continued on next figure)

(continued from previous figure)

E

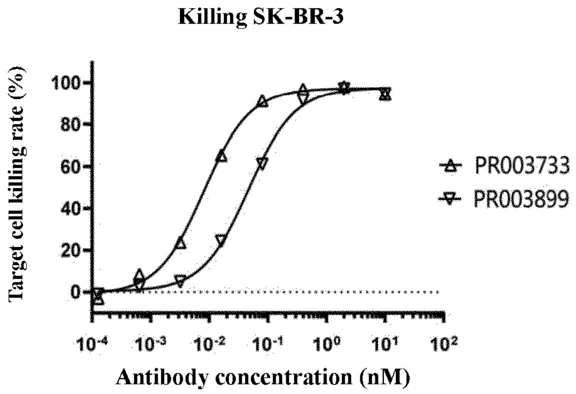


| Antibody | Maximum killing rate % | EC50 (nM) |
|---|---|---|
| PR003733 | 97.4 | 0.008086 |
| PR003899 | 97.5 | 0.04672 |

F

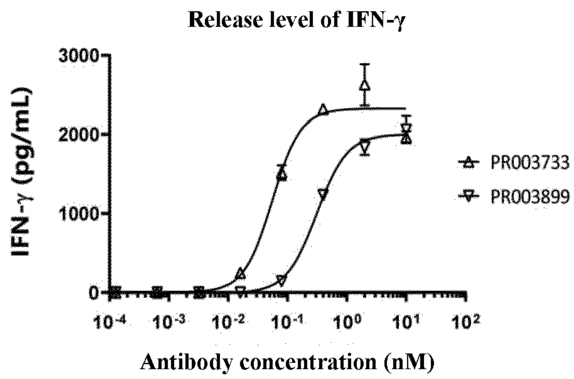


| Antibody | Maximum IFN-γ release value (pg/mL) | EC50 (nM) |
|---|---|---|
| PR003733 | 2320 | 0.05537 |
| PR003899 | 2002 | 0.3108 |

G

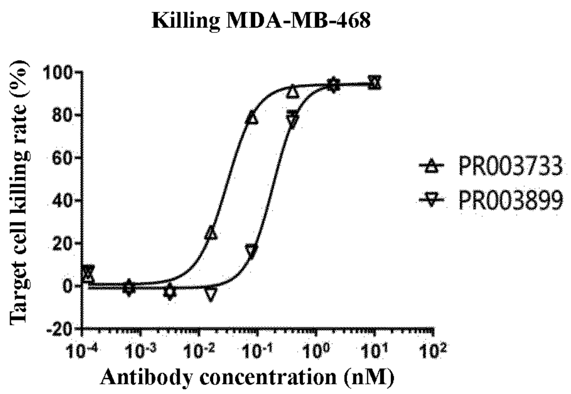


| Antibody | Maximum killing rate % | EC50 (nM) |
|---|---|---|
| PR003733 | 94.25 | 0.03007 |
| PR003899 | 94.75 | 0.1853 |

H

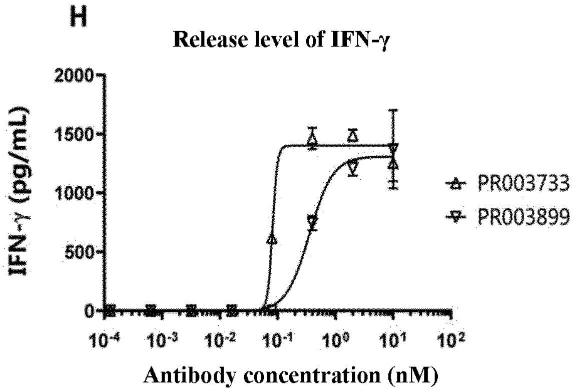


| Antibody | Maximum IFN-γ release value (pg/mL) | EC50 (nM) |
|---|---|---|
| PR003733 | 1401 | 0.08184 |
| PR003899 | 1309 | 0.3565 |

(continued on next figure)

(continued from previous figure)

I

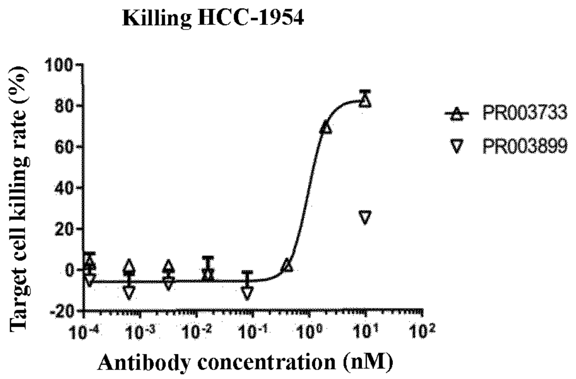


| Antibody | Maximum killing rate % | EC50 (nM) |
|---|---|---|
| PR003733 | 82.09 | 0.9994 |
| PR003899 | ~ | ~ |

J

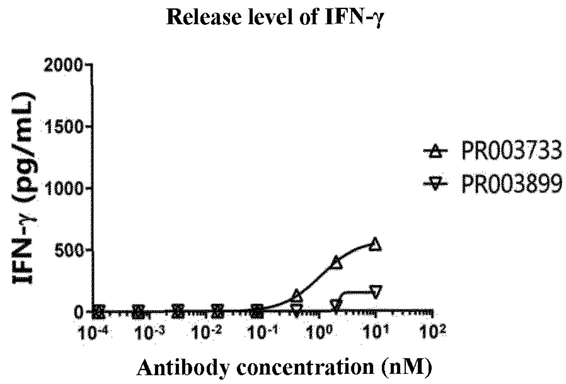


| Antibody | Maximum IFN-γ release value (pg/mL) | EC50 (nM) |
|---|---|---|
| PR003733 | 569.9 | 1.044 |
| PR003899 | ~ | ~ |

K

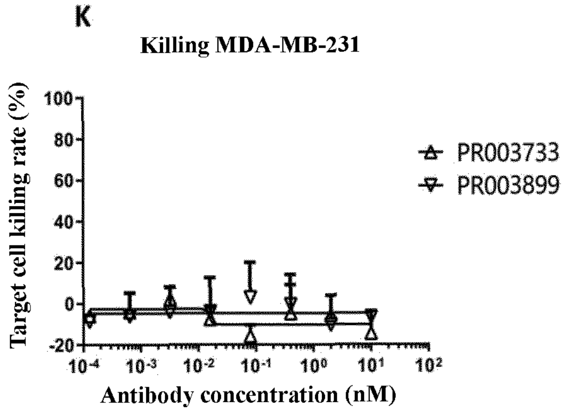


| Antibody | Maximum killing rate % | EC50 (nM) |
|---|---|---|
| PR003733 | ~ | ~ |
| PR003899 | ~ | ~ |

L

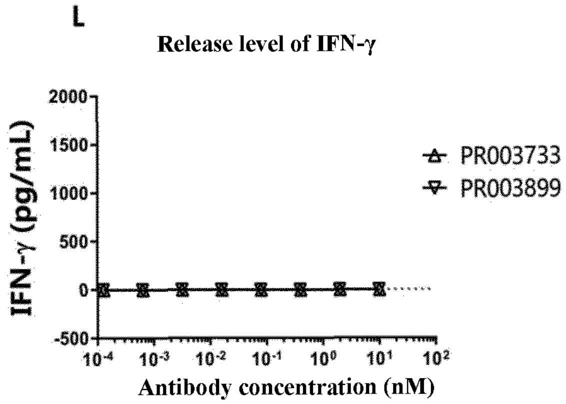


| Antibody | Maximum IFN-γ release value (pg/mL) | EC50 (nM) |
|---|---|---|
| PR003733 | ~ | ~ |
| PR003899 | ~ | ~ |

(continued on next figure)

(continued from previous figure)

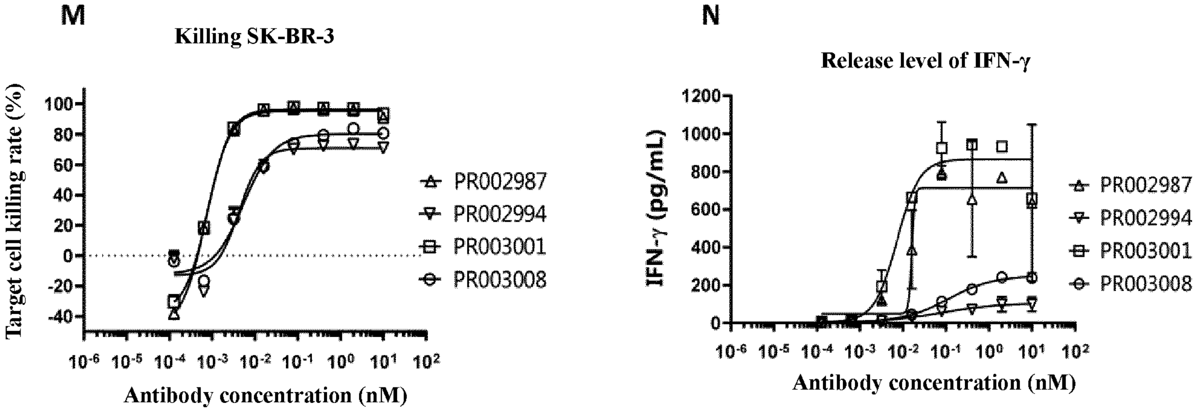


| Antibody | Maximum killing rate % | EC50 (nM) |
|---|---|---|
| PR002987 | 95.4 | 0.0007185 |
| PR002994 | 70.9 | 0.004208 |
| PR003001 | 96.5 | 0.0007887 |
| PR003008 | 80.3 | 0.00538 |

| Antibody | Maximum IFN-γ release value (pg/mL) | EC50 (nM) |
|---|---|---|
| PR002987 | 713.1 | ~0.01595 |
| PR002994 | 105.9 | 0.07633 |
| PR003001 | 864.7 | 0.007257 |
| PR003008 | 250.3 | 0.113 |

FIG. 30

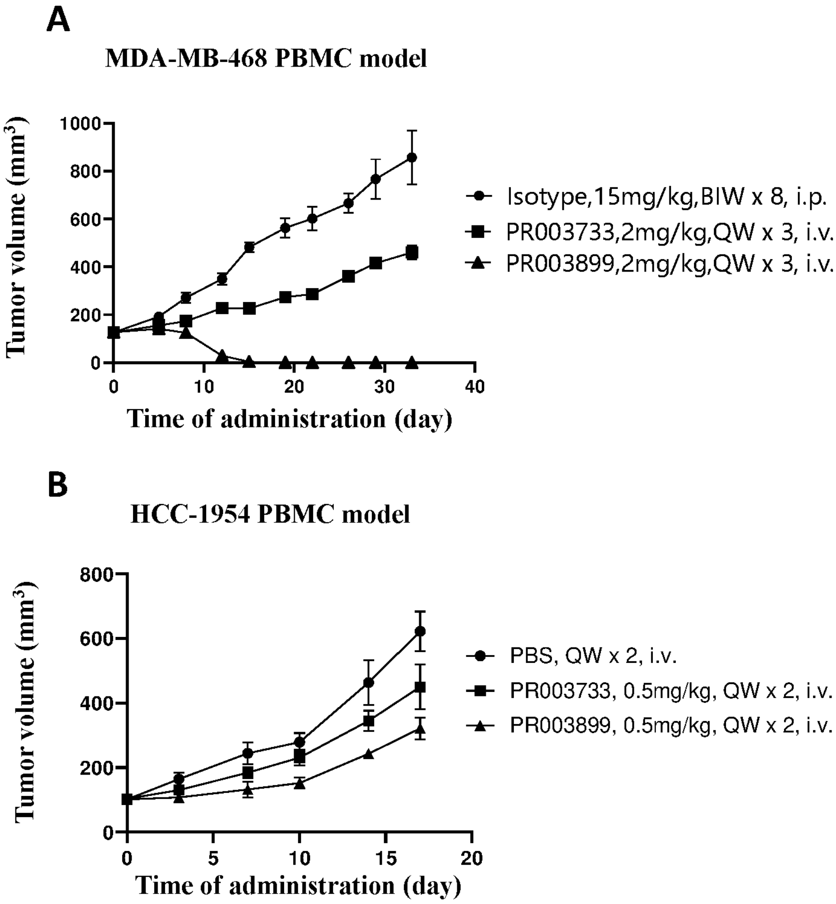


FIG. 31

## INTERNATIONAL SEARCH REPORT

International application No.
**PCT/CN2021/102952**

**A. CLASSIFICATION OF SUBJECT MATTER**

C07K 16/28(2006.01)i; A61K 39/395(2006.01)i; A61P 35/00(2006.01)i

According to International Patent Classification (IPC) or to both national classification and IPC

**B. FIELDS SEARCHED**

Minimum documentation searched (classification system followed by classification symbols)

C07K, A61K, A61P

Documentation searched other than minimum documentation to the extent that such documents are included in the fields searched

Electronic data base consulted during the international search (name of data base and, where practicable, search terms used)

VEN, CNABS, CNPTXT, EPTXT, USTXT, WOTXT, ISI Web of Knowledge, CNKI, NCBI GENBANK: 和铂医药, 抗体, 单克隆抗体, 双特异抗体, 小鼠, 肿瘤, B7H4, CD3, antibody, Bispecific, mouse, tumor, cancer

**C. DOCUMENTS CONSIDERED TO BE RELEVANT**

| Category* | Citation of document, with indication, where appropriate, of the relevant passages | Relevant to claim No. |
|---|---|---|
| A | WO 2016070001 A1 (JOUNCE THERAPEUTICS, INC.) 06 May 2016 (2016-05-06) entire document | 1-22 |
| A | IIZUKA, A. et al. "A T-cell–engaging B7-H4/CD3-bispecific Fab-scFv Antibody Targets Human Breast Cancer" *Clinical Cancer Research,* Vol. 25, No. 9, 08 February 2019 (2019-02-08), 2925-2934 | 1-22 |
| A | CN 110551221 A (GUANGZHOU EXCELMAB BIOMEDICAL TECHNOLOGY CO., LTD.) 10 December 2019 (2019-12-10) entire document | 1-22 |
| A | CN 108148137 A (THE FOURTH MILITARY MEDICAL UNIVERSITY OF PLA.) 12 June 2018 (2018-06-12) entire document | 1-22 |
| A | CN 102219856 A (HARBIN MEDICAL UNIVERSITY) 19 October 2011 (2011-10-19) entire document | 1-22 |
| A | WO 2009073533 A2 (MEDAREX, INC.) 11 June 2009 (2009-06-11) entire document | 1-22 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **13 September 2021** | **28 September 2021** |
| Name and mailing address of the ISA/CN<br>**China National Intellectual Property Administration (ISA/CN)<br>No. 6, Xitucheng Road, Jimenqiao, Haidian District, Beijing 100088<br>China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/102952**

**C. DOCUMENTS CONSIDERED TO BE RELEVANT**

| Category* | Citation of document, with indication, where appropriate, of the relevant passages | Relevant to claim No. |
|---|---|---|
| A | WO 2019147670 A1 (NEXTCURE, INC.) 01 August 2019 (2019-08-01)<br>entire document | 1-22 |
| A | 房鹏 等 (FANG, Peng et al.). "鼠抗人B7-H4单克隆抗体的制备及初步鉴定 (Preparation and Primary Identification of Mouse Anti-Human B7-H4 Monoclonal Antibody)"<br>*江苏医药 (Jiangsu Medical Journal),* Vol. 38, No. 12, 30 June 2012 (2012-06-30),<br>1386-1388 | 1-22 |
| A | DANGAJ, D. et al. "Novel Recombinant Human B7-H4 Antibodies Overcome Tumoral Immune Escape to Potentiate T-Cell Antitumor Responses"<br>*Cancer Research,* Vol. 73, No. 15, 30 May 2013 (2013-05-30),<br>4820-4829 | 1-22 |

Form PCT/ISA/210 (second sheet) (January 2015)

INTERNATIONAL SEARCH REPORT

International application No.
PCT/CN2021/102952

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/CN2021/102952**

| Patent document cited in search report | Publication date (day/month/year) | Patent family member(s) | Publication date (day/month/year) |
|---|---|---|---|
| WO 2016070001 A1 | 06 May 2016 | EP 3223865 A1 | 04 October 2017 |
| | | US 2017334999 A1 | 23 November 2017 |
| | | US 10626176 B2 | 21 April 2020 |
| | | EP 3223865 A4 | 03 October 2018 |
| CN 110551221 A | 10 December 2019 | CN 110551221 B | 05 March 2021 |
| | | WO 2021000530 A1 | 07 January 2021 |
| CN 108148137 A | 12 June 2018 | CN 108148137 B | 23 March 2021 |
| CN 102219856 A | 19 October 2011 | CN 102219856 B | 27 March 2013 |
| WO 2009073533 A2 | 11 June 2009 | TW 200938224 A | 16 September 2009 |
| | | US 2011085970 A1 | 14 April 2011 |
| | | MX 2010005830 A | 14 September 2010 |
| | | IL 205993 D0 | 30 November 2010 |
| | | JP 2011505372 A | 24 February 2011 |
| | | AU 2008334063 A2 | 20 January 2011 |
| | | CA 2706926 A1 | 11 June 2009 |
| | | AR 069747 A1 | 17 February 2010 |
| | | AU 2008334063 A1 | 11 June 2009 |
| | | CL 2008003526 A1 | 11 January 2010 |
| | | CN 101951959 A | 19 January 2011 |
| | | WO 2009073533 A3 | 26 November 2009 |
| | | BR PI0818963 A2 | 05 May 2015 |
| | | EA 201000910 A1 | 29 April 2011 |
| | | EP 2224958 A2 | 08 September 2010 |
| | | KR 20100093578 A | 25 August 2010 |
| WO 2019147670 A1 | 01 August 2019 | EP 3743447 A1 | 02 December 2020 |
| | | US 2021032346 A1 | 04 February 2021 |
| | | AU 2019211326 A1 | 10 September 2020 |
| | | CA 3089469 A1 | 01 August 2019 |
| | | KR 20200112913 A | 05 October 2020 |
| | | CN 112566934 A | 26 March 2021 |
| | | BR 112020014929 A2 | 08 December 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010618159
- WO 2016040724 A1
- WO 2019040780 A1
- WO 2009080251 A
- WO 2009080252 A


### Non-patent literature cited in the description


- **BERKOW et al.** The Merck Manual of Medical Information. Merck & Co. Inc, 2000
- **EBADI.** *CRC Desk Reference of Clinical Pharmacology,* 1998
- *J. Biol. Chem,* 1968, vol. 243, 3558
- **KABAT et al.** Sequences of Proteins of Immunological Interest. National Institutes of Health (U.S.), 1991
- *J Mol Biol,* 1997, vol. 273, 927-48